# EUROPEAN PATENT APPLICATION

(11) **EP 4 014 986 A1**
(43) Date of publication of application: **22.06.2022**
(21) Application number: 20851523.9
(22) Date of filing: 07.08.2020
(51) Int. Cl.: A61K 38/10, A61P 35/00

(54) **APPLICATION OF POLYPEPTIDE OR DERIVATIVE THEREOF**

(30) Priority: 13.08.2019 CN 201910743680
(71) Applicant: Chengdu Huitai Biomedicine Co., Ltd., Chengdu, Sichuan 610000 (CN)
(72) Inventor: WEI, De, Chengdu, Sichuan 610000 (CN); LI, Xiaomei, Chengdu, Sichuan 610000 (CN); DING, Yi, Chengdu, Sichuan 610000 (CN); XIAO, Ling, Chengdu, Sichuan 610000 (CN)
(74) Representative: Boult Wade Tennant LLP
(86) International application number: PCT/CN2020/107686
(87) International publication number: WO 2021/027704

(57) **Abstract**

An application of a polypeptide or a derivative thereof. Specifically provided are anti-tumor polypeptide drugs (amino acid sequence as represented by SEQ ID No. 1, and amino acid sequence obtained by deleting, replacing, adding and/or modifying one or more amino acids of the amino acid sequence as represented by SEQ ID No.1). These drugs have significant advantages in terms of drug target specificity, drug biological activity, drug toxicity, treatment cost, and the like. Polypeptides or derivatives thereof capable of inhibiting tumor cell activity and tumor metastasis are designed on the basis of cytokines involved in tumor microenvironment formation and homeostasis maintenance, and the polypeptides or derivatives thereof are further applied to the preparation of antitumor drugs.

## Description

### CROSS REFERENCE TO RELATED APPLICATIONS

This application claims the priority of Chinese Patent Application No. 201910743680.9, filed to China National Intellectual Property Administration on August 13, 2019, and titled with "APPLICATION OF POLYPEPTIDE OR DERIVATIVE THEREOF".

### FIELD

The present disclosure relates to the technical field of medical technology, specifically to the uses of polypeptides or derivatives thereof.

### BACKGROUND

Cancer (malignant tumor) is a serious and fatal disease with a high morbidity and mortality rate, which seriously affects human health. Although the understanding of the mechanism of tumorigenesis is constantly developing, the clinical treatment of malignant tumors is still a worldwide problem, especially for tumor metastasis and recurrence. In recent years, due to the early diagnosis of malignant tumors and targeted therapy of tumor growth suppression has been applied to the clinic, the survival rate of patients with malignant tumors has been improved. A series of new anti-tumor drugs have been applied in the clinic, which have a certain effect in inhibiting tumor growth and metastasis, especially targeted drugs such as neutralizing antibodies against various growth factors or small molecule kinase inhibitors. However, the current first-line therapies of anti-tumor commonly used in clinical practice are chemotherapy and radiotherapy, which have poor targeting, huge side effects, and are prone to cause treatment resistance, and their application in the clinical treatment of malignant tumors is limited. Some new targeted drugs also have deficiencies such as high off-target rate, high toxicity, strong side effects, and low efficacy, therefore could not meet the clinical needs for the effectiveness and safety of anti-tumor drugs. In addition, the clinical treatment of metastasis is still a worldwide problem. Therefore, new anti-tumor drugs with high targeting, strong specificity, and low toxic and side effects have become the key development direction of anti-tumor drug research and development, which can truly improve the clinical treatment effect and tumor accessibility.

Traditionally, tumors are usually classified based on the tissues or organs where the tumors happen. For example, malignant solid tumors can be divided into melanoma, glioma, lymphoma, esophageal cancer, lung cancer, liver cancer, pancreatic cancer, kidney cancer, breast cancer, stomach cancer, thyroid cancer, urothelial carcinoma, prostate cancer, colon cancer and the like. Although the diseased tissues and organs are different, malignant solid tumors have something in common in the pathological mechanism, that is, the formation and maintenance of tumor microenvironment, including the formation of tumor immunosuppressive microenvironment, activation of tumor related cells, accumulation of tumor related cytokines and extracellular matrix and the like. The formation and maintenance of tumor microenvironment play a key role in the occurrence, development and metastasis of tumor.

The tumor microenvironment is a complicated microenvironment composed of tumor cells, tumor stromal cells (e.g. tumor-associated fibroblasts), immune cells (e.g. tumor-associated macrophages), extracellular matrix (e.g. fibronectin, collagen) and the like. The interaction between cells and cells, cells and extracellular matrix jointly regulates the tumor microenvironment and promotes the occurrence and development of tumor. In the early stage of tumor development, the interaction between tumor cells and tumor related stromal cells promotes the formation of tumor microenvironment. Tumor cells activate tumor related stromal cells through secretion of a variety of cytokines (such as TGF-β, IL-10, LOX, ROS and the like). And the activated stromal cells express and secrete extracellular matrix, resulting in the accumulation of extracellular matrix in tumor microenvironment. Activated stromal cells also secrete a variety of cytokines (such as TGF-β, PDGF, VEGF, FGF, CTGF and the like) and promote tumorigenesis and development. At the same time, the formation of tumor microenvironment promotes tumor cells to escape the clearance of immune system and there are a large number of immunosuppressive factors in tumor microenvironment (PD-L1, TGF-β, IL-10, CSF-1 and GM-CSF) For example, programmed death ligand 1 (PD-L1) expressed and secreted by tumor cells can specifically bind to programmed cell death protein 1 (PD-1) on the surface of cytotoxic T cells, so as to inhibit the clearance of tumor cells by cytotoxic T cells. Transforming growth factors β (TGF-β), which is secreted by tumor cells, tumor stromal cells, tumor related immune cells and other cells, can effectively inhibit the immune activity of many immune cells in the tumor microenvironment, such as cytotoxic T cells, natural killer cells (NK cells), antigen presenting cells (APC), macrophages and so on, and promote tumor cells to escape the clearance of the immune system. In addition, the development of tumor microenvironment promotes tumor metastasis. Studies have shown that in the late stage of tumor development, tumor cells are affected by tumor microenvironment factors (such as extracellular matrix accumulation and cytokine regulation), tumor cells become interstitial, and the expression of migration and invasion related proteins such as fibronectin, N-cadherin and vimentin in tumor cells increases, which is manifested as the ability of tumor cell migration and invasion is enhanced, and lead to the metastasis of tumor cells to other parts of the body, that is, tumor metastasis (such as lung metastasis).

Therefore, targeted drugs against the key factors in the tumor microenvironment will directly inhibit or destroy the tumor microenvironment, enhance the clearance of tumor by immune system, block tumor infiltration and metastasis, and achieve the purpose of tumor treatment. In addition, selecting the key factors in the formation of tumor microenvironment as the target of drug will achieve a broad-spectrum anti-cancer effect against a variety of malignant solid tumors. For example, the currently marketed drug Keytruda is an anti-PD-1 antibody drug (Pembrolizumab), which inhibits the immune escape function of PD-1/PD-L1 in the tumor microenvironment and enhances the clearance of tumor cells by body's immune system to achieve the purpose of treating tumor. According to the clinic data, Keytruda has been approved for the treatment of a variety of malignant solid tumors including melanoma, non-small cell lung cancer, small cell lung cancer, classic Hodgkin's lymphoma, kidney cancer, head and neck squamous cell carcinoma, urothelial carcinoma, colorectal cancer, liver cancer, gastric cancer and the like.

In recent years, targeting peptide drugs have become one of the important areas of research and development of anti-tumor drug. The existing anti-tumor drugs are mainly chemical small molecule drugs and antibody drugs, which are still far from meeting the needs of clinical anti-tumor treatment in terms of effectiveness, targeting, specificity, or side effects. The active ingredients of peptide drugs are biologically active, and are generally amino acid chains formed by less than 100 amino acids. They are mainly obtained by three methods: separation and extraction from animals, plants and microorganisms, protein enzymatic hydrolysis, and artificial synthesis. Most of the current peptide drugs are derived from or mimic endogenous peptides or other natural peptides, which have clear structure and clear mechanism of action. And the metabolites of peptides are amino acids, which are the basic components of the body and will not accumulate in the body and have low toxic and side effects. In recent years, with the development of peptide synthesis and modification technology, artificially synthesized peptides have been able to meet the needs of peptide drug research and development in terms of preparation, yield, purity of products, and specific modifications. Peptide drugs have obvious advantages in the field of drug research and development. Compared with general organic small molecule drugs, peptide drugs have outstanding advantages such as high activity, small dosage, and low toxic and side effects. Compared with protein drugs, small peptide has relatively small immunogenicity, it can be chemically synthesized, the product purity is high, and the quality is controllable. Therefore, the development of anti-tumor peptide targeted drugs has obvious advantages in anti-tumor treatment, and will be a key research and development direction in the field of biomedical technology.

### SUMMARY

In view of this, the present invention provides uses of polypeptides or derivatives thereof. The polypeptide or derivative thereof (N1-N54) provided by the present invention can inhibit the migration ability of tumor cells, the expression of vimentin, fibronectin, and N-cadherin in tumor cells, and the invasion ability of tumor cells.

In order to achieve the above-mentioned purpose of the invention, the present invention provides the following technical solutions.

The present invention provides the uses of a polypeptide or a derivative thereof in the manufacture of a medicament for the prevention and/or treatment of a tumor, wherein the polypeptide comprises:
(I) an amino acid sequence set forth in SEQ ID NO: 1;
(II) an amino acid sequence derived from the amino acid sequence of (I) by deletion, substitution, addition and/or modification of one or more amino acids, and the polypeptide has a function identical or similar to that of the amino acid sequence of (I); or
(III) an amino acid sequence having more than 80% homology with the amino acid sequence of (I) or (II).

The polypeptide of the present invention refers to a class of compounds composed of amino acids connected by peptide bonds, and there is no limit to the number of amino acids.

The derivative of the present invention refers to a variant of the polypeptide. The derivative can be a product of deletion, substitution or addition of amino acid of the polypeptide. The derivative can also be a product of chemical modification at the end of the main chain or side chain of the polypeptide molecule, such as amino, carboxyl, sulfhydryl, phenolic hydroxyl, imidazolyl, guanidino, indolyl, methylthio and the like.

The chemical modification described in the present invention is to chemically modify the polypeptide at the level of polypeptide by using suitable modification methods and modifiers so as to improve the solubility, stability, and half-life of the modified polypeptide drugs, which can be determined by those skilled in the art by a conventional manner.

The purpose of the deletion, addition, substitution or modification of the amino acid sequence SEQ ID NO: 1 of the present invention is to make it suitable for the use of the present invention in preparing drugs for preventing and/or treating tumors.

The deletion, substitution, addition and/or modification of the amino acids can be carried out separately, or simultaneously, and can be carried out at the same amino acid position or at different amino acid positions.

The amino acid position in the present invention refers to the amino acid positions arranged from the amino terminal to the carboxyl terminal in the amino acid sequence of a polypeptide. The amino acid positions are relative. When amino acids are deleted or added in the amino acid sequence, the amino acid positions may change, and this change can be determined by a person skilled in the art.

The amino acids used for substitution or/and addition in the present invention include natural amino acids and unnatural amino acids, wherein natural amino acids refer to amino acids that exist in nature, and unnatural amino acids include D-amino acids and other artificially synthesized amino acids.

In some specific embodiments of the present invention, the deletion is selected from the group consisting of a deletion of an amino acid at the amino terminal of the polypeptide, a deletion of an amino acid at the carboxyl terminal of the polypeptide, a deletion of amino acid within the sequence of the polypeptide and a combination thereof.

In some specific embodiments of the present invention, the deletion is a deletion of an amino acid at the amino terminal, a deletion of an amino acid at the carboxyl terminal, or a deletion of an amino acid within the sequence of the polypeptide, respectively.

In some specific embodiments of the present invention, the deletion is a deletion of an amino acid at the amino terminal and a deletion of an amino acid at the carboxyl terminal of the polypeptide at the same time.

In some specific embodiments of the present invention, the deletion is a deletion of an amino acid at the amino terminal and a deletion of an amino acid within the sequence of the polypeptide at the same time.

In some specific embodiments of the present invention, the deletion is a deletion of an amino acid at the carboxyl terminal and a deletion of amino acid within the sequence of the polypeptide at the same time.

In some specific embodiments of the present invention, the deletion is a deletion of an amino acid at the amino terminal, a deletion of an amino acid at the carboxyl terminal, and a deletion of an amino acid within the sequence of the polypeptide at the same time.

In some specific embodiments of the present invention, the number of deleted amino acid is 1, 2, 3, 4, 5, 6, 7, 8, or 9.

In some specific embodiments of the present invention, the amino acid sequence of the polypeptide is as shown in SEQ ID NO: 2, SEQ ID NO: 4, SEQ ID NO: 6, SEQ ID NO: 8, SEQ ID NO: 12 or SEQ ID NO: 13.

In a specific embodiment of the present invention, the polypeptide is obtained by deletion of one amino acid (position 18) at the carboxyl terminal of the amino acid sequence of the polypeptide as shown in SEQ ID NO: 1, and the sequence is as shown in SEQ ID NO: 2.

In a specific embodiment of the present invention, the polypeptide is obtained by deletion of four amino acids at the carboxyl terminal (positions 15-18), and deletion of three amino acids at the amino terminal (positions 1-3) of the amino acid sequence of the polypeptide as shown in SEQ ID NO: 1 at the same time, and the sequence is as shown in SEQ ID NO: 4.

In a specific embodiment of the present invention, the polypeptide is obtained by deletion of four amino acids at the carboxyl terminal (positions 15-18) of the amino acid sequence of the polypeptide as shown in SEQ ID NO: 1, and the sequence is as shown in SEQ ID NO: 6.

In a specific embodiment of the present invention, the polypeptide is obtained by deletion of four amino acids at the carboxyl terminal (positions 15-18), and deletion of three amino acids within the sequence (positions 3, 4 and 11) of the amino acid sequence of the polypeptide as shown in SEQ ID NO: 1 at the same time, and the sequence is as shown in SEQ ID NO: 8.

In a specific embodiment of the present invention, the polypeptide is obtained by deletion of 1 amino acid at the carboxyl terminal (position 18), and deletion of two amino acids at the amino terminal (positions 1-2) of the amino acid sequence of the polypeptide as shown in SEQ ID NO: 1, and the sequence is as shown in SEQ ID NO: 12.

In a specific embodiment of the present invention, the polypeptide is obtained by deletion of four amino acids at the carboxyl terminal (positions 15-18), and deletion of five amino acids at the amino terminal (positions 1-5)of the amino acid sequence as shown in SEQ ID NO: 1 at the same time, and the sequence is as shown in SEQ ID NO: 13.

In some specific embodiments of the present invention, the addition is selected from the group consisting of an addition of an amino acid at the amino terminal of the polypeptide, an addition of an amino acid at the carboxyl terminal of the polypeptide, an addition of an amino acid within the sequence of the polypeptide and a combination thereof.

In some specific embodiments of the present invention, the addition is an addition of an amino acid at the amino terminal, an addition of an amino acid at the carboxyl terminal, and an addition of an amino acid within the sequence of the polypeptide, respectively.

In some specific embodiments of the present invention, the addition is an addition of an amino acid at the amino terminal, and an addition of an amino acid at the carboxyl terminal of the polypeptide at the same time.

In some specific embodiments of the present invention, the addition is an addition of an amino acid at the amino terminal and an addition of an amino acid within the sequence of the polypeptide at the same time.

In some specific embodiments of the present invention, the addition is an addition of an amino acid at the carboxyl terminal and an addition of an amino acid within the sequence of the polypeptide at the same time.

In some specific embodiments of the present invention, the addition is an addition of an amino acid at the amino terminal, an addition of an amino acid at the carboxyl terminal, and an addition of an amino acid within the sequence of the polypeptide at the same time.

In some specific embodiments of the present invention, the number of added amino acid is 1, 2, 3, 4 or 5.

In some specific embodiments of the present invention, the amino acids used for addition include natural amino acids and/or unnatural amino acids.

In some specific embodiments of the present invention, the amino acid sequence of the polypeptide is as shown in SEQ ID NO: 17 or SEQ ID NO: 18.

In a specific embodiment of the present invention, the polypeptide is obtained by addition of two amino acids to the carboxyl terminal of the amino acid sequence shown in SEQ ID NO: 1, and the sequence is as shown in SEQ ID NO: 17.

In a specific embodiment of the present invention, the polypeptide is obtained by addition of one amino acid at the amino terminal, and addition of one amino acid at the carboxyl terminal of the amino acid sequence shown in SEQ ID NO: 1 at the same time, and the sequence is as shown in SEQ ID NO: 18.

In some specific embodiments of the present invention, the substitution is selected from the group consisting of a substitution of an amino acid at the amino terminal of the polypeptide, a substitution of an amino acid at the carboxyl terminal of the polypeptide, a substitution of an amino acid within the sequence of the polypeptide and a combination thereof.

In some specific embodiments of the present invention, the substitution is a substitution of an amino acid at the amino terminal, a substitution of an amino acid at the carboxyl terminal, and an addition of an amino acid within the sequence of the polypeptide, respectively.

In some specific embodiments of the present invention, the substitution is a substitution of an amino acid at the amino terminal, and a substitution of an amino acid at the carboxyl terminal of the polypeptide at the same time.

In some specific embodiments of the present invention, the substitution is a substitution of an amino acid at the amino terminal, and a substitution of an amino acid within the sequence of the polypeptide at the same time.

In some specific embodiments of the present invention, the substitution is a substitution of an amino acid at the carboxyl terminal, and a substitution of an amino acid within the sequence of the polypeptide at the same time.

In some specific embodiments of the present invention, the substitution is a substitution of an amino acid at the amino terminal, a substitution of an amino acid at the carboxyl terminal, and a substitution of an amino acid within the sequence of the polypeptide at the same time.

In some specific embodiments of the present invention, the number of substituted amino acid is 1, 2, 3, 4 or 5.

In some specific embodiments of the present invention, the amino acids used for substitution include natural amino acids and/or unnatural amino acids.

In some specific embodiments of the present invention, the modification is selected from the group consisting of a modification of an amino acid at the amino terminal of the polypeptide, a modification of an amino acid at the carboxyl terminal of the polypeptide, a modification of an amino acid within the sequence of the polypeptide and a combination thereof.

In some specific embodiments of the present invention, the modification is a modification of an amino acid at the amino terminal, a modification of an amino acid at the carboxyl terminal, and a modification of an amino acid within the sequence of the polypeptide, respectively.

In some specific embodiments of the present invention, the modification is a modification of an amino acid at the amino terminal, and a modification of an amino acid at the carboxyl terminal of the polypeptide at the same time.

In some specific embodiments of the present invention, the modification is a modification of an amino acid at the amino terminal, and a modification of an amino acid within the sequence of the polypeptide at the same time.

In some specific embodiments of the present invention, the modification is a modification of an amino acid at the carboxyl terminal, and a modification of an amino acid within the sequence of the polypeptide at the same time.

In some specific embodiments of the present invention, the modification is a modification of an amino acid at the amino terminal, a modification of an amino acid at the carboxyl terminal, and a modification of an amino acid within the sequence of the polypeptide at the same time.

In some specific embodiments of the present invention, the modification is a chemical modification.

In some specific embodiments of the present invention, the chemical modification can change the main chain structure or side chain group of the peptide, including acetylation, amidation, glycosylation, polyethylene glycol (PEG) modification, fatty acid modification, other polypeptide modification techniques known in the art, or a combination thereof.

The acetylation and amidation of the present invention are commonly used methods for the modification of the terminals of the main chain of polypeptides, usually acetylation is carried out at the N-terminal of the polypeptide and amidation is carried out at the C-terminal of the polypeptide.

The glycosylation modification of the present invention refers to the covalent attachment of sugars with certain special functional groups in the polypeptide, including N-glycosylation, O-glycosylation, S-glycosylation, C-glycosylation and glycosyl phosphatidylinositol modification and the like. The N-glycosylation is to connect the sugar with the side chain via amide nitrogen of asparagine, and the O-glycosylation is to connect the sugar with the side chain via the oxygen residue of serine or threonine. The sugars include various monosaccharides, oligosaccharides and polysaccharides.

The PEG modification in the present invention refers to the modification at the functional groups with selected types of PEG, and the functional groups include the main chain amino group, side chain amino group, main chain carboxyl group, side chain carboxyl group, imidazole group, sulfhydryl group and hydroxyl group, as a modification site. The PEG is a macromolecular polymer polymerized by ethylene oxide, without limitation on structure or molecular weight. The types of PEG for modification include linear PEG, branched PEG, homobifunctional PEG derivatives, heterofunctional double-substituted PEG derivatives, and multi-arm functional PEG derivatives.

The fatty acid modification in the present invention refers to the covalent attachment of fatty acid structure with certain special functional groups in the polypeptide, including the modification of amino, carboxyl, sulfhydryl, and hydroxyl. Fatty acid modification can be divided into modification of unsaturated fatty acid and modification of saturated fatty acid. Modification of saturated fatty acid is mainly modified with myristic acid and palmitic acid, and modification of unsaturated fatty acid is mainly modified with oleic acid and linoleic acid.

The polypeptide modification of the present invention can use methods well known to those skilled in the art. The purpose of the modification of the present invention is to change the physicochemical properties of the polypeptides and improve the druggability of the polypeptides.

In some specific embodiments of the present invention, the number of modified amino acid is 1, 2, 3, 4, 5, 6, 7, 8, or 9.

In some specific embodiments of the present invention, the amino acids used for modification include natural amino acids and/or unnatural amino acids.

The polypeptide or derivative thereof provided by the present invention is a polypeptide obtained by deletion, substitution, addition and/or modification of an amino acid in the polypeptide shown in SEQ ID NO: 1. It may be the deletion, substitution, addition or modification of the polypeptide shown in SEQ ID NO: 1 respectively. It may also be at least two of deletion, substitution, addition and modification of the polypeptide shown in SEQ ID NO: 1 at the same time. The position of deletion, substitution, addition or modification may be at the amino-terminal, at the carboxy-terminal, or within the amino acid sequence of the polypeptide shown in SEQ ID NO: 1, and deletion, substitution, addition or modification of amino acid may be made respectively. It may also be deletions, substitutions, additions or modifications of amino acid at the amino terminal and carboxyl terminal of the amino acid sequence of the polypeptide shown in SEQ ID NO: 1 at the same time. It may also be deletions, substitutions, additions or modifications of amino acid at the amino terminal and within the amino acid sequence of the polypeptide shown in SEQ ID NO: 1 at the same time. It may also be deletions, substitutions, additions or modifications of amino acid at the carboxyl terminal and within the amino acid sequence of the polypeptide shown in SEQ ID NO: 1 at the same time. It may also be deletions, substitutions, additions or modifications of amino acid at the amino terminal, carboxyl terminal and within the amino acid sequence of the polypeptide shown in SEQ ID NO: 1 at the same time. The deletion, substitution, addition or modification of amino acid may be carried out at any amino acid positions in the sequence of the polypeptide, including the amino terminal, the carboxyl terminal and within the sequence.

In some specific embodiments of the present invention, the polypeptide is derived from SEQ ID NO: 1 by a manner selected from the group consisting of: deletion and addition of an amino acid at the same time, substitution and addition of an amino acid at the same time, deletion and substitution of an amino acid at the same time, deletion and modification of an amino acid at the same time, addition and modification of an amino acid at the same time, substitution and modification of an amino acid at the same time, deletion, addition and modification of an amino acid at the same time, substitution, addition and modification of an amino acid at the same time, deletion, substitution and modification of an amino acid at the same time, deletion, substitution, addition and modification of an amino acid at the same time, and a combination thereof.

In some specific embodiments of the present invention, the amino acid sequence of the polypeptide is selected from the group consisting of SEQ ID NO: 5, SEQ ID NO: 14, SEQ ID NO: 19, SEQ ID NO: 20, SEQ ID NO: 21, SEQ ID NO: 7, SEQ ID NO: 15, SEQ ID NO: 16, SEQ ID NO: 25, SEQ ID NO: 26, SEQ ID NO: 22, SEQ ID NO: 23, SEQ ID NO: 24, SEQ ID NO: 3, SEQ ID NO: 9, SEQ ID NO: 10, SEQ ID NO: 11, SEQ ID NO: 28, SEQ ID NO:29, SEQ ID NO: 30, SEQ ID NO: 31, SEQ ID NO: 27, SEQ ID NO: 37, SEQ ID NO: 38, SEQ ID NO: 39, SEQ ID NO: 32, SEQ ID NO: 33, SEQ ID NO: 40, SEQ ID NO: 41, SEQ ID NO: 42, SEQ ID NO: 43, SEQ ID NO: 44, SEQ ID NO: 34, SEQ ID NO: 35, SEQ ID NO: 36, SEQ ID NO: 50, SEQ ID NO: 51, SEQ ID NO: 52, SEQ ID NO: 53, SEQ ID NO: 54, SEQ ID NO: 45, SEQ ID NO: 46, SEQ ID NO: 48, SEQ ID NO: 49 and SEQ ID NO: 47.

In some specific embodiments of the present invention, the polypeptide is obtained by addition and deletion of amino acid in the sequence as shown in SEQ ID NO: 1 at the same time, and the amino acid sequence of the polypeptide is shown in SEQ ID NO: 5, SEQ ID NO: 14, SEQ ID NO: 19, SEQ ID NO: 20 or SEQ ID NO: 21.

In a specific embodiment of the present invention, the polypeptide is obtained by addition of one amino acid at the carboxyl terminal, and deletion of three amino acids (positions 1-3) at the amino terminal of the amino acid sequence of the polypeptide as shown in SEQ ID NO: 1 at the same time, and the sequence is as shown in SEQ ID NO: 5.

In a specific embodiment of the present invention, the polypeptide is obtained by deletion of five amino acids at the amino terminal (positions 1-5), and addition of one amino acid at the carboxyl terminal of the amino acid sequence shown in SEQ ID NO: 1, and the sequence is as shown in SEQ ID NO:14.

In a specific embodiment of the present invention, the polypeptide is obtained by deletion of five amino acids at the amino terminal (positions 1-5), and deletion of four amino acids deleted at the carboxyl terminus (position 15-18), and addition of two amino acids at the carboxyl terminal at of the amino acid sequence shown in SEQ ID NO: 1 at the same time, and the sequence is as shown in SEQ ID NO: 19.

In a specific embodiment of the present invention, the polypeptide is obtained by addition of one amino acid at the amino terminal, and deletion of one amino acid at the carboxyl terminal (position 18) of the amino acid sequence shown in SEQ ID NO: 1, and the sequence is as shown in SEQ ID NO: 20.

In a specific embodiment of the present invention, the polypeptide is obtained by deletion of five amino acids at the amino terminal (positions 1-5), and deletion of four amino acids deleted at the carboxyl terminus (position 15-18), and addition of one amino acid at the amino terminal of the amino acid sequence shown in SEQ ID NO: 1 at the same time, and the sequence is as shown in SEQ ID NO: 21.

In some specific embodiments of the present invention, the amino acid sequence shown in SEQ ID NO: 1 is substituted and added at the same time, and the sequence of the obtained polypeptide is as shown in SEQ ID NO: 7, as shown in SEQ ID NO: 15, as shown in SEQ ID NO: 16, as shown in SEQ ID NO: 25 or as shown in SEQ ID NO: 26.

In a specific embodiment of the present invention, the polypeptide is obtained by addition of one amino acid at the carboxyl terminal, and substitution of one amino acid at the 15th position (A15R) within the sequence of the amino acid sequence set forth in SEQ ID NO: 1, and the sequence is as shown in SEQ ID NO: 7.

In a specific embodiment of the present invention, the polypeptide is obtained by substitution of one amino acid at the 18th position (N18R) of the carboxyl terminal, and addition of one amino acid to the carboxyl terminal of the amino acid sequence set forth in SEQ ID NO: 1 at the same time, and the sequence is as shown in SEQ ID NO: 15.

In a specific embodiment of the present invention, the polypeptide is obtained by substitution of one amino acid at position 7 (A7R) within the sequence, and addition of one amino acid at the carboxyl terminus of the amino acid sequence set forth in SEQ ID NO: 1 at the same time, and the sequence is as shown in SEQ ID NO: 16.

In a specific embodiment of the present invention, the polypeptide is obtained by substitution of one amino acid at the carboxyl terminal (N18R) within the sequence, and addition of two amino acids at the carboxyl terminal of the amino acid sequence set forth in SEQ ID NO: 1 at the same time, and the sequence is as shown in SEQ ID NO: 25.

In a specific embodiment of the present invention, the polypeptide is obtained by substitution of one amino acid at position 15 (A15R) within the sequence, and addition of one amino acid at the amino terminal, and addition of one amino acid at the carboxyl terminal of the amino acid sequence set forth in SEQ ID NO: 1 at the same time, and the sequence is as shown in SEQ ID NO: 26.

In some specific embodiments of the present invention, the polypeptide is obtained by deletion and substitution of amino acids in SEQ ID NO: 1 at the same time, and the sequence is as shown in SEQ ID NO: 22, SEQ ID NO: 23 or SEQ ID NO: 24.

In a specific embodiment of the present invention, the polypeptide is obtained by substitution of one amino acid at position 7 (A7R) within the sequence, deletion of three amino acids at the amino terminal (A7R), and deletion of four amino acids at the carboxyl terminal (positions 15-18) of the amino acid sequence shown in SEQ ID NO: 1 at the same time, and the sequence is as shown in SEQ ID NO: 22.

In a specific embodiment of the present invention, the polypeptide is obtained by substitution of one amino acid at position 7 (A7R) within the sequence, and deletion of one amino acid at position 11 within the sequence, and deletion of four amino acids at the carboxyl terminal (positions 15-18) of the amino acid sequence shown in SEQ ID NO: 1 at the same time, and the sequence is as shown in SEQ ID NO: 23.

In a specific embodiment of the present invention, the polypeptide is obtained by substitution of one amino acid at position 7 (A7R) within the sequence, and deletion of two amino acids at the amino terminal (positions 1-2), and deletion of one amino acid at the carboxyl terminal (position 18) of the amino acid sequence set forth in SEQ ID NO: 1 at the same time, and the sequence is as shown in SEQ ID NO: 24.

In some specific embodiments of the present invention, the polypeptide is obtained by deletion and modification of amino acids in SEQ ID NO: 1 at the same time, and the sequence is as shown in SEQ ID NO: 3, SEQ ID NO: 9, SEQ ID NO: 10, SEQ ID NO: 11, SEQ ID NO: 28, SEQ ID NO: 29, SEQ ID NO: 30, or SEQ ID NO: 31.

In a specific embodiment of the present invention, the polypeptide is obtained by deletion of one amino acid at the carboxyl terminal (position 18), and modification with polyethylene glycol (PEG) at the amino terminal (position 1) of the amino acid sequence shown in SEQ ID NO: 1 at the same time, and the sequence is as shown in SEQ ID NO: 3.

In a specific embodiment of the present invention, the polypeptide is obtained by deletion of four amino acids at the carboxyl terminal (positions 15-18), and modification with polyethylene glycol (PEG) at the amino terminal (position 1) of the amino acid sequence shown in SEQ ID NO: 1 at the same time, and the sequence is as shown in SEQ ID NO: 9.

In a specific embodiment of the present invention, the polypeptide is obtained by deletion of four amino acids at the carboxyl terminal (positions 15-18), deletion of three amino acids at the amino terminal (positions 1-3), and modification with polyethylenee glycol (PEG) at the carboxyl terminal of the amino acid sequence shown in SEQ ID NO: 1 at the same time, and the sequence is as shown in SEQ ID NO: 10.

In a specific embodiment of the present invention, the polypeptide is obtained by deletion of four amino acids at the carboxyl terminal (positions 15-18), deletion of three amino acids at the amino terminal (positions 1-3), and modification with polyethylene glycol (PEG) at the amino terminal of the amino acid sequence shown in SEQ ID NO: 1 at the same time, and the sequence is as shown in SEQ ID NO: 11.

In a specific embodiment of the present invention, the polypeptide is obtained by deletion of two amino acids (positions 1-2) at the amino terminal, deletion of one amino acid at the carboxyl terminus (position 18), and modification with polyethylene glycol (PEG) at the amino terminal of the amino acid sequence shown in SEQ ID NO: 1 at the same time, and the sequence is as shown in SEQ ID NO: 28.

In a specific embodiment of the present invention, the polypeptide is obtained by deletion of five amino acids (positions 1-5) at the amino terminal, and deletion of four amino acids at the carboxyl terminal (positions 15-18), and modification with polyethylene glycol (PEG) at the amino terminal of the amino acid sequence shown in SEQ ID NO: 1 at the same time, and the sequence is as shown in SEQ ID NO: 29.

In a specific embodiment of the present invention, the polypeptide is obtained by deletion of five amino acids (positions 1-5) at the amino terminal, deletion of four amino acids at the carboxyl terminal (positions 15-18), and modification with polyethylene glycol (PEG) at the carboxyl terminal of the amino acid sequence shown in SEQ ID NO: 1 at the same time, and the sequence is as shown in SEQ ID NO: 30.

In a specific embodiment of the present invention, the polypeptide is obtained by deletion of three amino acids (position 3, position 4, and position 11) within the sequence, deletion of four amino acids at the carboxyl terminal (positions 15-18), and modification with polyethylene glycol (PEG) at the carboxyl terminal of the amino acid sequence shown in SEQ ID NO: 1 at the same time, and the sequence is as shown in SEQ ID NO: 31.

In some specific embodiments of the present invention, the amino acid sequence shown in SEQ ID NO: 1 is added and modified at the same time, and the sequence of the obtained polypeptide is as shown in SEQ ID NO: 27, as shown in SEQ ID NO: 37, as shown in SEQ ID NO: ID No. 38 or as shown in SEQ ID NO: 39.

In a specific embodiment of the present invention, the polypeptide is obtained by addition of one amino acid at the carboxyl terminal, and modification with polyethylene glycol (PEG) at the carboxyl terminal of the amino acid sequence shown in SEQ ID NO: 1 at the same time, and the sequence is as shown in SEQ ID NO: 27.

In a specific embodiment of the present invention, the polypeptide is obtained by addition of two amino acids to the carboxyl terminal, and modification with polyethylene glycol (PEG) at the amino terminal of the amino acid sequence shown in SEQ ID NO: 1 at the same time, and the sequence is as shown in SEQ ID NO: 37.

In a specific embodiment of the present invention, the polypeptide is obtained by addition of one amino acid at the amino terminal, addition of one amino acid at the carboxyl terminal, and modification with polyethylene glycol (PEG) at the carboxyl terminal of the amino acid sequence shown in SEQ ID NO: 1, and the sequence is as shown in SEQ ID NO: 38.

In a specific embodiment of the present invention, the polypeptide is obtained by addition of two amino acids to the carboxyl terminal, and modification with polyethylene glycol (PEG) at the carboxyl terminal of the amino acid sequence shown in SEQ ID NO: 1 at the same time, the sequence is as shown in SEQ ID NO: 39.

In some specific embodiments of the present invention, the polypeptide is obtained by deletion, addition and modification of amino acid in SEQ ID NO: 1 at the same time, and the sequence of the obtained polypeptide is as shown in SEQ ID NO: 32, SEQ ID NO: 33, SEQ ID NO: 40, SEQ ID NO: 41, SEQ ID NO: 42, SEQ ID NO: 43 or SEQ ID NO: 44.

In a specific embodiment of the present invention, the polypeptide is obtained by deletion of three amino acids at the amino terminal (positions 1-3), addition of one amino acid at the carboxyl terminal, and modification with polyethylene glycol (PEG) at the carboxyl terminal of the amino acid sequence shown in SEQ ID NO: 1 at the same time, and the sequence is as shown in SEQ ID NO: 32.

In a specific embodiment of the present invention, the polypeptide is obtained by deletion of five amino acids at the amino terminal (positions 1-5), addition of one amino acid at the carboxyl terminal, and modification with polyethylene glycol (PEG) at the carboxyl terminal of the amino acid sequence shown in SEQ ID NO: 1 at the same time, and the sequence is as shown in SEQ ID NO: 33.

In a specific embodiment of the present invention, the polypeptide is obtained by deletion of five amino acids at the amino terminal (positions 1-5), deletion of deletion of four amino acids at the carboxyl terminal (positions 15-18), modification with polyethylene glycol (PEG) at the amino terminal, and addition of two amino acids at the carboxyl terminal of the amino acid sequence shown in SEQ ID NO: 1 at the same time, and the sequence is as shown in SEQ ID NO: 40.

In a specific embodiment of the present invention, the polypeptide is obtained by addition of two amino acids at the amino terminal, deletion of one amino acid at the carboxyl terminal (position 18), and modification with polyethylene glycol (PEG) at the carboxyl terminal of the amino acid sequence shown in SEQ ID NO: 1 at the same time, and the sequence is as shown in SEQ ID NO: 41.

In a specific embodiment of the present invention, the polypeptide is obtained by deletion of five amino acids at the amino terminal (positions 1-5), deletion of four amino acids at the carboxyl terminal (positions 15-18), modification with polyethylene glycol (PEG) at the amino terminal, and addition of one amino acid at the carboxyl terminal of the amino acid sequence shown in SEQ ID NO: 1 at the same time, and the sequence is as shown in SEQ ID NO: 42.

In a specific embodiment of the present invention, the polypeptide is obtained by addition of one amino acid at the amino terminal, deletion of one amino acid at the carboxyl terminal (position 18), and modification with polyethylene glycol (PEG) at the carboxyl terminal of the amino acid sequence shown in SEQ ID NO: 1 at the same time, and the sequence is as shown in SEQ ID NO: 43.

In a specific embodiment of the present invention, the polypeptide is obtained by addition of one amino acid within the sequence (between position 2 and position 3), deletion of one amino acid at the carboxyl terminal (position 18), and modification with polyethylene glycol (PEG) at the carboxyl terminal of the amino acid sequence shown in SEQ ID NO: 1 at the same time, and the sequence is as shown in SEQ ID NO: 44.

In some specific embodiments of the present invention, the polypeptide is obtained by substitution, addition, and modification of amino acid in SEQ ID NO: 1 at the same time, and the sequence of the obtained polypeptide is as shown in SEQ ID NO: 34, SEQ ID NO: 35, SEQ ID NO: 36, SEQ ID NO: 50, SEQ ID NO: 51, SEQ ID NO: 52, SEQ ID NO: 53 or SEQ ID NO: 54.

In a specific embodiment of the present invention, the polypeptide is obtained by substitution of one amino acid at position 15 (A15R) within the sequence, addition of one amino acid at the carboxyl terminal, and modification with polyethylene glycol (PEG) at the amino terminal of the amino acid sequence shown in SEQ ID NO: 1 at the same time, and the sequence is as shown in SEQ ID NO: 34.

In a specific embodiment of the present invention, the polypeptide is obtained by substitution of one amino acid at position 15 (A15R) within the sequence, addition of one amino acid at the carboxyl terminal, and modification with polyethylene glycol (PEG) at the carboxyl terminal of the amino acid sequence shown in SEQ ID NO: 1 at the same time, and the sequence is as shown in SEQ ID NO: 35.

In a specific embodiment of the present invention, the polypeptide is obtained by substitution of one amino acid at position 7 (A7R) within the sequence, addition of one amino acid at the carboxyl terminal, and modification with polyethylene glycol (PEG) at the carboxyl terminal of the amino acid sequence shown in SEQ ID NO: 1 at the same time, and the sequence is as shown in SEQ ID NO: 36.

In a specific embodiment of the present invention, the polypeptide is obtained by substitution of one amino acid at the carboxyl terminal (N18R), addition of 2 amino acid at the carboxyl terminal, and modification with polyethylene glycol (PEG) at the amino terminal of the amino acid sequence shown in SEQ ID NO: 1 at the same time, and the sequence is as shown in SEQ ID NO: 50.

In a specific embodiment of the present invention, the polypeptide is obtained by substitution of one amino acid at position 15 (A15R) within the sequence, addition of one amino acid at the carboxyl terminal, addition of one amino acid at the amino terminal, and modification with polyethylene glycol (PEG) at the amino terminal of the amino acid sequence shown in SEQ ID NO: 1 at the same time, and the sequence is as shown in SEQ ID NO: 51.

In a specific embodiment of the present invention, the polypeptide is obtained by substitution of one amino acid at the carboxyl terminal (N18R), addition of two amino acids at the carboxyl terminal, and modification with polyethylene glycol (PEG) at the carboxyl terminal of the amino acid sequence shown in SEQ ID NO: 1 at the same time, and the sequence is as shown in SEQ ID NO: 52.

In a specific embodiment of the present invention, the polypeptide is obtained by substitution of one amino acid at position 15 (A15R) within the sequence, addition of one amino acids at the amino terminal, addition of one amino acids at the carboxyl terminal, and modification with polyethylene glycol (PEG) at the carboxyl terminal of the amino acid sequence shown in SEQ ID NO: 1 at the same time, and the sequence is shown in SEQ ID NO: 53.

In a specific embodiment of the present invention, the polypeptide is obtained by substitution of one amino acid at position 7 (A7R) within the sequence, addition of two amino acids at the carboxyl terminal, and modification with polyethylene glycol (PEG) at the carboxyl terminal of the amino acid sequence shown in SEQ ID NO: 1 at the same time, and the sequence is as shown in SEQ ID NO: 54.

In some specific embodiments of the present invention, the polypeptide is obtained by deletion, substitution and modification of amino acid in SEQ ID NO: 1 at the same time, and the sequence of the obtained polypeptide is as shown in SEQ ID NO: 45, SEQ ID NO: 46, SEQ ID NO: 48 or SEQ ID NO: 49.

In a specific embodiment of the present invention, the polypeptide is obtained by substitution of one amino acid at position 11 (V11R) within the sequence, deletion of five amino acids at the amino terminal (positions 1-5), deletion of four amino acids at the carboxyl terminal (positions 15-18), and modification with polyethylene glycol (PEG) at the carboxyl terminal of the amino acid sequence shown in SEQ ID NO:1 at the same time, and the sequence is shown in SEQ ID NO: 45.

In a specific embodiment of the present invention, the polypeptide is obtained by substitution of one amino acid at position 7 (A7R) within the sequence, deletion of one amino acid within the sequence (position 11), deletion of one amino acid at the carboxyl terminal (position 18), and modification with polyethylene glycol (PEG) at the amino terminal of the amino acid sequence shown in SEQ ID NO: 1 at the same time, and the sequence is shown in SEQ ID NO: 46.

In a specific embodiment of the present invention, the polypeptide is obtained by substitution of one amino acid at position 7 (A7R) within the sequence, deletion of one amino acid within the sequence (position 11), deletion of one amino acid at the carboxyl terminal (position 18), and modification with polyethylene glycol (PEG) at the carboxyl terminal of the amino acid sequence shown in SEQ ID NO: 1 at the same time, and the sequence is shown in SEQ ID NO: 48.

In a specific embodiment of the present invention, the polypeptide is obtained by substitution of one amino acid at position 7 (A7R) within the sequence, deletion of two amino acids at the amino terminal (positions 1-2), deletion of one amino acid at the carboxyl terminal (position 18), and modification with polyethylene glycol (PEG) at the carboxyl terminal of the amino acid sequence shown in SEQ ID NO: 1 at the same time, and the sequence is shown in SEQ ID NO: 49.

In some specific embodiments of the present invention, the polypeptide is obtained by deletion, substitution, addition and modification of amino acid in SEQ ID NO: 1at the same time, and the sequence of the obtained polypeptide is as shown in SEQ ID NO: 47.

In a specific embodiment of the present invention, the polypeptide is obtained by substitution of one amino acid at the carboxyl terminal (N18R), deletion of two amino acids at the amino terminal (positions 1-2), addition of one amino acid at the carboxyl terminal, and modification with polyethylene glycol (PEG) at the carboxyl terminal of the amino acid sequence shown in SEQ ID NO: 1, and the sequence is shown in SEQ ID NO: 47.

In the present invention, the amino acid sequence of the polypeptide has more than 80% homology with the amino acid sequence of (I) or (II); preferably, the amino acid sequence of the polypeptide has more than 85% homology with the amino acid sequence of (I) or (II); more preferably, the amino acid sequence of the polypeptide has more than 90% homology with the amino acid sequence of (I) or (II); more preferably, the amino acid sequence of the polypeptide has more than 95% homology with the amino acid sequence of (I) or (II); most preferably, the amino acid sequence of the polypeptide has more than 97% homology with the amino acid sequence of (I) or (II).

The preparation methods of the polypeptides or derivatives thereof in the present invention include natural extraction, enzymatic hydrolysis, fermentation, recombination expression, and chemical synthesis.

In some specific embodiments of the present invention, the tumor is selected from the group consisting of a head and neck cancer, a respiratory system tumor, a gastrointestinal tumor, a urinary system tumor, a male reproductive tumor, a gynecologic tumor, a skin cancer, an endothelial cell tumor, a brain tumor, a nervous system tumor, an endocrine organ tumor and a combination thereof.

In some specific embodiments of the present invention, the head and neck cancer is selected from the group consisting of a lip cancer, an oral cancer, a salivary gland cancer, an oropharyngeal cancer, a nasopharyngeal cancer, a hypopharyngeal cancer and a combination thereof.

In some specific embodiments of the present invention, the respiratory system tumor is selected from the group consisting of a laryngeal cancer, a lung cancer, a mesothelioma and a combination thereof.

In some specific embodiments of the present invention, the gastrointestinal tumor is selected from the group consisting of a colorectal cancer, an anal cancer, an esophageal cancer, a gastric cancer, a liver cancer, a gallbladder carcinoma, a pancreatic cancer and a combination thereof.

In some specific embodiments of the present invention, the urinary system tumor is selected from the group consisting of a kidney cancer, a bladder cancer and a combination thereof.

In some specific embodiments of the present invention, the male reproductive tumor is selected from the group consisting of a penile cancer, a prostate cancer, a testicular cancer and a combination thereof.

In some specific embodiments of the present invention, the gynecologic tumor is selected from the group consisting of a breast cancer, a vulvar cancer, a vaginal cancer, a cervical cancer, a corpus carcinoma, an ovarian cancer and a combination thereof.

In some specific embodiments of the present invention, the skin cancer is selected from the group consisting of a melanoma, a non-melanoma skin cancer and a combination thereof.

In some specific embodiments of the present invention, the endothelial cell tumor includes Kaposi's sarcoma; the brain tumor includes brain cancer; the nervous system tumor includes central nervous system tumor; and the endocrine organ tumor includes thyroid cancer.

In some specific embodiments of the present invention, the medicament comprises an active ingredient selected from the group consisting of the polypeptide or a derivative thereof as a single active ingredient, a combination of the polypeptide and a derivative thereof, a combination of the polypeptide or a derivative thereof with other medicament, a conjugate of the polypeptide or a derivative thereof labelled with a chemical or a biomarker, and a solid medium or a semi-solid medium coupled with the polypeptide, the derivative thereof, the conjugate or a combination thereof, and a pharmaceutically acceptable carrier.

The medicaments for the prevention and/or treatment of tumors provided by the present invention contain a safe and effective amount of the polypeptides or derivatives thereof of the present invention. The safe and effective amount refers to the content of the active ingredient that is effective enough to avoid serious side effects when administered to a subject in need within the scope of reasonable medical judgment. Although the safe and effective amount varies with the following factors: the selected polypeptide (for example, considering the structure, stability and half-life of the polypeptide); the selected route of administration; the condition and severity to be treated; the age and body type of the subject to be treated, weight and physical condition; medical history of the subject to be treated; duration of treatment; desired therapeutic effect and similar factors, these can be determined by those skilled in the art in a conventional manner.

The polypeptides or derivatives thereof provided by the present invention can be used directly as active pharmaceutical ingredients, and can also be used to prepare drugs for the prevention and/or treatment of tumors with pharmaceutically acceptable carriers.

In some specific embodiments of the present invention, the pharmaceutically acceptable carrier is selected from the group consisting of a diluent, a filler, an excipient, a binder, a wetting agent, a disintegrant, an effervescent agent, a surfactant, an absorption enhancer, a lubricant, an adsorption carrier, a sustained-release microsphere, an implant, an in-situ microsphere, a liposome, amicroemulsion, an in-situ hydrogel, a nanoparticle, a protease inhibitor, a biological adhesive, a fusion protein, an antibody, a polypeptide and a combination thereof.

In some specific embodiments of the present invention, the dosage form of the medicament may be tablets, injections, capsules, granules, ophthalmic preparations, inhalation preparations, ointments, creams, sprays, aerosols, gels, powders, paints, implants, lotions, or a combination thereof.

In some specific embodiments of the present invention, the route of administration of the drug may be oral administration, pulmonary administration, nasal administration, transdermal administration, ocular administration, intravenous drip, intraperitoneal injection, subcutaneous injection, intramuscular injections, or a combination thereof.

In some specific embodiments of the present invention, the tumor cells include but not limited to: human lung cancer cells (95D), human lung squamous carcinoma cells (NCI-H226), human lung cancer cells (A549), luciferase-labeled human lung cancer cells (A549-luc), human pancreatic cancer cells (PANC-1), human liver/ascites adenocarcinoma cells (SK-HEP-1), human liver cancer cells (HepG2), human breast cancer cells (MDA-MB-453), human breast cancer cells (MDA-MB-231), human breast cancer cells (MCF-7), human melanoma cells (A375), human oral epidermoid cancer cells (KB), human pharyngeal squamous-cell carcinoma cells (Fadu), human colon cancer cells (HCT-116), human thyroid cancer cells (FRO) and human prostate cancer cells (22RV1).

In the present invention, A549-luc cells were purchased from Shanghai Meixuan Biotechnology Co., Ltd., and other tumor cells were purchased from the Cell Bank of the Chinese Academy of Sciences and the American Type Culture Collection (ATCC). Experimental animals used in the present invention were 4-6 weeks old female immunodeficiency mice (BALB/c-nu/nu, Beijing Vital River Laboratory Animal Technology Co., Ltd.). Tumor animal model used in the present invention is subcutaneous xenograft animal model. The number of cells inoculated subcutaneously in mice is about 2×10⁶/200µL. When the volume of the subcutaneous tumor is greater than 100mm³, the mice are randomly divided into groups and subjected to treatment experiments. When the mass volume of the tumors in model control group reaches 2000 mm³ or the animal dies, the treatment ends. The animals are dissected, the tumors are taken out, and the weight and volume of the tumors are measured. During the period of the entire experiment, the mice are observed and the body weight is measured every other day. For the metastatic cancer model, the number of tumor cells inoculated into the tail vein is about 2×10⁶/100×L, and the mice are divided into groups and subjected to treatment experiment one hour after inoculation. The duration of the experiment is 28 days, and the *in vivo* fluorescence imaging of mice lung is performed after the treatment. The mice are observed and the fluorescence intensity of the mouse lungs is measured every other week. Route of administration is intratumoral injection or intravenous injection, and the dosage and frequency of administration are determined according to the design of the specific experiments. Experimental groups are model control group, polypeptides or derivatives thereof treatment groups (N1-N54). Number of experimental animals in each treatment group is five.

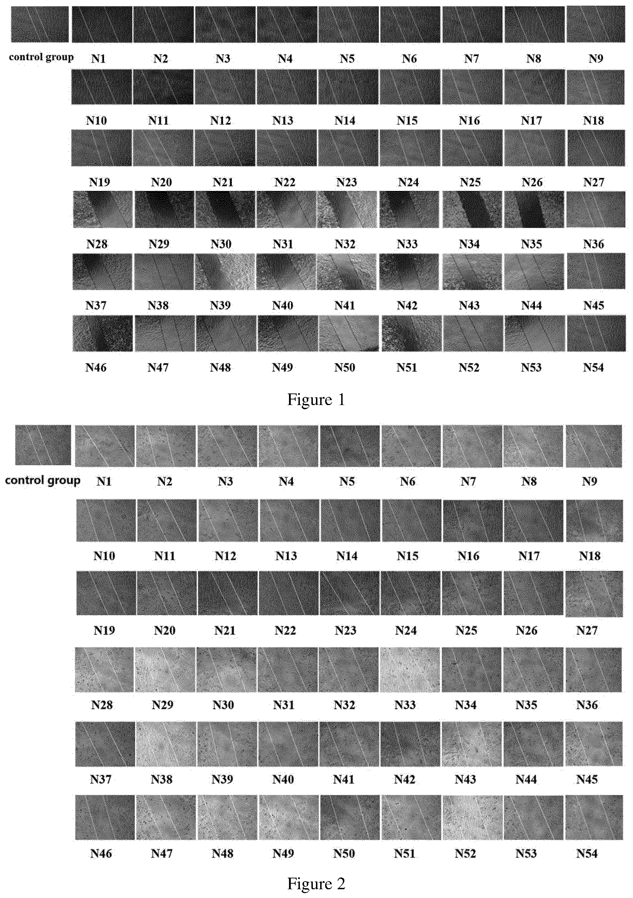

In one embodiment of the present invention, scratch assay is used to evaluate the inhibitory effect of the polypeptide or derivative thereof (N1-N54) on the migration ability of human non-small cell lung cancer cells (A549). The experimental method is as described in Example 2. Figure 1 shows the microscope images of cell migration. Table 1 shows the statistical results of the migration rate (mean ± SD, *P).* The results show that compared to the control group, the migration rate of A549 decreased significantly to different degrees after being treated with 10 µM polypeptide or derivative thereof (N1-N54) for 48 hours, demonstrating that the polypeptide or derivative thereof (N1-N54) inhibits the migration ability of A549 cells.

In one embodiment of the present invention, scratch assay is used to evaluate the inhibitory effect of the polypeptide or derivative thereof (N1-N54) on the migration ability of human lung cancer cells (95D). The experimental method is as described in Example 2. Figure 2 shows the microscope images of cell migration. Table 2 shows the statistical results of the migration rate (mean±SD, *P*). The results show that compared to the control group, the migration rate of 95D decreased significantly to different degrees after being treated with 10 µM polypeptide or derivative thereof for 48 hours, demonstrating that the polypeptide or derivative thereof (N1-N54) inhibits the migration ability of 95D cells.

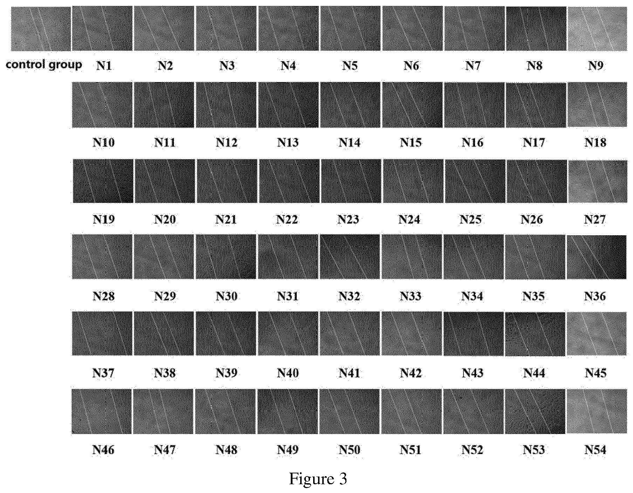

In one embodiment of the present invention, scratch assay is used to evaluate the inhibitory effect of the polypeptide or derivative thereof (N1-N54) on the migration ability of human lung squamous carcinoma cells (NCI-H226). The experimental method is as described in Example 2. Figure 3 shows the microscope images of cell migration. Table 3 shows the statistical results of the migration rate (mean ± SD, *P).* The results show that compared to the control group, the migration rate of NCI-H226 decreased significantly to different degrees after being treated with 10µM polypeptide or derivative thereof for 48 hours, demonstrating that the polypeptide or derivative thereof (N1-N54) inhibits the migration ability of NCI-H226 cells.

In one embodiment of the present invention, efficacy test is carried out in animal model of lung cancer subcutaneous xenograft. The tumor cells are human lung cancer cells A549, and the mode of administration is intratumoral injection. Experimental groups are model control group and treatment groups of polypeptide or derivative thereof (polypeptide sequence: N1-N12). Dosage in N1-N12 treatment group is 5 mg/kg, once every other day. The duration of treatment is 10 days. Table 4 shows the statistical results of the weight and volume of the tumor (mean ± SD, *P).* The results show that compared to the model control group, the weight and volume of the tumor of the N1-N12 treatment groups are decreased significantly to different degrees, demonstrating that the polypeptide or derivative thereof (N1-N12) effectively inhibits the development of tumors derived from human lung cancer cells.

In one embodiment of the present invention, efficacy test is carried out in animal model of lung cancer subcutaneous xenograft. The tumor cells are human lung cancer cells A549, and the mode of administration is intratumoral injection. Experimental groups are model control group and polypeptide or derivative thereof treatment group (polypeptide sequence: N13-N32). Dosage in N13-N32 treatment groups is 5 mg/kg, once every other day. The duration of treatment is 20 days. Table 5 showed the statistical results of the weight and volume of the tumors (mean ± SD, *P).* The results show that compared to the model control group, the weight and volume of the tumors of the N13-N32 treatment groups are decreased significantly to different degrees, demonstrating that the polypeptide or derivative thereof (N13-N32) effectively inhibits the development of tumors derived from human lung cancer cells.

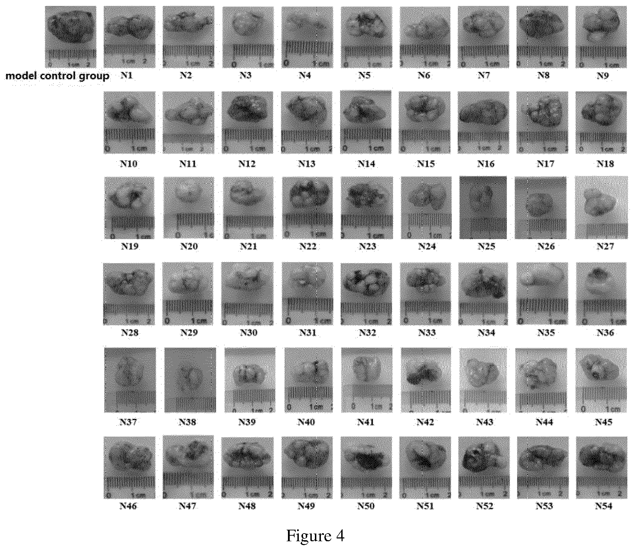

In one embodiment of the present invention, efficacy test is carried out on animal model of lung cancer subcutaneous xenograft. The tumor cells are human lung cancer cells A549, and the mode of administration is intravenous injection. There is a total of three experiments are set up to evaluate N1-N15, N16-N30, and N31-N54 respectively. The experimental procedures, mode of administration, dosage and frequency of administration, and detection methods are consistent in the three experiments. The experimental groups are model control group and polypeptide or derivative thereof treatment groups (N1-N15, N16-N30, or N31-N54). The drug dosage in the N1-N54 treatment groups is 60 mg/kg, once every other day, and the treatment duration is 20 days. The experimental results are shown in Figure 4 and Table 6. Figure 4 shows the representative images of tumors in each group after the treatment. Table 6 shows the statistical results of the weight and volume of tumors (mean ± SD, *P).* The results show that compared to the model control group, the weight and volume of the tumors in the N1-N54 treatment groups are significantly reduced to different degrees, demonstrating that the polypeptide or derivative thereof (N1-N54) of the present invention effectively inhibits the development of tumors derived from human lung cancer cells.

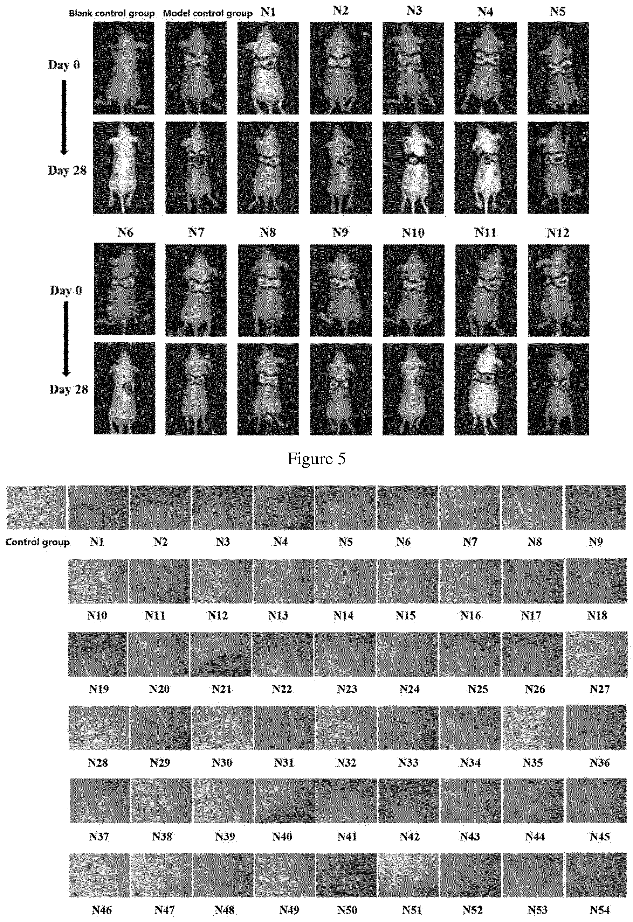

In one embodiment of the present invention, efficacy test is carried out in animal model of lung metastatic cancer. The lung cancer cells are human lung cancer cell line A549-luc, and the mode of administration is intravenous injection. The experimental groups are model control group and polypeptide or derivative thereof treatment groups (N1-N12). The drug dosage of the N1-N12 treatment groups is 30 mg/kg, once every other day. Figure 5 shows the live fluorescence images of the lungs in each group before and after treatment. Compared to the model control group, the fluorescence intensity of the lungs in the N1-N12 treatment groups are reduced to different degrees, demonstrating that the polypeptide or derivative thereof (N1-N12) of the present invention inhibits the development of lung metastatic tumors.

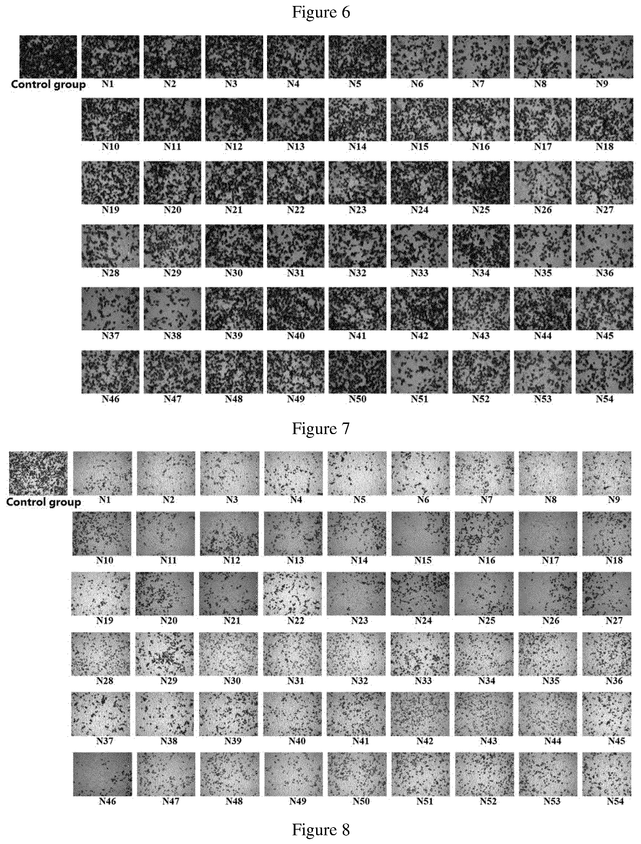

In one embodiment of the present invention, scratch assay is used to evaluate the inhibitory effect of the polypeptide or derivative thereof (N1-N54) on the migration ability of human pancreatic cancer cells (PANC-1). Figure 6 shows the microscope images of cell migration. Table 7 shows the statistical results of the migration rate (mean ± SD, *P).* The results show that compared to the control group, the migration rate of PANC-1 cells decrease significantly to different degrees after being treated with 10 µM polypeptide or derivative thereof for 48 hours, demonstrating that the polypeptide or derivative thereof (N1-N54) inhibits the migration ability of PANC-1 cells.

In one embodiment of the present invention, transwell migration assay is used to evaluate the inhibitory effect of the polypeptide or derivative thereof (N1-N54) on the migration ability of human pancreatic cancer cells (PANC-1). Figure 7 shows the images of the cells that migrate to the lower surface of the semipermeable membrane. Table 8 shows the statistical results of the number of cells that migrate to the lower surface of the semipermeable membrane (mean ± SD, *P).* The results show that compared to the control group, the number of cells migrated to the lower surface of the semipermeable membrane in the N1-N54 treatment groups is significantly less, demonstrating that the polypeptide or derivative thereof (N1-N54) of the present invention inhibits the migration ability of human pancreatic cancer cells.

In one embodiment of the present invention, transwell invasion assay is used to evaluate the inhibitory effect of the polypeptide or derivative thereof (N1-N54) on the invasion ability of human pancreatic cancer cells (PANC-1). Figure 8 shows the images of the cells that migrate to the lower surface of the semipermeable membrane. Table 9 shows the statistical results of the number of cells that migrate to the lower surface of the semipermeable membrane (mean ± SD, *P).* The results show that compared to the control group, the number of cells migrated to the lower surface of the semipermeable membrane in the N1-N54 treatment groups is significantly less, demonstrating that the polypeptide or derivative thereof (N1-N54) of the present invention inhibits the invasion ability of human pancreatic cancer cells.

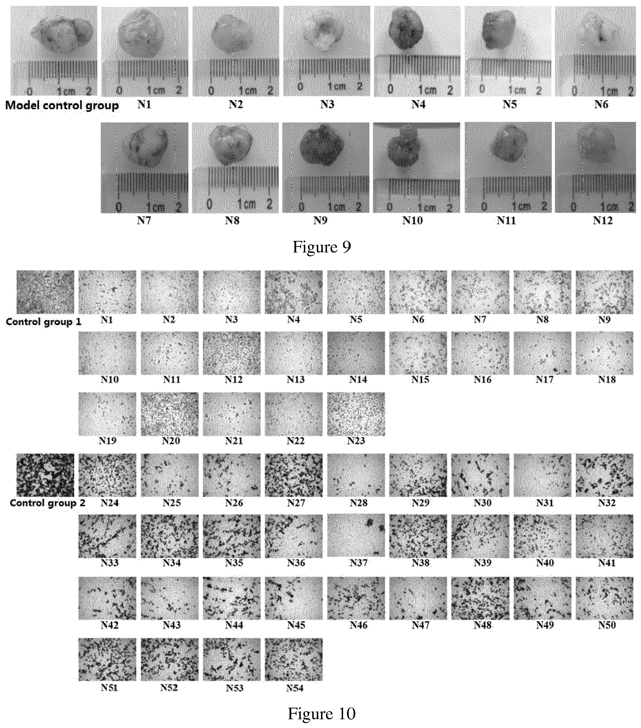

In one embodiment of the present invention, efficacy test is carried out on animal model of pancreatic cancer subcutaneous xenograft. The pancreatic cancer cells are human pancreatic cancer cell line PANC-1, and the mode of administration is intratumoral injection. The experimental groups are model control group and polypeptide or derivative thereof treatment groups (N1-N12). The dosage of N1-N12 treatment groups are 5 mg/kg, once every other day. The duration of treatment is 24 days. The experimental results are shown in Figure 9 and Table 10. Figure 9 shows the images of tumors in each group after treatment. Table 10 shows the statistical results of the weight and volume of the tumors. The results show that compared to the model control group, the weight and volume of the tumors in the N1-N12 treatment groups are decreased significantly to different degrees, demonstrating that the polypeptide or derivative thereof (N1-N12) effectively inhibits the development of tumors derived from human pancreatic cancer cells.

In one embodiment of the present invention, transwell migration assay is used to evaluate the inhibitory effect of the polypeptide or derivative thereof (N1-N54) on the migration ability of human liver adenocarcinoma cells (SK-HEP-1). Figure 10 shows the images of the cells that migrate to the lower surface of the semipermeable membrane. Table 11 shows the statistical results of the number of cells that migrate to the lower surface of the semipermeable membrane (mean ± SD, *P).* The results show that compared to the control group, the number of cells migrate to the lower surface of the semipermeable membrane in the N1-N54 treatment groups is significantly less, demonstrating that the polypeptide or derivative thereof (N1-N54) of the present invention inhibits the migration ability of human liver cancer cells.

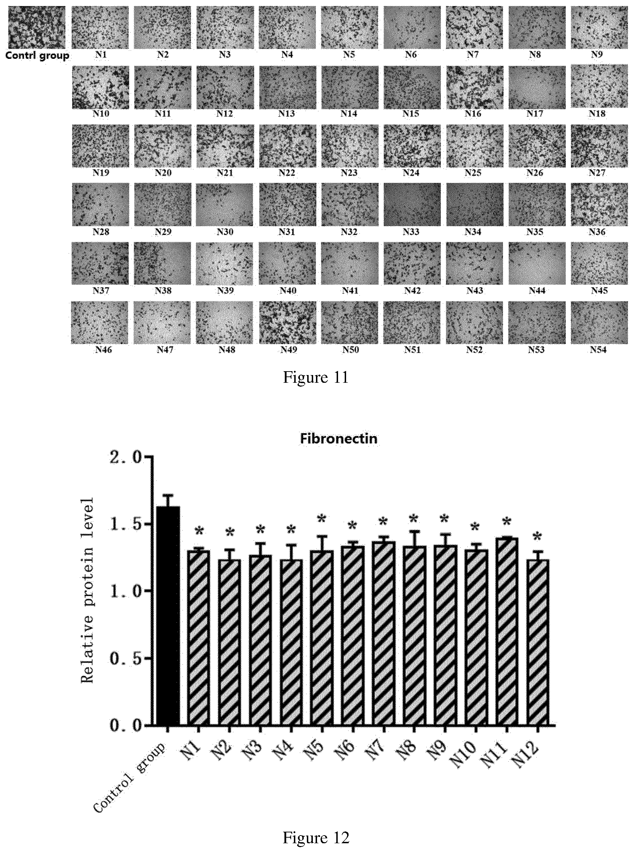

In one embodiment of the present invention, transwell invasion assay is used to evaluate the inhibitory effect of the polypeptide or derivative thereof (N1-N54) on the invasion ability of human liver adenocarcinoma cells (SK-HEP-1). Figure 11 shows the images of the cells that migrate to the lower surface of the semipermeable membrane. Table 12 shows the statistical results of the number of cells migrate to the lower surface of the semipermeable membrane (mean ± SD, *P).* The results show that compared to the control group, the number of cells migrate to the lower surface of the semipermeable membrane in the N1-N54 treatment groups is significantly less, demonstrating that the polypeptide or derivative thereof (N1-N54) of the present invention inhibits the invasion ability of human liver cancer cells.

In one embodiment of the present invention, the ability of inhibiting the fibronectin expression in human liver adenocarcinoma cells (SK-HEP-1) is tested. SK-HEP-1 cells are cultured *in vitro,* cells in logarithmic growth phase are collected, and the cells are cultured evenly in a 24-well plate at a density of 1-3×10⁵ cells/well. The cells are cultured in a cell incubator for 4-5h. PBS is added to the control group. PBS containing polypeptide or derivative thereof (N1-N12) is added to the treatment groups and the final concentration of the polypeptide or derivative thereof in the well is 10 µM. After incubating for 24 hours, the cells are collected and the total protein was extracted. ELISA is used to detect the content of fibronectin in total protein. The results are shown in Figure 12 and Table 13. The fibronectin level in the N1-N12 treatment groups is significantly less than the control group, demonstrating that the polypeptide or derivative thereof (N1-N12) of the present invention inhibits the expression of fibronectin in human liver cancer cells.

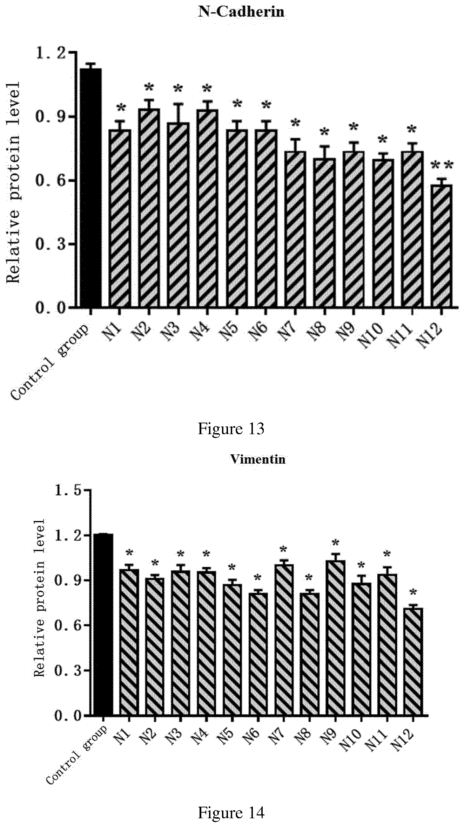

In one embodiment of the present invention, the ability of inhibiting the expression of N-cadherin in human liver adenocarcinoma cells (SK-HEP-1) by polypeptide or derivative thereof (N1-N12) is tested. The results are shown in Figure 13 and Table 14. Compared to the control group, the N-cadherin level in the N1-N12 treatment groups decreases significantly, demonstrating that the polypeptide or derivative thereof (N1-N12) of the present invention inhibits the expression of N-calcium in human liver cancer cells.

In one embodiment of the present invention, the ability of inhibiting the expression of vimentin in human liver adenocarcinoma cells (SK-HEP-1) by polypeptide or derivative thereof (N1-N12) is tested. The results are shown in Figure 14 and Table 15. Compared to the control group, the vimentin level in the N1-N12 treatment groups decreases significantly, demonstrating that the polypeptide or derivative thereof (N1-N12) of the present invention inhibits the expression of vimentin in human liver cancer cells.

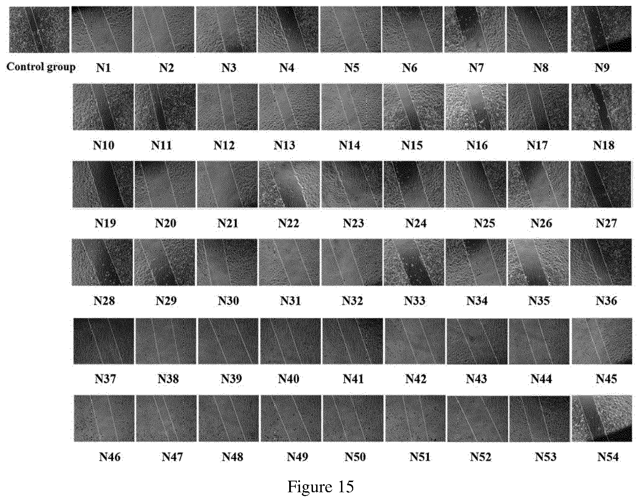

In one embodiment of the present invention, scratch assay is used to evaluate the inhibitory effect of the polypeptide or derivative thereof (N1-N54) on the migration ability of human liver cancer cells (HepG2). Figure 15 shows the microscope images of cell migration. Table 16 shows the statistical results of the migration rate (mean ± SD, *P).* The results show that compared to the control group, the migration rate of HepG2 cells decreases significantly to different degrees after the treatment with the polypeptide or derivative thereof for 48 hours, demonstrating that the polypeptide or derivative thereof (N1-N54) inhibits the migration ability of HepG2 cells.

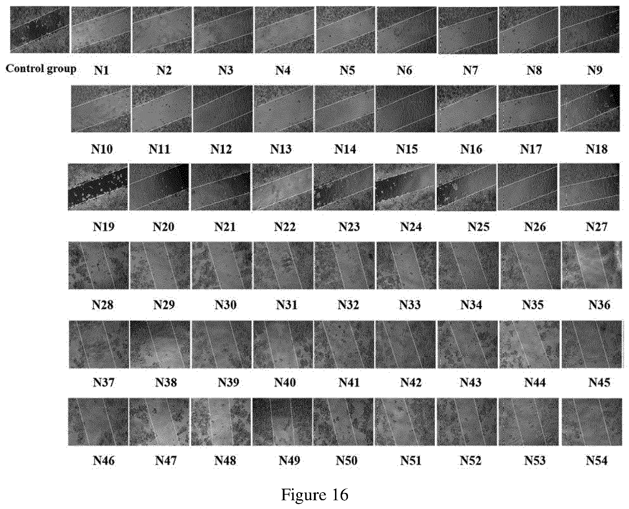

In one embodiment of the present invention, scratch assay is used to evaluate the inhibitory effect of the polypeptide or derivative thereof (N1-N54) on the migration ability of human breast cancer cells (MDA-MB-453). Figure 16 shows the microscope images of cell migration. Table 17 shows the statistical results of the migration rate (mean ± SD, *P).* The results show that compared to the control group, the migration rate of MDA-MB-453 cells decreases significantly to different degrees after the treatment with the polypeptide or derivative thereof for 48 hours, demonstrating that the polypeptide or derivative thereof (N1-N54) inhibits the migration ability of MDA-MB-453 cells.

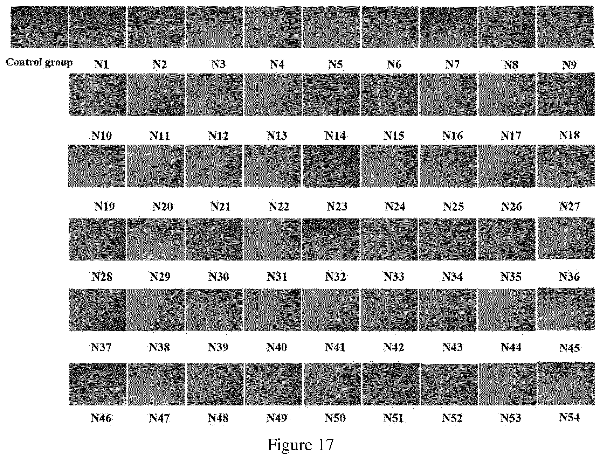

In one embodiment of the present invention, scratch assay is used to evaluate the inhibitory effect of the polypeptide or derivative thereof (N1-N54) on the migration ability of human breast cancer cells (MDA-MB-231). Figure 17 shows the microscope images of cell migration. Table 18 shows the statistical results of the migration rate (mean ± SD, *P).* The results show that compared to the control group, the migration rate of MDA-MB-231 cells decreases significantly to different degrees after the treatment with the polypeptide or derivative thereof for 48 hours, demonstrating that the polypeptide or derivative thereof (N1-N54) inhibits the migration ability of MDA-MB-231 cells.

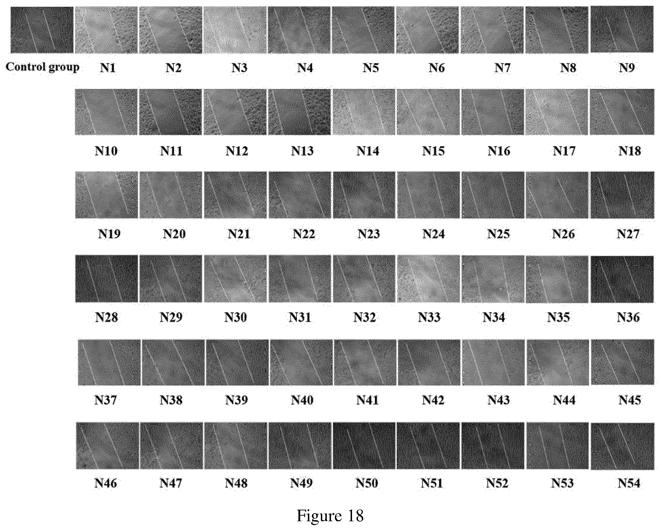

In one embodiment of the present invention, scratch assay is used to evaluate the inhibitory effect of the polypeptide or derivative thereof (N1-N54) on the migration ability of human breast cancer cells (MCF-7). Figure 18 shows the microscope images of cell migration. Table 19 shows the statistical results of the migration rate (mean ± SD, *P).* The results show that compared to the control group, the migration rate of MCF-7 cells decreases significantly to different degrees after the treatment with the polypeptide or derivative thereof for 48 hours, demonstrating that the polypeptide or derivative thereof (N1-N54) inhibits the migration ability of MCF-7 cells.

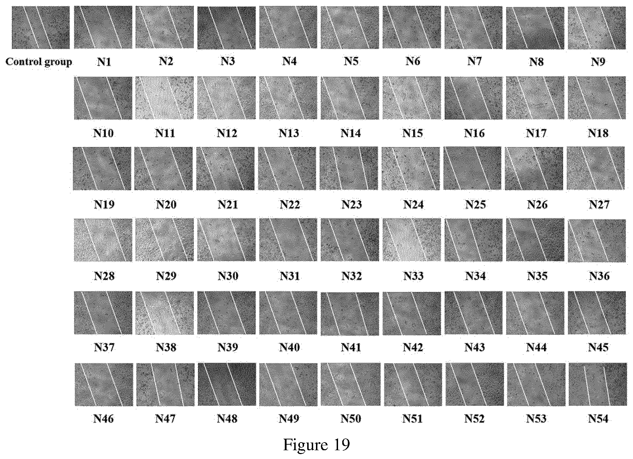

In one embodiment of the present invention, scratch assay is used to evaluate the inhibitory effect of the polypeptide or derivative thereof (N1-N54) on the migration ability of human melanoma cells (A375). Figure 19 shows the microscope images of cell migration. Table 20 shows the statistical results of the migration rate (mean ± SD, *P).* The results showed that compared to the control group, the migration rate of A375 cells decreases significantly to different degrees after the treatment with the polypeptide or derivative thereof for 48 hours, demonstrating that the polypeptide or derivative thereof (N1-N54) inhibits the migration ability of A375 cells.

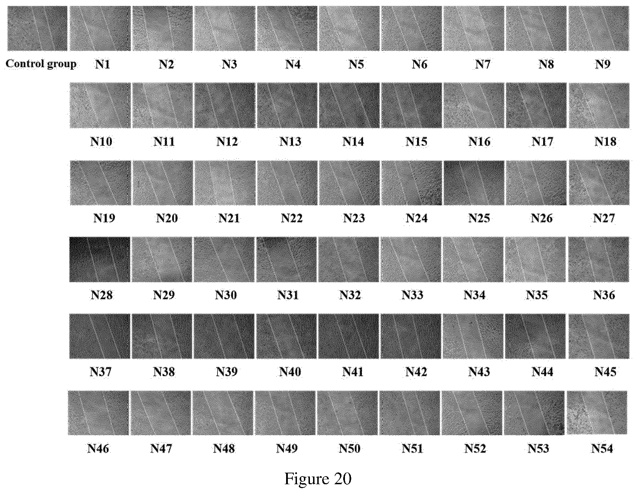

In one embodiment of the present invention, scratch assay is used to evaluate the inhibitory effect of the polypeptide or derivative thereof (N1-N54) on the migration ability of human oral epidermoid carcinoma KB cells. Figure 20 shows the microscope images of cell migration. Table 21 shows the statistical results of the migration rate (mean ± SD, *P).* The results show that compared to the control group, the migration rate of KB cells decreases significantly to different degrees after the treatment with the polypeptide or derivative thereof for 48 hours, demonstrating that the polypeptide or derivative thereof (N1-N54) inhibits the migration ability of KB cells.

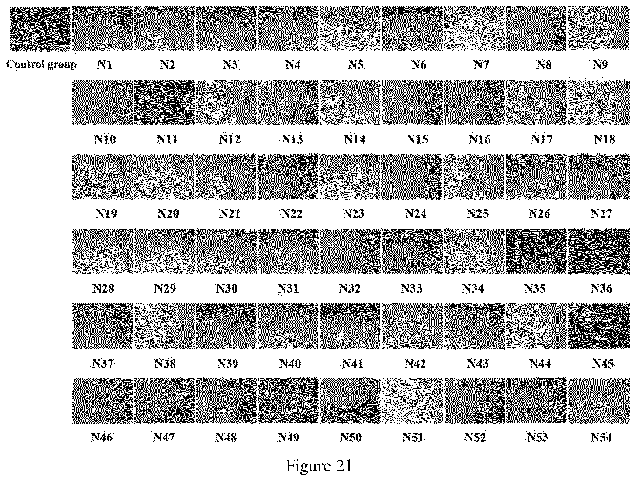

In one embodiment of the present invention, scratch assay is used to evaluate the inhibitory effect of the polypeptide or derivative thereof (N1-N54) on the migration ability of human pharyngeal squamous-cell carcinoma cells (Fadu). Figure 21 shows the microscope images of cell migration. Table 22 shows the statistical results of the migration rate (mean ± SD, *P).* The results show that compared to the control group, the migration rate of Fadu cells decreases significantly to different degrees after treating with 10 µM of the polypeptide or derivative thereof for 48 hours, showing the polypeptide or derivative thereof (N1-N54) inhibits the migration ability of Fadu cells.

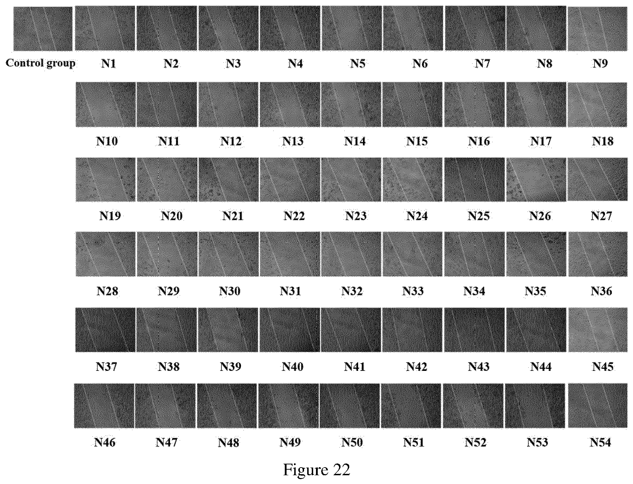

In one embodiment of the present invention, scratch assay is used to evaluate the inhibitory effect of the polypeptide or derivative thereof (N1-N54) on the migration ability of human colon cancer cells (HCT-116). Figure 22 shows the microscope images of cell migration. Table 23 showed the results of migration rate statistics (mean ± SD, *P).* The results showed that compared to the control group, the migration rate of HCT-116 decreases significantly to different degrees after treating with 10 µM of polypeptide or derivative thereof for 48 hours, demonstrating that the polypeptide or derivative thereof (N1-N54) inhibits the migration ability of HCT-116 cells.

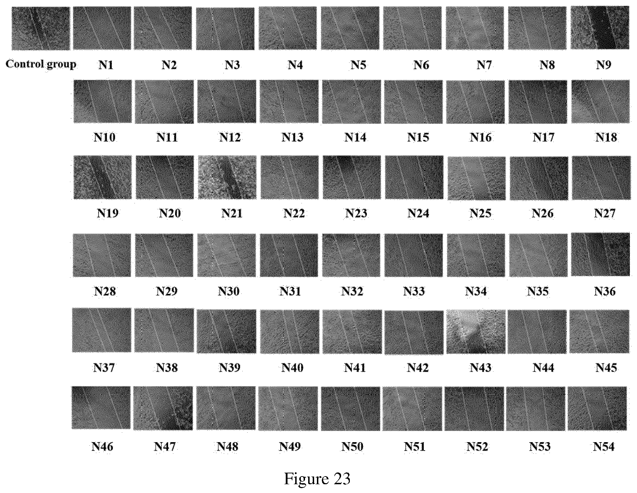

In one embodiment of the present invention, scratch assay is used to evaluate the inhibitory effect of the polypeptide or derivative thereof (N1-N54) on the migration ability of human thyroid cancer cells (FRO). Figure 23 shows the microscope images of cell migration. Table 24 showed the statistical results of the migration rate (mean ± SD, *P).* The results showed that compared to the control group, the migration rate of FRO decreases significantly to different degrees after treating with 10 µM of polypeptide or derivative thereof for 48 hours, showing the polypeptide or derivative thereof (N1-N54) inhibits the migration ability of FRO cells.

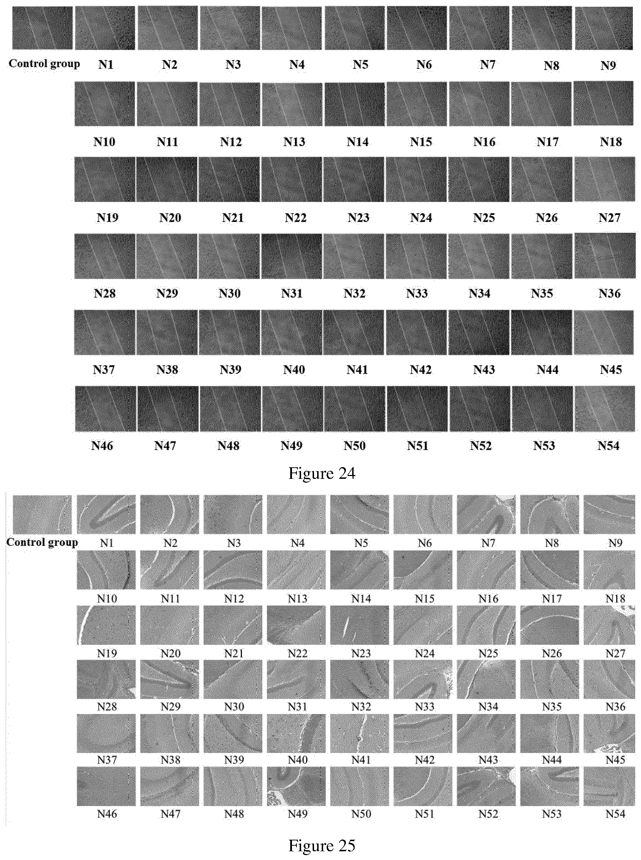

In one embodiment of the present invention, scratch assay is used to evaluate the inhibitory effect of the polypeptide or derivative thereof (N1-N54) on the migration ability of human prostate cancer cells (22RV1). Figure 24 shows the microscope images of cell migration. Table 25 shows the statistical results of the migration rate. The results show that compared to the control group, the migration rate of 22RV1 cells decreases significantly to different degrees after treating with 10 µM of polypeptide or derivative thereof for 48 hours, demonstrating that the polypeptide or derivative thereof (N1-N54) inhibits the migration ability of 22RV1 cells.

In one embodiment of the present invention, high-dose acute toxicity test is carried out. The results show that intravenous injection of any one of N1-N54 would not cause toxicity to mouse organs.

In one embodiment of the present invention, the effect of the polypeptide or derivative thereof on the coagulation function of mice is tested. The results are shown in Table 26. The activated coagulation time (ACT) in the treatment groups of N1 to N12 and the control group does not change significantly, indicating that polypeptide drugs have no influence on the coagulation function of mice.

In one embodiment of the present invention, immunogenicity of the drugs is tested. The results are shown in Table 27. Compared with the control group, the IgG content in each treatment group of N1- N12 on the 14th and 25th day after administration has no significant change, indicating that the polypeptide drugs barely have immunogenicity in the body.

### BRIEF DESCRIPTION OF DRAWINGS

In order to explain the embodiments of the present invention or the technical solutions in the prior art more clearly, the followings are brief introduce of the drawings that need to be used in the description.
Figure 1 shows the microscope images of cell migration results of human lung cancer cells (A549) in Example 6 (scratch assay), in which the inhibitory effect of the polypeptide and its derivatives (N1-N54) on the migration ability of human lung cancer cells (A549) is tested. The results demonstrate that the polypeptide and its derivatives (N1-N54) significantly inhibit the migration of human lung cancer cells. There are two types of group in the figure: the control group and the polypeptide or its derivative treatment group (N1-N54). The drug dosage of the treatment group is 10 µM and the treatment time is 48 hours.
Figure 2 shows the microscope images of cell migration results of human lung cancer cells (95D) in Example 7 (scratch assay), in which the inhibitory effect of the polypeptide and its derivatives (N1-N54) on the migration ability of human lung cancer cells (95D) is tested. The results demonstrate that the polypeptide and its derivatives (N1-N54) significantly inhibit the migration of human lung cancer cells. There are two types of group in the figure: the control group and the polypeptide or its derivative treatment group (N1-N54). The drug dosage of the treatment group is 10 µM and the treatment time is 48 hours.
Figure 3 shows the microscope images of cell migration results of human lung squamous-cell carcinoma cells (NCI-H226) in Example 8 (scratch assay), in which the inhibitory effect of the polypeptide and its derivatives (N1-N54) on the migration ability of human lung squamous-cell carcinoma cells (NCI-H226) is tested. The results demonstrate that the polypeptide and its derivatives (N1-N54) significantly inhibit the migration of human lung cancer cells. There are two types of group in the figure: the control group and the polypeptide or its derivative treatment group (N1-N54). The drug dosage of the treatment group (N1-N54) is 10 µM and the treatment time is 48 hours.
Figure 4 shows the images of the subcutaneous tumors after being peeled from the experimental animal in Example 11, in which the polypeptide or its derivatives (N1-N54) is administered by intravenous injection and the inhibitory effect on the growth of subcutaneous tumor derived from lung cancer cells is shown. The results show that the polypeptide and derivatives thereof (N1-N54) significantly inhibit the growth of tumor derived from lung cancer cells. There are two types of group in the figure: the control group and the polypeptide or its derivative treatment group (N1-N54). The drug dosage of the treatment group (N1-N54) is 60 mg/kg, and the administration frequency is once every other day.
Figure 5 shows the results of the live fluorescence imaging of the experimental animals in Example 12, in which the polypeptide or its derivatives (N1-N12) is administered by intravenous injection and the inhibitory effect on the growth of metastatic tumor derived from lung cancer cells is shown. The results show that the polypeptide and derivatives thereof (N1-N12) significantly inhibit the formation of lung tumor. There are two types of group in the figure: the control group and the polypeptide or its derivative treatment group (N1-N12). The drug dosage of the treatment group (N1-N12) is 60 mg/kg, and the administration frequency is once every other day.
Figure 6 shows the microscope images of cell migration results of human pancreatic cancer cells (PANC-1) in Example 13 (scratch assay), in which the inhibitory effect of the polypeptide and its derivatives (N1-N54) on the migration ability of human pancreatic cancer cells (PANC-1) is tested. The results demonstrate that the polypeptide and its derivatives (N1-N54) significantly inhibit the migration of human pancreatic cancer cells. There are two types of group in the figure: the control group and the polypeptide or its derivative treatment group (N1-N54). The drug dosage of the treatment group is 10 µM and the treatment time is 48 hours.
Figure 7 shows the microscope images of cell migration results of human pancreatic cancer cells (PANC-1) in Example 14 (transwell migration assay), in which the inhibitory effect of the polypeptide and its derivatives (N1-N54) on the migration ability of human pancreatic cancer cells (PANC-1) is tested. The results demonstrate that the polypeptide and its derivatives (N1-N54) significantly inhibit the migration of human pancreatic cancer cells. There are two types of group in the figure: the control group and the polypeptide or its derivative treatment group (N1-N54). The drug dosage of the treatment group (N1-N54) is 10 µM and the treatment time is 24 hours.
Figure 8 shows the microscope images of cell invasion results of human pancreatic cancer cells (PANC-1) in Example 15 (transwell invasion assay), in which the inhibitory effect of the polypeptide and its derivatives (N1-N54) on the invasion ability of human pancreatic cancer cells (PANC-1) is tested. The results demonstrate that the polypeptide and its derivatives (N1-N54) significantly inhibit the invasion of human pancreatic cancer cells. There are two types of group in the figure: the control group and the polypeptide or its derivative treatment group (N1-N54). The drug dosage of the treatment group (N1-N54) is 10 µM and the treatment time is 24 hours.
Figure 9 shows the images of the subcutaneous tumors after being peeled from the experimental animal in Example 16, in which the polypeptide or its derivatives (N1-N12) is administered by intratumoral injection and the inhibitory effect on the growth of subcutaneous tumor derived from pancreatic cancer cells is shown. The results show that the polypeptide and derivatives thereof (N1-N12) significantly inhibit the growth of tumor derived from pancreatic cancer cells. There are two types of group in the figure: the control group and the polypeptide or its derivative treatment group (N1-N12). The drug dosage of the treatment group (N1-N12) is 60 mg/kg, and the administration frequency is once every other day.
Figure 10 shows the microscope images of cell migration results of human liver adenocarcinoma cells (SK-HEP-1) in Example 17 (transwell migration assay), in which the inhibitory effect of the polypeptide and its derivatives (N1-N54) on the migration ability of human liver adenocarcinoma cells (SK-HEP-1) is tested. The results demonstrate that the polypeptide and its derivatives (N1-N54) significantly inhibit the migration of human liver adenocarcinoma cells. There are two types of group in the figure: the control group and the polypeptide or its derivative treatment group (N1-N54). The drug dosage of the treatment group (N1-N54) is 10 µM and the treatment time is 24 hours.
Figure 11 shows the microscope images of cell invasion results of human liver adenocarcinoma cells (SK-HEP-1) in Example 18 (transwell invasion assay), in which the inhibitory effect of the polypeptide and its derivatives (N1-N54) on the invasion ability of human liver adenocarcinoma cells (SK-HEP-1) is tested. The results demonstrate that the polypeptide and its derivatives (N1-N54) significantly inhibit the invasion of human pancreatic cancer cells. There are two types of group in the figure: the control group and the polypeptide or its derivative treatment group (N1-N54). The drug dosage of the treatment group (N1-N54) is 10 µM and the treatment time is 24 hours.
Figure 12 shows the results of the detection of the fibronectin expression in human liver adenocarcinoma cells (SK-HEP-1) in Example 19, in which the inhibitory effect of the polypeptide and derivatives thereof (N1-N12) on the fibronectin expression of human liver adenocarcinoma cells (SK-HEP-1) is shown. The results demonstrate that the polypeptide and its derivatives (N1-N12) significantly inhibit fibronectin expression in liver cancer cells. There are two types of group in the figure: the control group and the polypeptide or its derivative treatment group (N1-N12). The drug dosage of the treatment group (N1-N12) is 10 µM and the treatment time is 24 hours.
Figure 13 shows the results of the detection of the N-cadherin expression in human liver adenocarcinoma cells (SK-HEP-1) in Example 20, in which the inhibitory effect of the polypeptide and derivatives thereof (N1-N12) on the N-cadherin expression of human liver adenocarcinoma cells (SK-HEP-1) is shown. The results demonstrate that the polypeptide and its derivatives (N1-N12) significantly inhibit N-cadherin expression in liver cancer cells. There are two types of group in the figure: the control group and the polypeptide or its derivative treatment group (N1-N12). The drug dosage of the treatment group (N1-N12) is 10 µM and the treatment time is 24 hours.
Figure 14 shows the results of the detection of the vimentin expression in human liver adenocarcinoma cells (SK-HEP-1) in Example 21, in which the inhibitory effect of the polypeptide and derivatives thereof (N1-N12) on the vimentin expression of human liver adenocarcinoma cells (SK-HEP-1) is shown. The results demonstrate that the polypeptide and its derivatives (N1-N12) significantly inhibit vimentin expression in liver cancer cells. There are two types of group in the figure: the control group and the polypeptide or its derivative treatment group (N1-N12). The drug dosage of the treatment group (N1-N12) is 10 µM and the treatment time is 24 hours.
Figure 15 shows the microscope images of cell migration results of human liver cancer cells (HepG2) in Example 22 (scratch assay), in which the inhibitory effect of the polypeptide and its derivatives (N1-N54) on the migration ability of human liver cancer cells (HepG2) is tested. The results demonstrate that the polypeptide and its derivatives (N1-N54) significantly inhibit the migration of human liver cancer cells. There are two types of group in the figure: the control group and the polypeptide or its derivative treatment group (N1-N54). The drug dosage of the treatment group is 10 µM and the treatment time is 48 hours.
Figure 16 shows the microscope images of cell migration results of human breast cancer cells (MDA-MB-453) in Example 23 (scratch assay), in which the inhibitory effect of the polypeptide and its derivatives (N1-N54) on the migration ability of human breast cancer cells (MDA-MB-453) is tested. The results demonstrate that the polypeptide and its derivatives (N1-N54) significantly inhibit the migration of human breast cancer cells. There are two types of group in the figure: the control group and the polypeptide or its derivative treatment group (N1-N54). The drug dosage of the treatment group is 10 µM and the treatment time is 48 hours.
Figure 17 shows the microscope images of cell migration results of human breast cancer cells (MDA-MB-231) in Example 24 (scratch assay), in which the inhibitory effect of the polypeptide and its derivatives (N1-N54) on the migration ability of human breast cancer cells (MDA-MB-231) is tested. The results demonstrate that the polypeptide and its derivatives (N1-N54) significantly inhibit the migration of human breast cancer cells. There are two types of group in the figure: the control group and the polypeptide or its derivative treatment group (N1-N54). The drug dosage of the treatment group is 10 µM and the treatment time is 48 hours.
Figure 18 shows the microscope images of cell migration results of human breast cancer cells (MCF-7) in Example 25 (scratch assay), in which the inhibitory effect of the polypeptide and its derivatives (N1-N54) on the migration ability of human breast cancer cells (MCF-7) is tested. The results demonstrate that the polypeptide and its derivatives (N1-N54) significantly inhibit the migration of human breast cancer cells. There are two types of group in the figure: the control group and the polypeptide or its derivative treatment group (N1-N54). The drug dosage of the treatment group is 10 µM and the treatment time is 48 hours.
Figure 19 shows the microscope images of cell migration results of human melanoma cells (A375) in Example 26 (scratch assay), in which the inhibitory effect of the polypeptide and its derivatives (N1-N54) on the migration ability of human melanoma cells (A375) is tested. The results demonstrate that the polypeptide and its derivatives (N1-N54) significantly inhibit the migration of human melanoma cells. There are two types of group in the figure: the control group and the polypeptide or its derivative treatment group (N1-N54). The drug dosage of the treatment group is 10 µM and the treatment time is 48 hours.
Figure 20 shows the microscope images of cell migration results of human oral epidermoid carcinoma KB cells in Example 27 (scratch assay), in which the inhibitory effect of the polypeptide and its derivatives (N1-N54) on the migration ability of human oral epidermoid carcinoma KB cells is tested. The results demonstrate that the polypeptide and its derivatives (N1-N54) significantly inhibit the migration of human oral epidermoid carcinoma cells. There are two types of group in the figure: the control group and the polypeptide or its derivative treatment group (N1-N54). The drug dosage of the treatment group is 10 µM and the treatment time is 48 hours.
Figure 21 shows the microscope images of cell migration results of human pharyngeal squamous cell carcinoma cells (Fadu) in Example 28 (scratch assay), in which the inhibitory effect of the polypeptide and its derivatives (N1-N54) on the migration ability of human pharyngeal squamous cell carcinoma cells (Fadu) is tested. The results demonstrate that the polypeptide and its derivatives (N1-N54) significantly inhibit the migration of human pharyngeal squamous cell carcinoma cells. There are two types of group in the figure: the control group and the polypeptide or its derivative treatment group (N1-N54). The drug dosage of the treatment group is 10 µM and the treatment time is 48 hours.
Figure 22 shows the microscope images of cell migration results of human colon cancer cells (HCT 116) in Example 29 (scratch assay), in which the inhibitory effect of the polypeptide and its derivatives (N1-N54) on the migration ability of human colon cancer cells (HCT 116) is tested. The results demonstrate that the polypeptide and its derivatives (N1-N54) significantly inhibit the migration of human colon cancer cells. There are two types of group in the figure: the control group and the polypeptide or its derivative treatment group (N1-N54). The drug dosage of the treatment group is 10 µM and the treatment time is 48 hours.
Figure 23 shows the microscope images of cell migration results of human thyroid cancer cells (FRO) in Example 30 (scratch assay), in which the inhibitory effect of the polypeptide and its derivatives (N1-N54) on the migration ability of human thyroid cancer cells (FRO) is tested. The results demonstrate that the polypeptide and its derivatives (N1-N54) significantly inhibit the migration of human thyroid cancer cells. There are two types of group in the figure: the control group and the polypeptide or its derivative treatment group (N1-N54). The drug dosage of the treatment group is 10 µM and the treatment time is 48 hours.
Figure 24 shows the microscope images of cell migration results of human prostate cancer cells (22RV1) in Example 31 (scratch assay), in which the inhibitory effect of the polypeptide and its derivatives (N1-N54) on the migration ability of human prostate cancer cells (22RV1) is tested. The results demonstrate that the polypeptide and its derivatives (N1-N54) significantly inhibit the migration of human prostate cancer cells. There are two types of group in the figure: the control group and the polypeptide or its derivative treatment group (N1-N54). The drug dosage of the treatment group is 10 µM and the treatment time is 48 hours.
Figure 25 shows the images of the brain tissue sections from the experimental animals in Example 32. The results show that the polypeptide and derivatives thereof (N1-N54) of the present invention have no drug toxicity to the mouse brain.

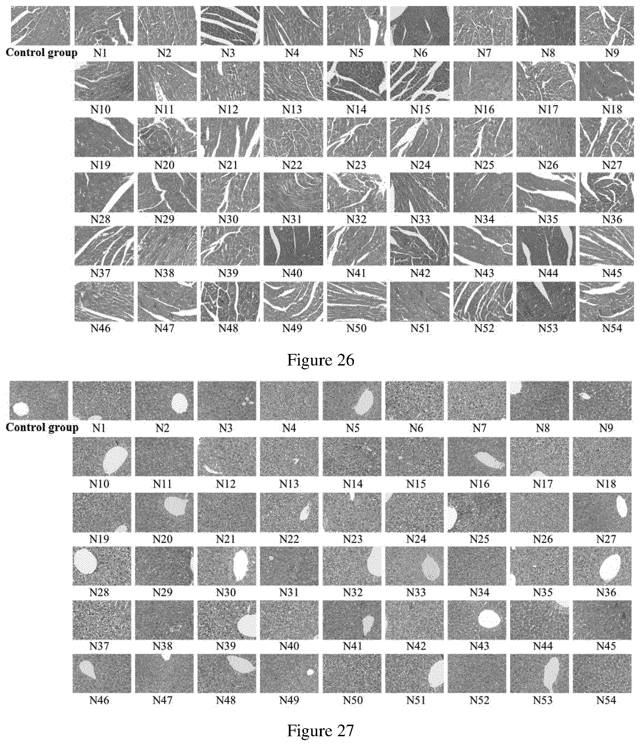

Figure 26 shows the images of the heart tissue sections from the experimental animals in Example 32. The results show that the polypeptide and derivatives thereof (N1-N54) of the present invention have no drug toxicity to the mouse heart.
Figure 27 shows the images of the liver tissue sections from the experimental animals in Example 32. The results show that the polypeptide and derivatives thereof (N1-N54) of the present invention have no drug toxicity to the mouse liver.

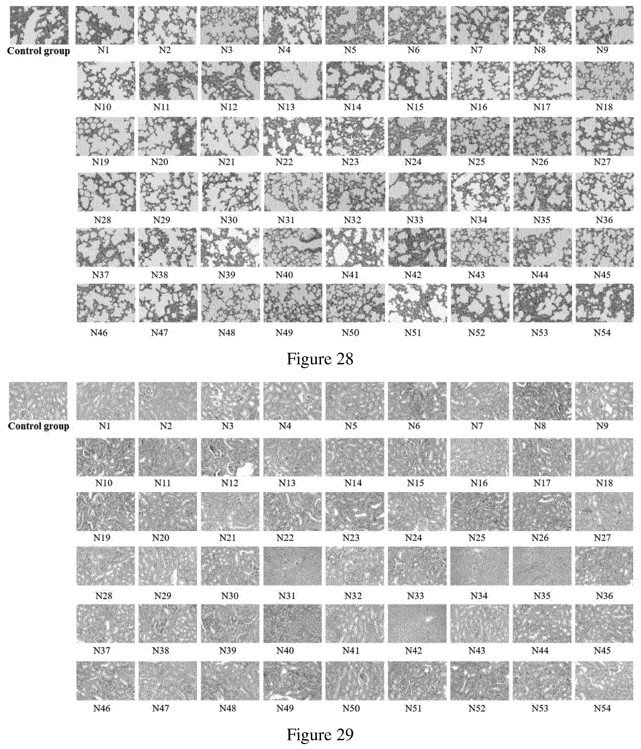

Figure 28 shows the images of the lung tissue sections from the experimental animals in Example 32. The results show that the polypeptide and derivatives thereof (N1-N54) of the present invention have no drug toxicity to the mouse lung.
Figure 29 shows the images of the kidney tissue sections from the experimental animals in Example 32. The results show that the polypeptide and derivatives thereof (N1-N54) of the present invention have no drug toxicity to the mouse kidney.

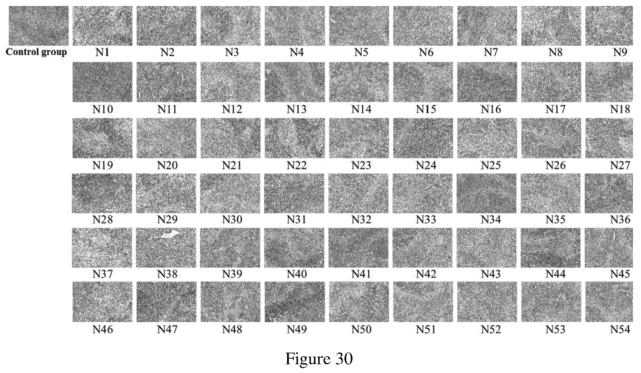

Figure 30 shows the images of the spleen tissue sections from the experimental animals in Example 32. The results show that the polypeptide and derivatives thereof (N1-N54) of the present invention have no drug toxicity to the mouse spleen.

### DETAILED DESCRIPTION

The present invention discloses the uses of polypeptides or derivatives thereof. The skilled person in the art can learn from the content of this article and improve the process parameters appropriately. In particular, it should be pointed out that all similar substitutions and modifications are obvious to those skilled in the art, and they are all deemed to be included in the present invention. The method and use of the present invention have been described through the preferred embodiments.

Unless otherwise specified, all scientific and technological terms used herein have the same meaning as understood by those of ordinary skill in the art. For definitions and terms in this field, professionals can refer to Current Protocols in Molecular Biology (Ausubel). The abbreviation for amino acid residues is the standard 3-letter and/or 1-letter code used in the art to refer to one of the 20 commonly used L-amino acids.

The reagents used in the present invention are all available in the market.

In conjunction with the following examples, the present invention is further illustrated.

### Example 1 Preparation of polypeptides

The peptides used in the examples were synthesized by a solid-phase peptide synthesizer. The Fmoc-protected resin was used as the starting material, and the solid-phase synthesis method was used to couple amino acids according to the amino acid sequence to synthesize the fully protected peptide on the resin. All amino acids used were L-amino acids. The peptide resin was cleaved by common cleavage reagents. The peptides were cleaved from the resin, and the side chain protecting groups were removed. After centrifugation and drying, the crude peptides were obtained. Using preparative HPLC, the crude peptides were purified and the specific fractions were collected. After freeze-drying, the peptide products were obtained.

PEG modification method: mPEG-SC and polypeptides (molar ratio 1.5-2.0:1) were weighed and added into 40 mL-100 mLPBS buffer (pH 5-8.5), reacted overnight at 4°C. The reaction products were purified by semi-preparative high-performance liquid chromatographic method. The target products were collected, pre-frozen in a cryogenic refrigerator at -70°C overnight, and then lyophilized in a freeze dryer for about 30 hours to obtain the PEG-modified polypeptides.

### Example 2 Efficacy test of inhibiting tumor cell migration-scratch assay

In the scratch assay, the cell migration rate was negatively correlated with the inhibitory effect of the drug component on tumor cell migration ability. The cell migration inhibition experiment was described as follows: tumor cells were cultured *in vitro,* cells in logarithmic growth phase were collected and seeded evenly in a 24-well plate at a density of 1-2×10⁵ cells/well; the cells were cultured in a cell incubator until a monolayer was formed; a scratch tool was used to make a "-" (straight) scratch on the monolayer of cells; the cells were washed 1-3 times with PBS, and a medium (with 2% fetal bovine serum) containing specific test components was added to the culture. Experimental grouping: PBS control group; polypeptide or derivative thereof treatment group (N1-N54), 10 µM. Three independent parallel wells were set up in each group, and the cells were cultured continuously in a cell incubator for 48 hours. The cells were observed with an inverted microscope at 0 h and 48 h respectively and images were taken. At least 3 representative images for each well were taken. ImageJ software was used to count the migration distance of the cells, and the cell migration rate was calculated as: cell migration rate (%) = (width of original scratch - width of current scratch)/ width of original scratch ×100.

### Example 3 Efficacy test of inhibiting tumor cell migration-Transwell cell migration assay

The Transwell chamber system was used in the transwell cell migration experiment. The chamber has an upper layer and a lower layer, which are separated by an artificial semipermeable membrane in the middle. Cells that have migrated through the semi-permeable membrane will attach to the lower surface of the semi-permeable membrane. The migration ability is evaluated by counting the number of cells on the lower surface of the semi-permeable membrane. The number of cells that have migrated is negatively correlated with the inhibitory effect of the drug on the migration ability of the tumor cells. Transwell migration assay was performed as follows: tumor cells were cultured *in vitro,* cells in logarithmic growth phase were collected, and cell suspension was prepared with fresh serum-free medium at a density of 2-3×10⁵ cells/mL. Cells were inoculated, 100 µL/well cell suspension (serum-free medium) in the upper chamber and 600 µL/well complete culture medium in the lower chamber. After incubated in 37°C, 5% CO₂ incubator for 4-5 hours, serum-free medium (100 µL/well) was added to the control group, and serum-free medium containing polypeptide or derivative thereof (N1-N54) was added to the upper chamber of the treatment group (100 µL/well), the final concentration of the peptide or derivative thereof in the upper chamber was 10 µM. The cells were incubated continuously for 24 hours, and then fixed with methanol and stained with crystal violet. Images were taken under a microscope and the number of migrated cells was counted with ImageJ.

### Example 4 Efficacy test of inhibiting tumor cell invasion-Transwell cell invasion assay

The Transwell chamber system was used in the transwell cell invasion experiment. The chamber has an upper layer and a lower layer, which are separated by an artificial semipermeable membrane in the middle. The upper surface of the semipermeable membrane is coated with a layer of artificial matrigel to simulate extracellular matrix. Cells that have invaded through the matrigel and semi-permeable membrane will attach to the lower surface of the semi-permeable membrane. The invasion ability is evaluated by counting the number of cells on the lower surface of the semi-permeable membrane. The number of cells that have invaded is negatively correlated with the inhibitory effect of the drug on the invasion ability of the tumor cells. Transwell invasion assay was performed as follows: tumor cells were cultured *in vitro,* cells in logarithmic growth phase were collected, and cell suspension was prepared with fresh serum-free medium at a density of 2-3×10⁵ cells/mL. Cells were inoculated, 100 µL/well cell suspension (serum-free medium) in the upper chamber and 600 µL/well complete culture medium in the lower chamber. After incubated in 37°C, 5% CO₂ incubator for 4-5 hours, serum-free medium (100 µL/well) was added to the control group, and serum-free medium containing polypeptide or derivative thereof (N1-N54) was added to the upper chamber of the treatment group (100 µL/well), the final concentration of the peptide or derivative thereof in the upper chamber was 10 µM. The cells were incubated continuously for 24 hours, and then fixed with methanol and stained with crystal violet. Images were taken under a microscope and the number of invaded cells was counted with ImageJ.

### Example 5 Evaluation of efficacy in animal tumor model

Tumor cells: A549-luc cells are obtained from Shanghai Meixuan Biotechnology Co., Ltd., and other tumor cells are obtained from the Cell Bank of the Chinese Academy of Sciences and the American Type Culture Collection (ATCC).

Experimental animals: 4-6 weeks old female immunodeficiency mice (BALB/c-nu/nu, Beijing Vital River Laboratory Animal Technology Co., Ltd.).

### Animal tumor model:

Subcutaneous xenograft animal model. The number of cells inoculated subcutaneously in mice was about 2×10⁶/200 µL. When the volume of the subcutaneous tumor was greater than 100 mm³, the mice were randomly divided into groups and subjected to treatment. When the mass volume of the tumors in model control group reached 2000 mm³ or the animal dies, the treatment ended. The animals were dissected, the tumors were taken out, and the weight and volume of the tumors were measured. During the period of the entire experiment, the mice were observed and the body weight was measured every other day.

Metastatic tumor model. The number of tumor cells inoculated into the tail vein was about 2×10⁶/100 µL, and the mice were divided into groups and subjected to treatment one hour after inoculation. The duration of the experiment was 28 days, and the *in vivo* fluorescence imaging of mice lung was performed after the treatment. The mice were observed and the fluorescence intensity of the mouse lungs is measured every other week.

Route of administration: intratumoral injection or intravenous injection, and the dosage and frequency of administration were determined according to the design of the specific experiments.

Experimental grouping: model control group, polypeptide or derivative thereof treatment group (N1-N54).

Number of experimental animals: 5 experimental animals in each treatment group.

### Example 6 Inhibition on the migration ability of human non-small cell lung cancer cells (A549) (scratch assay)

This example was a scratch assay to evaluate the inhibitory effect of the polypeptide or derivative thereof (N1-N54) on the migration ability of human non-small cell lung cancer (A549). The experimental method was as described in Example 2. Figure 1 shows the microscope images of cell migration. Table 1 shows the statistical results of the migration rate (mean ± SD, *P).* The results show that compared to the control group, the migration rate of A549 cells decreased significantly to different degrees after being treated with polypeptide or derivative thereof (N1-N54) for 48 hours, demonstrating that the polypeptide and derivatives thereof (N1-N54) inhibited the migration ability of A549 cells.

**Table 1 Statistical results of cell migration rate**

| **Group** | Migration rate (%) (mean ± SD, *P*) | **Group** | Migration rate (%) (mean ± SD, *P)* | **Group** | Migration rate (%) (mean ± SD, *P)* |
|---|---|---|---|---|---|
| Control group | 75.31±8.19 | | | | |
| N1 | 18.26±8.77, ^{∗∗} | N19 | 53.74±5.46,^{∗} | N37 | 49.45±10.09,^{∗} |
| N2 | 47.79±3.12, ^{∗∗} | N20 | 55.77±5.06,^{∗} | N38 | 46.92±4.49,^{∗∗} |
| N3 | 51.86±2.15,^{∗∗} | N21 | 31.06±4.94,^{∗∗} | N39 | 56.39±6.30,^{∗} |
| N4 | 51.53±3.72,^{∗} | N22 | 33.59±5.62,^{∗∗} | N40 | 20.15±8.91,^{∗∗} |
| N5 | 46.66±5.92,^{∗∗} | N23 | 26.03±4.21,^{∗∗∗} | N41 | 56.03±3.75,^{∗} |
| N6 | 40.67±3.66,^{∗∗} | N24 | 31.05±3.60,^{∗∗} | N42 | 60.74±3.40,^{∗} |
| N7 | 48.64±3.10,^{∗∗} | N25 | 34.66±7.73,^{∗∗} | N43 | 57.74±5.39,^{∗} |
| N8 | 32.62±5.83,^{∗∗} | N26 | 25.04±6.01,^{∗∗} | N44 | 53.59±5.62,^{∗} |
| N9 | 56.92±4.49,^{∗∗} | N27 | 37.20±6.23,^{∗∗} | N45 | 56.03±4.21,^{∗} |
| N10 | 26.86±8.78,^{∗∗} | N28 | 17.20±6.23,^{∗∗∗} | N46 | 58.05±3.60,^{∗} |
| N11 | 30.79±6.74,^{∗∗} | N29 | 20.55±3.73,^{∗∗∗} | N47 | 54.66±4.73,^{∗} |
| N12 | 20.05±8.79,^{∗∗} | N30 | 24.57±3.19,^{∗∗∗} | N48 | 22.04±3.01,^{∗∗∗} |
| N13 | 19.37±9.40,^{∗∗} | N31 | 24.44±8.15,^{∗∗} | N49 | 27.20±6.23,^{∗∗} |
| N14 | 15.27±8.75,^{∗∗∗} | N32 | 28.06±8.55,^{∗∗} | N50 | 57.74±4.39,^{∗} |
| N15 | 14.98±9.09,^{∗∗} | N33 | 33.22±4.51,^{∗∗} | N51 | 53.59±5.62,^{∗} |
| N16 | 30.57±5.41,^{∗∗} | N34 | 30.51±8.12,^{∗∗} | N52 | 56.03±4.21,^{∗} |
| N17 | 18.31±9.26,^{∗∗} | N35 | 32.27±9.53,^{∗∗} | N53 | 58.05±3.60,^{∗} |
| N18 | 55.71±4.05,^{∗} | N36 | 55.97±3.87,^{∗∗} | N54 | 54.66±4.73,^{∗} |
| Note: ^{∗}, *P*<0.05; ^{∗∗}, *P*<0.01;^{∗∗∗}, *P*<0.001 | | | | | |

### Example 7 Inhibition on the migration ability of human lung cancer cells (95D) (scratch assay)

This example was a scratch assay to evaluate the inhibitory effect of the polypeptide or derivative thereof (N1-N54) on the migration ability of human lung cancer cells (95D). The experimental method was as described in Example 2. Figure 2 shows the microscope images of cell migration. Table 2 shows the statistical results of the migration rate (mean ± SD, *P).* The results show that compared to the control group, the migration rate of 95D cells decreased significantly to different degrees after being treated with 10 µM polypeptide or derivative thereof for 48 hours, demonstrating that the polypeptide and derivatives thereof (N1-N54) inhibited the migration ability of 95D cells.

**Table 2 Statistical results of cell migration rate**

| **Group** | Migration rate (%) (mean ± SD, *P*) | **Group** | Migration rate (%) (mean ± SD, *P*) | **Group** | Migration rate (%) (mean ± SD, *P)* |
|---|---|---|---|---|---|
| Control group | 55.31±3.19 | | | | |
| N1 | 27.31±4.35,^{∗∗} | N19 | 42.04±5.90,^{∗} | N37 | 27.76±6.51,^{∗∗} |
| N2 | 36.44±4.16,^{∗∗} | N20 | 43.15±5.46,^{∗} | N38 | 37.60±3.67^{∗∗}, |
| N3 | 30.19±5.91,^{∗∗} | N21 | 40.02±4.20,^{∗∗} | N39 | 43.19±4.82,^{∗} |
| N4 | 31.65±3.82,^{∗∗} | N22 | 38.35±5.04,^{∗∗} | N40 | 36.23±5.05,^{∗∗} |
| N5 | 28.27±5.00,^{∗∗} | N23 | 30.38±4.68,^{∗∗} | N41 | 32.62±3.79,^{∗∗} |
| N6 | 39.20±4.24,^{∗∗} | N24 | 35.78±4.79,^{∗∗} | N42 | 35.42±4.97,^{∗∗} |
| N7 | 35.72±3.68,^{∗∗} | N25 | 40.70±3.25,^{∗∗} | N43 | 30.38±4.71,^{∗∗} |
| N8 | 35.13±4.45,^{∗∗} | N26 | 37.20±5.94,^{∗∗} | N44 | 37.20±6.81,^{∗} |
| N9 | 32.27±4.69,^{∗∗} | N27 | 37.20±6.23,^{∗} | N45 | 33.76±5.38,^{∗∗} |
| N10 | 34.35±5.74,^{∗∗} | N28 | 28.08±3.38,^{∗∗∗} | N46 | 35.06±5.72,^{∗∗} |
| N11 | 28.28±3.68,^{∗∗∗} | N29 | 30.30±4.81,^{∗∗} | N47 | 23.86±3.61,^{∗∗∗} |
| N12 | 28.72±2.96,^{∗∗} | N30 | 33.91±3.36,^{∗∗} | N48 | 25.07±6.31,^{∗∗} |
| N13 | 21.21±4.41,^{∗∗∗} | N31 | 25.70±3.17,^{∗∗} | N49 | 29.37±3.62,^{∗∗} |
| N14 | 32.55±5.71,^{∗∗} | N32 | 29.47±3.42,^{∗∗} | N50 | 31.05±4.32,^{∗∗} |
| N15 | 24.09±6.40,^{∗∗} | N33 | 30.73±5.87,^{∗∗} | N51 | 30.04±5.08,^{∗∗} |
| N16 | 17.00±8.00,^{∗∗} | N34 | 13.53±4.35,^{∗∗∗} | N52 | 27.19±5.67,^{∗∗} |
| N17 | 18.31±8.26,^{∗∗} | N35 | 22.47±5.12,^{∗∗∗} | N53 | 21.05±6.60,^{∗∗} |
| N18 | 25.71±4.05,^{∗∗∗} | N36 | 29.70±7.24,^{∗∗} | N54 | 24.66±6.13,^{∗∗} |
| Note: ^{∗}, *P*<0.05; ^{∗∗}, *P*<0.01; ^{∗∗∗}, *P*<0.001 | | | | | |

### Example 8 Inhibition on the migration ability of human lung squamous cell carcinoma cells (NCI-H226) (scratch assay)

This example was a scratch assay to evaluate the inhibitory effect of the polypeptide or derivative thereof (N1-N54) on the migration ability of human lung squamous carcinoma cells (NCI-H226). The experimental method was as described in Example 2. Figure 3 shows the microscope images of cell migration. Table 3 shows the statistical results of the migration rate (mean ± SD, *P).* The results show that compared to the control group, the migration rate of NCI-H226 cells decreased significantly to different degrees after being treated with 10 µM polypeptide or derivative thereof for 48 hours, demonstrating that the polypeptide and derivatives thereof (N1-N54) inhibited the migration ability of NCI-H226 cells.

**Table 3 Statistical results of cell migration rate**

| **Group** | Migration rate (%) (mean ± SD, *P)* | **Group** | Migration rate (%) (mean ± SD, *P)* | **Group** | Migration rate (%) (mean ± SD, *P)* |
|---|---|---|---|---|---|
| Control group | 65.31±3.19 | | | | |
| N1 | 12.68±9.07,^{∗∗∗} | N19 | 16.80±8.02,^{∗∗∗} | N37 | 11.33±9.49,^{∗∗∗} |
| N2 | 12.42±8.44,^{∗∗∗} | N20 | 19.54±6.51,^{∗∗∗} | N38 | 47.66±6.39,^{∗} |
| N3 | 16.38±6.85,^{∗∗∗} | N21 | 13.58±6.67,^{∗∗∗} | N39 | 21.82±5.54,^{∗∗∗} |
| N4 | 10.00±10.27,^{∗∗} | N22 | 18.69±5.25,^{∗∗∗} | N40 | 17.00±6.49,^{∗∗∗} |
| N5 | 10.65±9.94,^{∗∗∗} | N23 | 13.24±8.19,^{∗∗∗} | N41 | 15.30±8.69,^{∗∗∗} |
| N6 | 19.72±8.97,^{∗∗} | N24 | 15.74±8.70,^{∗∗∗} | N42 | 17.46±6.33,^{∗∗∗} |
| N7 | 18.83±4.53,^{∗∗∗} | N25 | 15.03±8.16,^{∗∗∗} | N43 | 16.38±7.71,^{∗∗∗} |
| N8 | 20.29±4.29,^{∗∗∗} | N26 | 18.41±7.90,^{∗∗∗} | N44 | 14.20±7.81,^{∗∗∗} |
| N9 | 35.47±5.20,^{∗∗} | N27 | 31.19±5.96,^{∗∗∗} | N45 | 23.76±5.38,^{∗∗∗} |
| N10 | 17.75±5.87,^{∗∗∗} | N28 | 18.08±7.38,^{∗∗∗} | N46 | 15.06±8.72,^{∗∗∗} |
| N11 | 21.77±5.03,^{∗∗∗} | N29 | 40.30±4.81,^{∗∗} | N47 | 53.86±3.61,^{∗} |
| N12 | 13.18±6.31,^{∗∗∗} | N30 | 13.91±7.36,^{∗∗∗} | N48 | 25.07±4.31,^{∗∗∗} |
| N13 | 11.29±8.97,^{∗∗∗} | N31 | 15.70±8.17,^{∗∗∗} | N49 | 38.90±5.49,^{∗∗} |
| N14 | 19.97±2.94,^{∗∗∗} | N32 | 19.47±6.42,^{∗∗∗} | N50 | 33.20±3.59,^{∗∗} |
| N15 | 35.05±3.95,^{∗∗} | N33 | 40.73±4.87,^{∗∗} | N51 | 33.16±4.43,^{∗∗} |
| N16 | 21.10±8.01,^{∗∗∗} | N34 | 36.53±4.35,^{∗∗} | N52 | 27.19±4.67,^{∗∗∗} |
| N17 | 43.38±4.83,^{∗∗} | N35 | 42.47±5.12,^{∗∗} | N53 | 41.05±4.60,^{∗∗} |
| N18 | 48.17±3.85,^{∗∗} | N36 | 55.70±3.24,^{∗} | N54 | 24.66±5.13,^{∗∗∗} |
| Note: ^{∗}, *P*<0.05; ^{∗∗}, *P*<0.01; ^{∗∗∗}, *P*<0.001 | | | | | |

### Example 9 Efficacy test on animal model of lung cancer subcutaneous xenograft (intratumoral injection)

The method used for the drug efficacy test in the animal model of the subcutaneous xenograft derived from lung cancer cells was as described in Example 5. The tumor cells were human lung cancer cells (A549), and the route of administration was intratumoral injection. There were two groups: the model control group and the polypeptide or its derivative treatment group (N1-N12). The drug dosage of the treatment group was 5 mg/kg, once every other day and the treatment time was 10 days. Table 4 shows the statistical results of the weight and volume of the tumors (mean ± SD, *P).* The results show that compared to the model control group, the weight and volume of the tumors of the N1-N12 treatment groups were decreased significantly to different degrees, demonstrating that the polypeptide and derivatives thereof (N1-N12) of the present invention effectively inhibited the development of tumors derived from human lung cancer cells.

**Table 4 Weight and volume of the tumors after treatment**

| Group | Weight of tumor (g) (mean ± SD) | Volume of tumor 3 (mm) (mean ± SD) | Group | Weight of tumor (g) (mean ± SD) | Volume of tumor 3 (mm) (mean ± SD) |
|---|---|---|---|---|---|
| Control group | 1.45±0.15 | 1269.32±115.39 | | | |
| N1 | 0.87±0.08 | 851.64±67.38 | N7 | 0.81±0.16 | 741.86±147.66 |
| N2 | 0.86±0.13 | 809.89±129.93 | N8 | 0.68±0.11 | 783.99±69.19 |
| N3 | 0.79±0.18 | 712.59±200.32 | N9 | 0.72±0.09 | 781.14±123.66 |
| N4 | 0.63±0.12 | 740.79±140.87 | N10 | 0.75±0.07 | 705.83±182.18 |
| N5 | 0.69±0.14 | 738.11±167.03 | N11 | 0.73±0.08 | 756.57±204.03 |
| N6 | 0.79±0.18 | 769.35±155.34 | N12 | 0.73±0.09 | 726.15±144.96 |

### Example 10 Efficacy test on animal model of lung cancer subcutaneous xenograft (intratumor injection)

The procedure of the efficacy test in this example, route and dosage of administration were the same as in Example 9. The peptides used in the experiment were N13-N32. The treatment time was 20 days. Table 5 shows the statistical results (mean ± SD, *P)* of the weight and volume of the tumors. The results show that compared to the model control group, the weight and volume of the tumors in N13-N32 treatment groups were decreased significantly to different degrees, demonstrating that the polypeptide and derivatives thereof (N13-N32) of the present invention effectively inhibited the development of tumors derived from human lung cancer cells.

**Table 5 Weight and volume of the tumors after treatment**

| Group | Weight of tumor (g) (mean ± SD) | Volume of tumor 3 (mm) (mean ± SD) | Group | Weight of tumor (g) (mean ± SD) | Volume of tumor 3 (mm) (mean ± SD) |
|---|---|---|---|---|---|
| Control group | 1.92±0.13 | 1700.16±305.72 | | | |
| N13 | 1.09±0.09 | 986.7±261.02 | N23 | 0.83±0.29 | 838.73±218 |
| N14 | 1.09±0.29 | 913.68±219.62 | N24 | 0.83±0.29 | 908.37±256.24 |
| N15 | 0.63±0.13 | 793.32±173.3 | N25 | 0.71±0.18 | 958.87±351.9 |
| N16 | 0.83±0.27 | 897.27±176.16 | N26 | 0.8±0.22 | 1053.55±175.83 |
| N17 | 0.76±0.26 | 893.18±234.75 | N27 | 0.95±0.22 | 1059.51±161.22 |
| N18 | 0.75±0.26 | 969.37±243.04 | N28 | 0.81±0.24 | 1055.02+222.45 |
| N19 | 0.81±0.29 | 963.18±185.31 | N29 | 0.82±0.22 | 949±227.4 |
| N20 | 0.89±0.22 | 1037.56±256.6 | N30 | 0.9±0.22 | 913.9±204.07 |
| N21 | 0.9±0.28 | 967.35±247.89 | N31 | 0.71±0.16 | 900.88±255.61 |
| N22 | 0.83±0.33 | 835.73±184.67 | N32 | 0.88±0.25 | 984.03±184.86 |

### Example 11 Efficacy test on animal model of lung cancer subcutaneous xenograft (intravenous injection)

The method used in the efficacy test on the lung cancer subcutaneous xenograft animal model was as described in Example 5. The lung cancer cells were the human lung cancer cells (A549), and the route of administration was intravenous injection. This example involved a total of 3 experiments to evaluate N1-N15, N16-N30, and N31-N54 respectively. The experimental procedure, route of administration, dosage and frequency of administration, and detection method were all consistent in the three experiments. The experimental groups were model control group and polypeptide or derivative thereof treatment groups (N1-N15, N16-N30, or N31-N54). The drug dosage of the N1-N54 treatment groups was 60 mg/kg, and the treatment time was 20 days. The experimental results were shown in Figure 4 and Table 6. Figure 4 shows the representative images of tumors in each group after the treatment. Table 6 shows the statistical results of the weight and volume of tumors (mean ± SD, *P*). The results showed that compared to the model control group, the weight and volume of the tumors of the N1-N54 treatment group were decreased significantly to different degrees, demonstrating that the polypeptide and derivatives thereof (N1-N54) of the present invention effectively inhibited the development of tumors derived from human lung cancer cells.

**Table 6 Weight and volume of the tumors after treatment**

| Group | Weight of tumor (g) (mean ± SD) | Volume of tumor 3 (mm) (mean ± SD) | Group | Weight of tumor (g) (mean ± SD) | Volume of tumor 3 (mm) (mean ± SD) |
|---|---|---|---|---|---|
| Control group | 2.53±0.17 | 3373.26±581.44 | | | |
| N1 | 1.01±0.2 | 1340.25±319.59 | N28 | 0.91±0.21 | 1286.52±344.21 |
| N2 | 0.6±0.28 | 1297.28±359.77 | N29 | 1±0.17 | 1365.48±435.32 |
| N3 | 0.56±0.16 | 1511.56±433.12 | N30 | 1.01±0.34 | 1187.13±246.31 |
| N4 | 0.56±0.19 | 1515.07±507.99 | N31 | 0.62±0.2 | 1439.74±546.27 |
| N5 | 0.71±0.12 | 1353.09±422.65 | N32 | 0.88±0.38 | 1630.6±424.84 |
| N6 | 0.88±0.19 | 1361.03±248.72 | N33 | 0.57±0.24 | 1699.23±709.14 |
| N7 | 0.81±0.15 | 1189.39±200.83 | N34 | 1.06±0.15 | 1472.21±914.38 |
| N8 | 1.02±0.21 | 1359.06±275.17 | N35 | 0.75±0.24 | 1097.54±216.63 |
| N9 | 0.82±0.29 | 1241.9±388.92 | N36 | 0.66±0.25 | 1004.87±240.09 |
| N10 | 0.86±0.17 | 1211.62±670.09 | N37 | 0.89±0.39 | 915.97±199.07 |
| N11 | 1.3±0.23 | 1419.28±615.42 | N38 | 0.71±0.27 | 927.9±205.31 |
| N12 | 0.85±0.19 | 1275.34±221.5 | N39 | 0.98±0.3 | 918.84±288.77 |
| N13 | 0.8±0.17 | 1320.14±147.22 | N40 | 0.98±0.36 | 997.42±267.79 |
| N14 | 0.7±0.26 | 1431.61±215.71 | N41 | 0.84±0.34 | 1075.33±350.78 |
| N15 | 0.95±0.18 | 1307.48±416.26 | N42 | 0.83±0.31 | 1149.73±346.36 |
| N16 | 1.05±0.26 | 1442.4±293.63 | N43 | 0.85±0.14 | 1218.7±190.78 |
| N17 | 0.9±0.16 | 1301.58±329.64 | N44 | 1.02±0.41 | 1220.92±126.33 |
| N18 | 1.3±0.24 | 1161.51±297.07 | N45 | 0.97±0.28 | 1204.87±212.94 |
| N19 | 0.81±0.19 | 1235.44±432.58 | N46 | 0.81±0.2 | 1750.75±493.84 |
| N20 | 0.56±0.18 | 1072.16±390.44 | N47 | 0.75±0.39 | 1748.82±508.08 |
| N21 | 0.71±0.22 | 1065.78±432.05 | N48 | 0.71±0.37 | 1558.12±436.42 |
| N22 | 0.89±0.19 | 1288.08±577.81 | N49 | 0.73±0.26 | 1513.42±287.49 |
| N23 | 0.65±0.17 | 993.11±298.65 | N50 | 0.85±0.27 | 1463.11±472.43 |
| N24 | 0.81±0.25 | 1099.3±512.53 | N51 | 0.75±0.37 | 1412.56±599.92 |
| N25 | 0.69±0.21 | 1169.92±572.47 | N52 | 0.7±0.2 | 1425.2±555.76 |
| N26 | 0.57±0.22 | 1163.91±457.09 | N53 | 0.75±0.27 | 1549.08±539.99 |
| N27 | 1.14±0.23 | 1402.37±405.66 | N54 | 0.89±0.37 | 1381.65±396.21 |

### Example 12 Efficacy test on animal model of lung metastatic cancer (intravenous injection)

The method used in the efficacy test on lung cancer metastasis animal model was as described in Example 5. The lung cancer cells were the human lung cancer cells A549-luc, and the route of administration was intravenous injection. The experimental groups were model control group and polypeptide or derivative thereof treatment groups (N1-N12). The drug dosage of the N1-N12 treatment group was 30 mg/kg, once every other day. Figure 5 shows the live fluorescence images of the lungs from each group before and after treatment. Compared to the model control group, the fluorescence intensity of the lungs from the N1-N12 treatment groups was reduced to different degrees, demonstrating that the polypeptide and derivatives thereof (N1-N12) of the present invention effectively inhibited the development of lung metastatic tumors.

### Example 13 Inhibition on the migration ability of human pancreatic cancer cells (PANC-1) (scratch assay)

This example was a scratch assay to evaluate the inhibitory effect of the polypeptide or derivative thereof (N1-N54) on the migration ability of human pancreatic cancer cells (PANC-1). The experimental method was as described in Example 2. Figure 6 shows the microscope images of cell migration. Table 7 shows the statistical results of the migration rate (mean ± SD, *P).* The results show that compared to the control group, the migration rate of PANC-1 decreases significantly to different degrees after being treated with polypeptide or derivative thereof for 48 hours, demonstrating that the polypeptide and derivatives thereof (N1-N54) inhibited the migration ability of PANC-1 cells.

**Table 7 Statistical results of cell migration rate**

| **Group** | Migration rate (%) (mean ± SD, *P)* | **Group** | Migration rate (%) (mean ± SD, *P)* | **Group** | Migration rate (%) (mean ± SD, *P)* |
|---|---|---|---|---|---|
| Control group | 66.65±3.37 | | | | |
| N1 | 18.89±6.68,^{∗∗∗} | N19 | 19.76±7.14,^{∗∗∗} | N37 | 18.23±6.77,^{∗∗∗} |
| N2 | 32.74±4.26,^{∗∗} | N20 | 41.56±6.68,^{∗} | N38 | 24.87±5.81,^{∗∗∗} |
| N3 | 29.67±5.64,^{∗∗∗} | N21 | 10.54±9.03,^{∗∗∗} | N39 | 16.29±8.00,^{∗∗∗} |
| N4 | 19.04±8.54,^{∗∗∗} | N22 | 11.44±8.52, ^{∗∗∗} | N40 | 13.24±8.56,^{∗∗∗} |
| N5 | 21.63±6.16,^{∗∗∗} | N23 | 14.16±8.36,^{∗∗∗} | N41 | 14.44±7.40,^{∗∗∗} |
| N6 | 36.66±5.57,^{∗∗} | N24 | 13.99±7.57,^{∗∗∗} | N42 | 24.26±7.91,^{∗∗} |
| N7 | 37.07±7.83,^{∗∗} | N25 | 25.36±7.32,^{∗∗∗} | N43 | 18.87±6.90,^{∗∗∗} |
| N8 | 35.11±5.96,^{∗∗∗} | N26 | 26.65±6.37,^{∗∗∗} | N44 | 16.45±7.77,^{∗∗∗} |
| N9 | 29.12±5.84,^{∗∗∗} | N27 | 27.19±7.04,^{∗∗∗} | N45 | 45.16±5.74,^{∗∗} |
| N10 | 32.80±5.89,^{∗∗} | N28 | 32.25±5.40,^{∗∗} | N46 | 26.80±6.50,^{∗∗∗} |
| N11 | 28.37±7.57,^{∗∗} | N29 | 40.62±4.97,^{∗} | N47 | 36.05±5.62,^{∗∗} |
| N12 | 23.26±6.90,^{∗∗} | N30 | 16.60±5.85,^{∗∗∗} | N48 | 20.60±4.71,^{∗∗∗} |
| N13 | 18.28±8.38,^{∗∗∗} | N31 | 13.64±7.59,^{∗∗∗} | N49 | 17.71±7.65,^{∗∗∗} |
| N14 | 26.63±6.67,^{∗∗∗} | N32 | 15.49±8.35,^{∗∗∗} | N50 | 14.92±8.62,^{∗∗∗} |
| N15 | 14.85±5.08,^{∗∗∗} | N33 | 39.27±3.14,^{∗} | N51 | 20.85±8.10,^{∗∗∗} |
| N16 | 19.15±4.97,^{∗∗∗} | N34 | 28.58+3.87,^{∗∗∗} | N52 | 23.11±4.27,^{∗∗∗} |
| N17 | 15.47±5.29^{∗∗∗} | N35 | 36.83±5.92,^{∗∗} | N53 | 15.85±7.32,^{∗∗∗} |
| N18 | 13.26±7.39,^{∗∗∗} | N36 | 29.81±5.39,^{∗∗∗∗∗} | N54 | 16.62±5.73,^{∗∗∗} |
| Note: ^{∗}, *P<*0.05;^{∗∗} *P*<0.01; ^{∗∗∗}, *P*<0.001 | | | | | |

### Example 14 Inhibition on the migration ability of human pancreatic cancer cells (PANC-1) (transwell migration assay)

This example was a transwell cell migration experiment to evaluate the inhibitory effect of the polypeptide or derivative thereof (N1-N54) on the migration ability of human pancreatic cancer cells (PANC-1). The experimental method was as described in Example 3. Figure 7 shows the microscope images of the cells that migrated to the lower surface of the semipermeable membrane. Table 8 shows the statistical results of the number of cells that migrated to the lower surface of the semipermeable membrane (mean ± SD, *P).* The results show that compared to the control group, the number of cells migrated to the lower surface of the semipermeable membrane in the N1-N54 treatment groups is significantly less, demonstrating that the polypeptide and derivatives thereof (N1-N54) of the present invention inhibited the migration ability of human pancreatic cancer cells.

**Table 8 Statistical results of the number of migrated cells**

| Group | Number of cells (mean ± SD, *P* ) | Group | Number of celsl (mean ± SD, *P* ) | Group | Number of cells (mean ± SD, *P* ) |
|---|---|---|---|---|---|
| Control group | 360±60 | | | | |
| N1 | 245±27,^{∗} | N19 | 238±27,^{∗} | N37 | 113±11,^{∗∗∗} |
| N2 | 221±35,^{∗∗} | N20 | 244±23,^{∗} | N38 | 116±27,^{∗∗∗} |
| N3 | 224±28,^{∗∗} | N21 | 216±12,^{∗∗∗} | N39 | 262±13,^{∗} |
| N4 | 268±20,^{∗} | N22 | 200±18,^{∗∗∗} | N40 | |
| N5 | 234±27,^{∗} | N23 | 200±14,^{∗∗∗} | N41 | 248±22,^{∗} |
| N6 | 144±12,^{∗∗∗} | N24 | 239±24,^{∗} | N42 | 236±14,^{∗∗} |
| N7 | 132±15,^{∗∗∗} | N25 | 217±31,^{∗∗} | N43 | 157±15,^{∗∗∗} |
| N8 | 133±20,^{∗∗∗} | N26 | 119±17,^{∗∗∗} | N44 | 233±28,^{∗} |
| N9 | 148±16,^{∗∗∗∗} | N27 | 143±25,^{∗∗∗} | N45 | 135±10,^{∗∗∗} |
| N10 | 254±20,^{∗} | N28 | 148±21,^{∗∗∗} | N46 | 145±11,^{∗∗∗} |
| N11 | 239±26,^{∗} | N29 | 178±31,^{∗∗∗} | N47 | 135±10,^{∗∗∗} |
| N12 | 246±14,^{∗} | N30 | 243±33,^{∗} | N48 | 152±18,^{∗∗∗} |
| N13 | 255±24,^{∗} | N31 | 217±40,^{∗∗} | N49 | 137±29,^{∗∗∗} |
| N14 | 263±16,^{∗} | N32 | 219±13,^{∗∗∗} | N50 | 230±11,^{∗∗} |
| N15 | 220±18,^{∗∗} | N33 | 178±14,^{∗∗∗} | N51 | 214±17,^{∗∗∗} |
| N16 | 230±28,^{∗} | N34 | 248±19,^{∗} | N52 | 153±16,^{∗∗∗} |
| N17 | 244±14,^{∗} | N35 | 151±10,^{∗∗∗} | N53 | 131±8,^{∗∗∗} |
| N18 | 232±24,^{∗} | N36 | 124±13,^{∗∗∗} | N54 | 128±19,^{∗∗∗} |
| Note: ^{∗}, *P*<*0.05;* ^{∗∗}, *P*<0.01; ^{∗∗∗}, *P*<0.001 | | | | | |

### Example 15 Inhibition on the invasion ability of human pancreatic cancer cells (PANC-1) (transwell invasion assay)

This example was the transwell cell invasion experiment to evaluate the inhibitory effect of the polypeptide or derivative thereof (N1-N54) on the invasion ability of human pancreatic cancer cells (PANC-1). The experimental method was as described in Example 4. Figure 8 shows the microscope images of the cells that migrated to the lower surface of the semipermeable membrane. Table 9 shows the statistical results of the number of cells that migrated to the lower surface of the semipermeable membrane (mean ± SD, *P).* The results show that compared to the control group, the number of cells migrated to the lower surface of the semipermeable membrane in the N1-N54 treatment groups decreases significantly, demonstrating that the polypeptide and derivatives thereof (N1-N54) of the present invention inhibited the invasion ability of human pancreatic cancer cells.

**Table 9 Statistical results of the number of invaded cells**

| Group | Number of cells (mean ± SD, *P*) | Group | Number of cells (mean ± SD, *P)* | Group | Number of cells (mean ± SD, *P)* |
|---|---|---|---|---|---|
| Control group | 727±68 | | | | |
| N1 | 83±26,^{∗∗∗∗} | N19 | 213±20,^{∗∗∗∗} | N37 | 359±34^{∗∗} |
| N2 | 93±19,^{∗∗∗∗} | N20 | 273±12,^{∗∗∗∗} | N38 | 262±21,^{∗∗∗∗} |
| N3 | 111±10^{∗∗∗∗} | N21 | 269±28,^{∗∗∗∗} | N39 | 278±16,^{∗∗∗∗} |
| N4 | 37±4,^{∗∗∗∗} | N22 | 220±65,^{∗∗∗∗} | N40 | 273±21,^{∗∗∗∗} |
| N5 | 109±7,^{∗∗∗∗} | N23 | 196±26,^{∗∗∗∗} | N41 | 288±38,^{∗∗∗∗} |
| N6 | 110±5, ^{∗∗∗∗} | N24 | 226±18,^{∗∗∗∗} | N42 | 399±21,^{∗∗∗} |
| N7 | 70±17,^{∗∗∗∗} | N25 | 247±17,^{∗∗∗∗} | N43 | 251±10,^{∗∗∗∗} |
| N8 | 65±16,^{∗∗∗∗} | N26 | 231±18,^{∗∗∗∗} | N44 | 349±30,^{∗∗∗} |
| N9 | 93±6,^{∗∗∗∗} | N27 | 242±35,^{∗∗∗∗} | N45 | 358±23,^{∗∗∗} |
| N10 | 159±12,^{∗∗∗∗} | N28 | 247±15,^{∗∗∗∗} | N46 | 125±17,^{∗∗∗∗} |
| N11 | 66±14,^{∗∗∗∗} | N29 | 213±17,^{∗∗∗∗} | N47 | 133±29,^{∗∗∗∗} |
| N12 | 180±17,^{∗∗∗∗} | N30 | 237±15,^{∗∗∗∗} | N48 | 149±12,^{∗∗∗∗} |
| N13 | 161±19,^{∗∗∗∗} | N31 | 365±57,^{∗∗∗} | N49 | 141±7,^{∗∗∗∗} |
| N14 | 157±37,^{∗∗∗∗} | N32 | 251±10,^{∗∗∗∗} | N50 | 310±30,^{∗∗∗} |
| N15 | 104±9,^{∗∗∗∗} | N33 | 345±27,^{∗∗∗} | N51 | 282±28,^{∗∗∗∗} |
| N16 | 128±25,^{∗∗∗∗} | N34 | 346±30,^{∗∗∗} | N52 | 237±27,^{∗∗∗∗} |
| N17 | 95±6,^{∗∗∗∗} | N35 | 358±23,^{∗∗∗} | N53 | 228±25,^{∗∗∗∗} |
| N18 | 118±14,^{∗∗∗∗} | N36 | 384±22,^{∗∗∗} | N54 | 231±26,^{∗∗∗∗} |
| Note: ^{∗∗∗}, *P*<0.001; ^{∗∗∗∗}, *P*<0.0001 | | | | | |

### Example 16 Efficacy test on an animal model of pancreatic cancer subcutaneous xenograft (intratumoral injection)

The method used in the efficacy test on the pancreatic cancer subcutaneous xenograft animal model was as described in Example 5. The pancreatic cancer cells were human pancreatic cancer cells (PANC-1), and the route of administration was intratumoral injection. The experimental groups were model control group and polypeptide or derivative thereof treatment groups (N1-N12). The drug dosage of the N1-N12 treatment group was 5mg/kg, once every other day, and the treatment time was 25 days. The experimental results were shown in Figure 9 and Table 10. Figure 9 shows the images of tumors in each group after treatment. Table 10 shows the statistical results of the weight and volume of the tumors. The results show that compared to the model control group, the weight and volume of the tumors in the N1-N12 treatment groups were both less, demonstrating that the polypeptide and derivatives thereof (N1-N12) of the present invention effectively inhibited the development of tumors derived from human pancreatic cancer cells.

**Table 10 Weight and volume of the tumors after treatment**

| Group | Weight of tumor (g) (mean ± SD) | Volume of tumor 3 (mm) (mean ± SD) | Group | Weight of tumor (g) (mean ± SD) | Volume of tumor 3 (mm) (mean ± SD) |
|---|---|---|---|---|---|
| Control group | 1.2±0.11 | 1431.72±174.33 | | | |
| N1 | 0.52±0.13 | 704.1±152.43 | N7 | 0.74±0.09 | 671.47±150.71 |
| N2 | 0.72±0.12 | 761.37±150.52 | N8 | 0.59±0.11 | 769.54±160.54 |
| N3 | 0.6±0.1 | 695.11±111.9 | N9 | 0.5±0.09 | 765.6±105.04 |
| N4 | 0.62±0.07 | 673.03±157.33 | N10 | 0.57±0.11 | 667.99±188.73 |
| N5 | 0.54±0.1 | 696.52±127.05 | N11 | 0.62±0.12 | 785.02±147.38 |
| N6 | 0.61±0.07 | 538.26±121.04 | N12 | 0.71±0.09 | 863.2±129.61 |

### Example 17 Inhibition on the migration ability of human liver adenocarcinoma cells (SK-HEP-1) (transwell migration assay)

This example was a transwell cell migration experiment to evaluate the inhibitory effect of the polypeptide or derivative thereof (N1-N54) on the migration ability of human liver adenocarcinoma cells (SK-HEP-1). The experimental method was as described in Example 3. Figure 10 shows the microscope images of the cells that migrated to the lower surface of the semipermeable membrane. Table 11 shows the statistical results of the number of cells that migrated to the lower surface of the semipermeable membrane (mean ± SD, *P).* The results show that compared to the control group, the number of cells migrated to the lower surface of the semipermeable membrane in the N1-N54 treatment groups decreases significantly, demonstrating that the polypeptide and derivatives thereof (N1-N54) of the present invention inhibited the migration ability of human liver cancer cells.

**Table 11 Statistical results of the number of migrated cells**

| Group | Number of cells (mean ± SD, *P*) | Group | Number of cells (mean ± SD, *P)* | Group | Number of cells (mean ± SD, *P)* |
|---|---|---|---|---|---|
| Control group 1 | 782±108 | Control group 2 | 768±86 | | |
| N1 | 87±10, ^{∗∗∗∗} | N19 | 82+6,^{∗∗∗∗} | N37 | 84±7 ,^{∗∗∗∗} |
| N2 | 104±10, ^{∗∗∗∗} | N20 | 299±35 ^{∗∗∗∗} | N38 | 380±32 ,^{∗∗∗} |
| N3 | 112±10,^{∗∗∗∗} | N21 | 34±7,^{∗∗∗∗} | N39 | 261±25,^{∗∗∗∗} |
| N4 | 357±21,^{∗∗∗} | N22 | 42±17,^{∗∗∗∗} | N40 | 290±33,^{∗∗∗∗} |
| N5 | 167±12 ,^{∗∗∗∗} | N23 | 276±24,^{∗∗∗∗} | N41 | 280±24, ^{∗∗∗∗} |
| N6 | 157±13 ,^{∗∗∗∗} | N24 | 265±24,^{∗∗∗∗} | N42 | 106±17,^{∗∗∗∗} |
| N7 | 127±16 ,^{∗∗∗∗} | N25 | 58±15 ,^{∗∗∗∗} | N43 | 157±29 ,^{∗∗∗∗} |
| N8 | 139±13,^{∗∗∗∗} | N26 | 68±19 ,^{∗∗∗∗} | N44 | 165±18,^{∗∗∗∗} |
| N9 | 174±15 ,^{∗∗∗∗} | N27 | 469±56 ,^{∗∗} | N45 | 162±15,^{∗∗∗∗} |
| N10 | 54±5,^{∗∗∗∗} | N28 | 51±25,^{∗∗∗∗} | N46 | 172±15 ,^{∗∗∗∗} |
| N11 | 80±6 ,^{∗∗∗∗} | N29 | 254±14 ,^{∗∗∗∗} | N47 | 179±17,^{∗∗∗∗} |
| N12 | 405±49 ,^{∗∗} | N30 | 156±22,^{∗∗∗∗} | N48 | 374±22,^{∗∗∗} |
| N13 | 25±12,^{∗∗∗∗} | N31 | 90±6 ,^{∗∗∗∗} | N49 | 137±11,^{∗∗∗∗} |
| N14 | 61±14 ,^{∗∗∗∗} | N32 | 201±13,^{∗∗∗∗} | N50 | 192±16 ,^{∗∗∗∗} |
| N15 | 131±10,^{∗∗∗∗} | N33 | 354±31,^{∗∗∗} | N51 | 306±46,^{∗∗∗} |
| N16 | 74±18,^{∗∗∗∗} | N34 | 260±32,^{∗∗∗∗} | N52 | 318±37,^{∗∗∗} |
| N17 | 50±5 ,^{∗∗∗∗} | N35 | 315±36 ,^{∗∗∗} | N53 | 265±32 ,^{∗∗∗∗} |
| N18 | 96±28 ,^{∗∗∗∗} | N36 | 169±22 ,^{∗∗∗∗} | N54 | 345±3 ,^{∗∗∗∗} |
| **Note:** ^{∗∗}, P<0.01; ^{∗∗∗}, *P*<0.001; ^{∗∗∗∗}, *P*<0.0001; N1-N23 vs control group 1; N24-N54 vs control group 2. | | | | | |

### Example 18 Inhibition on the invasion ability of human liver adenocarcinoma cells (SK-HEP-1) (transwell invasion assay)

This example was a transwell cell invasion experiment to evaluate the inhibitory effect of the polypeptide or derivative thereof (N1-N54) on the invasion ability of human liver adenocarcinoma cells (SK-HEP-1). The experimental method was as described in Example 4. Figure 11 shows the microscope images of the cells that migrated to the lower surface of the semipermeable membrane. Table 12 shows the statistical results of the number of cells that migrated to the lower surface of the semipermeable membrane (mean ± SD, *P).* The results show that compared to the control group, the number of cells migrated to the lower surface of the semipermeable membrane in the N1-N54 treatment groups decreases significantly, demonstrating that the polypeptide and derivatives thereof (N1-N54) of the present invention inhibited the invasion ability of human liver cancer cells.

**Table 12 Statistical results of the number of invaded cells**

| Group | Number of cells (mean ± SD, *P*) | Group | Number of cells (mean ± SD, *P*) | Group | Number of cells (mean ± SD, *P)* |
|---|---|---|---|---|---|
| Control group | 767±91 | | | | |
| N1 | 343±38,^{∗∗∗} | N19 | 348±40,^{∗∗∗} | N37 | 233±13,^{∗∗∗∗} |
| N2 | 432±47,^{∗∗} | N20 | 242±22,^{∗∗∗∗} | N38 | 249±42,^{∗∗∗∗} |
| N3 | 372±62,^{∗∗∗} | N21 | 356±60,^{∗∗∗} | N39 | 88±25,^{∗∗∗∗} |
| N4 | 411±75,^{∗∗} | N22 | 430±51,^{∗∗} | N40 | 123±26,^{∗∗∗∗} |
| N5 | 353±52,^{∗∗∗} | N23 | 456±45,^{∗∗} | N41 | 58±12,^{∗∗∗∗} |
| N6 | 145±29,^{∗∗∗∗} | N24 | 332±30,^{∗∗∗} | N42 | 149±29,^{∗∗∗∗} |
| N7 | 463±52,^{∗∗} | N25 | 411±30,^{∗∗∗} | N43 | 107±16,^{∗∗∗∗} |
| N8 | 277±38,^{∗∗∗∗} | N26 | 371±27,^{∗∗∗} | N44 | 30±5,^{∗∗∗∗} |
| N9 | 288±35,^{∗∗∗} | N27 | 369±52,^{∗∗∗} | N45 | 155±10,^{∗∗∗∗} |
| N10 | 460±43,^{∗∗} | N28 | 214±31,^{∗∗∗∗} | N46 | 102±23,^{∗∗∗∗} |
| N11 | 398±36,^{∗∗∗} | N29 | 322±56,^{∗∗∗} | N47 | 85±16,^{∗∗∗∗} |
| N12 | 308±42,^{∗∗∗} | N30 | 85±20,^{∗∗∗∗} | N48 | 109±10,^{∗∗∗∗} |
| N13 | 258±32,^{∗∗∗∗} | N31 | 269±17,^{∗∗∗∗} | N49 | 335±32,^{∗∗∗} |
| N14 | 329±54,^{∗∗∗} | N32 | 252±7,^{∗∗∗∗} | N50 | 243±46,^{∗∗∗∗} |
| N15 | 418±36,^{∗∗} | N33 | 197±32,^{∗∗∗∗} | N51 | 248±39,^{∗∗∗∗} |
| N16 | 398±43,^{∗∗∗} | N34 | 187±38,^{∗∗∗∗} | N52 | 132±29,^{∗∗∗∗} |
| N17 | 165±28,^{∗∗∗∗} | N35 | 338±43,^{∗∗∗} | N53 | 143±19,^{∗∗∗∗} |
| N18 | 262±21,^{∗∗∗∗∗} | N36 | 413±63,^{∗∗} | N54 | 151±28,^{∗∗∗∗} |
| **Note:** ^{∗∗}, *P*<0.01; ^{∗∗∗}, *P*<0.001; ^{∗∗∗∗}, *P*<0.0001 | | | | | |

### Example 19 Inhibition on fibronectin expression in human liver adenocarcinoma cells (SK-HEP-1)

SK-HEP-1 cells were cultured *in vitro,* cells in the logarithmic growth phase were collected, and the cells were seeded evenly in a 24-well plate at a density of 1-3×10⁵ cells/well. The cells were cultured in a cell incubator for 4-5h. PBS was added to the control group. PBS containing polypeptide or derivative thereof (N1-N12) was added to the treatment group. The final concentration of the polypeptide or derivative thereof in the well was 10 µM After incubating for 24 hours, the cells were collected and the total protein was extracted. ELISA was used to detect the content of fibronectin in total protein. The results were shown in Figure 12 and Table 13. Compared to the control group, the fibronectin level in the N1-N12 treatment groups decreased significantly, demonstrating that the polypeptide and derivatives thereof (N1-N12) of the present invention inhibited the expression of fibronectin in human liver cancer cells.

**Table 13**

| Group | Relative protein level (mean ± SD, P) | Group | Relative protein level (mean ± SD, P) | Group | Relative protein level (mean ± SD, *P)* |
|---|---|---|---|---|---|
| Control group | 1.62±0.09 | | | | |
| N1 | 1.3±0.02,^{∗∗} | N5 | 1.3±0.11,^{∗} | N9 | 1.33±0.09,^{∗} |
| N2 | 1.23±0.08,^{∗∗} | N6 | 1.33±0.04,^{∗∗} | N10 | 1.3±0.05,^{∗∗} |
| N3 | 1.26±0.09,^{∗∗} | N7 | 1.36±0.04,^{∗} | N11 | 1.39±0.01,^{∗} |
| N4 | 1.23±0.11,^{∗∗} | N8 | 1.33±0.11,^{∗} | N12 | 1.23±0.07,^{∗∗} |
| Note: ^{∗}, *P*<0.05; ^{∗∗}, *P*<0.01 | | | | | |

### Example 20 Inhibition on N-cadherin expression in human liver adenocarcinoma cells (SK-HEP-1)

The experimental method for testing the inhibition on the expression of N-cadherin in human liver adenocarcinoma cells (SK-HEP-1) by the polypeptide or derivative thereof (N1-N12) was as described in Example 19. The results are shown in Figure 13 and Table 14. Compared to the control group, N-cadherin level in the N1-N12 treatment groups decreased significantly, demonstrating that the polypeptide and derivatives thereof (N1-N12) of the present invention inhibited the expression of N-cadherin in human liver cancer cells.

**Table 14**

| Group | Relative protein level (mean ± SD, *P)* | Group | Relative protein level (mean ± SD, *P)* | Group | Relative protein level (mean ± SD, *P)* |
|---|---|---|---|---|---|
| Control group | 1.12±0.03 | | | | |
| N1 | 0.83±0.04,^{∗∗∗} | N5 | 0.83±0.04,^{∗∗∗} | N9 | 0.73±0.04,^{∗∗∗} |
| N2 | 0.93±0.04,^{∗∗} | N6 | 0.83±0.04,^{∗∗∗} | N10 | 0.69±0.03,^{∗∗∗∗} |
| N3 | 0.87±0.09,^{∗} | N7 | 0.73±0.06,^{∗∗∗} | N11 | 0.73±0.04,^{∗∗∗} |
| N4 | 0.93±0.04,^{∗∗} | N8 | 0.7±0.06,^{∗∗∗} | N12 | 0.58±0.03,^{∗∗∗} |
| Note: ^{∗}, P<0.05; ^{∗∗}, P<0.01; ^{∗∗∗}, P<0.001;^{∗∗∗∗}, P<0.0001 | | | | | |

### Example 21 Inhibition on vimentin expression in human liver adenocarcinoma cell (SK-HEP-1)

The experimental method for testing the inhibition on the expression of vimentin in human liver adenocarcinoma cells (SK-HEP-1) by the polypeptide or derivative thereof (N1-N12) was as described in Example 19. The results are shown in Figure 14 and Table 15. Compared to the control group, vimentin level in the N1-N12 treatment groups decreased significantly, demonstrating that the polypeptide and derivatives thereof (N1-N12) of the present invention inhibited the expression of vimentin in human liver cancer cells.

**Table 15**

| Group | Relative protein level (mean ± SD, *P*) | Group | Relative protein level (mean ± SD, *P*) | Group | Relative protein level (mean ± SD, *P)* |
|---|---|---|---|---|---|
| Control group | 1.2±0.01 | | | | |
| N1 | 0.97±0.03, ^{∗∗∗} | N5 | 0.87±0.03, ^{∗∗∗∗} | N9 | 1.03±0.05, ^{∗∗} |
| N2 | 0.91±0.03, ^{∗∗∗∗} | N6 | 0.81±0.03, ^{∗∗∗∗} | N10 | 0.88±0.06, ^{∗∗∗} |
| N3 | 0.96±0.05, ^{∗∗} | N7 | 1±0.03, ^{∗∗∗} | N11 | 0.94±0.05, ^{∗∗∗} |
| N4 | 0.95±0.03, ^{∗∗∗} | N8 | 0.81±0.03, ^{∗∗∗∗} | N12 | 0.71±0.03, ^{∗∗∗∗} |
| Note: ^{∗∗}, P<0.01; ^{∗∗∗}, P<0.001;^{∗∗∗∗}, P<0.0001 | | | | | |

### Example 22 Inhibition on the migration ability of human liver cancer cells (HepG2) (scratch assay)

This example was a scratch assay to evaluate the inhibitory effect of the polypeptide or derivative thereof (N1-N54) on the migration ability of human liver cancer cells (HepG2). The experimental method was as described in Example 2. Figure 15 shows the microscope images of cell migration. Table 16 shows the statistical results of the migration rate (mean ± SD, *P).* The results show that compared to the control group, the migration rate of HepG2 cells decreased significantly to different degrees after the treatment with the polypeptide or derivative thereof for 48 hours, demonstrating that the polypeptide and derivatives thereof (N1-N54) inhibited the migration ability of HepG2 cells.

**Table 16 Statistical results of cell migration rate**

| Group | Migration rate (%) (mean ± SD, *P)* | Group | Migration rate (%) (mean ± SD, *P)* | Group | Migration rate (%) (mean ± SD, *P)* |
|---|---|---|---|---|---|
| Control group | 71.01±6.63 | | | | |
| N1 | 17.42±7.70,^{∗∗∗} | N19 | 12.10±6.15,^{∗∗∗} | N37 | 13.81±6.14,^{∗∗∗} |
| N2 | 13.05±6.50,^{∗∗∗} | N20 | 14.22±6.12,^{∗∗∗} | N38 | 12.81±7.32,^{∗∗∗} |
| N3 | 17.57±6.98,^{∗∗∗} | N21 | 15.00±7.29,^{∗∗∗} | N39 | 15.73±6.79,^{∗∗∗} |
| N4 | 25.37±6.11,^{∗∗∗} | N22 | 18.61±8.35,^{∗∗∗} | N40 | 32.55±4.09,^{∗∗} |
| N5 | 19.84±5.04,^{∗∗∗} | N23 | 17.22±7.12,^{∗∗∗} | N41 | 25.59±6.26,^{∗∗∗} |
| N6 | 16.65±6.70,^{∗∗∗} | N24 | 16.38±6.48,^{∗∗∗} | N42 | 30.92±4.57,^{∗∗} |
| N7 | 11.54±9.00,^{∗∗∗} | N25 | 14.22±7.10,^{∗∗∗} | N43 | 26.54±7.89,^{∗∗} |
| N8 | 18.65±6.88,^{∗∗∗} | N26 | 16.86±7.90,^{∗∗∗} | N44 | 45.17±3.61,^{∗∗} |
| N9 | 35.11±5.16,^{∗∗} | N27 | 39.99±3.19,^{∗∗} | N45 | 52.98±4.08,^{∗} |
| N10 | 39.45±5.00,^{∗∗} | N28 | 12.25±8.40,^{∗∗∗} | N46 | 23.81±9.84,^{∗∗} |
| N11 | 53.36±5.49,^{∗} | N29 | 10.62±8.97,^{∗∗∗} | N47 | 54.67±7.02,^{∗} |
| N12 | 48.01±5.38,^{∗∗} | N30 | 16.60±7.85,^{∗∗∗} | N48 | 25.43±5.00,^{∗∗∗} |
| N13 | 45.91±4.70,^{∗∗} | N31 | 13.64±7.59,^{∗∗∗} | N49 | 16.45±5.88,^{∗∗∗} |
| N14 | 48.61±4.63,^{∗∗} | N32 | 15.49±8.35,^{∗∗∗} | N50 | 16.03±4.37,^{∗∗∗} |
| N15 | 53.20±5.58,^{∗} | N33 | 19.27±8.14,^{∗∗∗} | N51 | 28.61±5.89,^{∗∗} |
| N16 | 43.25±5.18,^{∗∗} | N34 | 18.58±7.87,^{∗∗∗} | N52 | 15.39±7.19,^{∗∗∗} |
| N17 | 43.42±3.16,^{∗∗} | N35 | 16.83±7.92,^{∗∗∗} | N53 | 35.85±4.32,^{∗∗} |
| N18 | 48.73±3.52,^{∗∗} | N36 | 36.81±5.39,^{∗∗} | N54 | 46.62±3.73,^{∗∗} |
| **Note:** ^{∗}, *P<0.05;* ^{∗∗}, P<0.01; ^{∗∗∗}, P<0.001 | | | | | |

### Example 23 Inhibition on the migration ability of human breast cancer cells (MDA-MB-453) (scratch assay)

This example was a scratch assay to evaluate the inhibitory effect of the polypeptide or derivative thereof (N1-N54) on the migration ability of human breast cancer cells (MDA-MB-453). The experimental method was as described in Example 2. Figure 16 shows the microscope images of cell migration. Table 17 shows the statistical results of the migration rate (mean ± SD, *P).* The results show that compared to the control group, the migration rate of MDA-MB-453 cells decreased significantly to different degrees after the treatment with the polypeptide or derivative thereof for 48 hours, demonstrating that the polypeptide and derivatives thereof (N1-N54) inhibited the migration ability of MDA-MB-453 cells.

**Table 17 Statistical results of cell migration rate**

| Group | Migration rate (%) (mean ± SD, *P*) | Group | Migration rate (%) (mean ± SD, *P)* | Group | Migration rate (%) (mean ± SD, *P)* |
|---|---|---|---|---|---|
| Control group | 32.42±2.04 | | | | |
| N1 | 13.05±2.05,^{∗∗} | N19 | 25.49±2.69,^{∗} | N37 | 15.13±3.55,^{∗∗} |
| N2 | 12.96±3.13,^{∗∗} | N20 | 7.67±1.35,^{∗∗} | N38 | 12.46±2.5,^{∗∗∗} |
| N3 | 13.04±3.5,^{∗∗} | N21 | 13.59±2.07,^{∗∗} | N39 | 11.02±6.8,^{∗∗} |
| N4 | 15.74±4.83,^{∗} | N22 | 8.54±1.57,^{∗∗} | N40 | 13.88±6.51,^{∗∗} |
| N5 | 14.74±3.23,^{∗∗} | N23 | 18.2±3.53,^{∗∗} | N41 | 11.5±3.23,^{∗∗∗} |
| N6 | 13.01±3.14,^{∗∗} | N24 | 15.84±4.44,^{∗} | N42 | 14.12±9.89,^{∗∗} |
| N7 | 12.64±2.73,^{∗∗} | N25 | 21.05±2.53,^{∗} | N43 | 10.38±6.04,^{∗∗} |
| N8 | 9.44±2.09,^{∗∗∗} | N26 | 6.19±1.42,^{∗∗∗} | N44 | 15.78±3.27,^{∗∗} |
| N9 | 7.19±2.38,^{∗∗∗} | N27 | 21.24±3.73,^{∗} | N45 | 5.53±2.87,^{∗∗∗} |
| N10 | 6.9±1.65,^{∗∗∗} | N28 | 11.32±5.61,^{∗∗} | N46 | 5.99±2.1,^{∗∗∗} |
| N11 | 4.49±1.6,^{∗∗∗} | N29 | 13.73±3.29,^{∗∗} | N47 | 14.66±2.51,^{∗∗∗} |
| N12 | 6.32±1.78,^{∗∗∗} | N30 | 15.77±2.33,^{∗∗∗} | N48 | 12.62±3.25,^{∗∗} |
| N13 | 9.28±2.43,^{∗∗∗} | N31 | 10.49±3.13,^{∗∗∗} | N49 | 15.79±4.48,^{∗∗} |
| N14 | 7.58±2.34,^{∗∗∗} | N32 | 17.55±3.48,^{∗∗} | N50 | 18.35±3.91,^{∗∗} |
| N15 | 8.95±2.93,^{∗∗∗} | N33 | 10.76±4.13,^{∗∗∗} | N51 | 10.6±2.33,^{∗∗∗} |
| N16 | 8.11±1.71,^{∗∗∗} | N34 | 9.57±3.68,^{∗∗∗} | N52 | 13.87±3.13,^{∗∗∗} |
| N17 | 4.51±1.96,^{∗∗∗} | N35 | 8.46±2.77,^{∗∗∗} | N53 | 8.02±3.35,^{∗∗∗} |
| N18 | 9.97±2.44,^{∗∗∗} | N36 | 9.48±3.71,^{∗∗∗} | N54 | 3.19±1.08,^{∗∗∗} |
| Note: ^{∗}, P<0.05; ^{∗∗}, P<0.01; ^{∗∗∗}, P<0.001 | | | | | |

### Example 24 Inhibition on the migration ability of human breast cancer cells (MDA-MB-231) (scratch assay)

This example was a scratch assay to evaluate the inhibitory effect of the polypeptide or derivative thereof (N1-N54) on the migration ability of human breast cancer cells (MDA-MB-231). The experimental method was as described in Example 2. Figure 17 shows the microscope images of cell migration. Table 18 shows the statistical results of the migration rate (mean ± SD, *P).* The results show that compared to the control group, the migration rate of MDA-MB-231 cells decreased significantly to different degrees after the treatment with the polypeptide or derivative thereof for 48 hours, demonstrating that the polypeptide and derivatives thereof inhibited the migration ability of MDA-MB-231 cells.

**Table 18 Statistical results of cell migration rate**

| Group | Migration rate (%) (mean ± SD, *P)* | Group | Migration rate (%) (mean ± SD, *P)* | Group | Migration rate (%) (mean ± SD, *P)* |
|---|---|---|---|---|---|
| Control group | 36.19±5.15 | | | | |
| N1 | 21.83±3.73,^{∗} | N19 | 15.23±3.54,^{∗} | N37 | 20.43±3.23,^{∗} |
| N2 | 20.92±2.16,^{∗} | N20 | 20.18±3.38,^{∗} | N38 | 18.99±3.25,^{∗} |
| N3 | 21.98±2.74,^{∗} | N21 | 19.95±5.47,^{∗} | N39 | 19.08±3.17,^{∗} |
| N4 | 21.44±2.59,^{∗} | N22 | 16.94±3.17,^{∗} | N40 | 21.12±4.15,^{∗} |
| N5 | 22.13±3.22,^{∗} | N23 | 14.39±2.33,^{∗∗} | N41 | 14.75±4.28,^{∗∗} |
| N6 | 17.42±4.98,^{∗} | N24 | 20.97±3.37,^{∗} | N42 | 17.91±3.66,^{∗∗} |
| N7 | 21.04±2.46,^{∗} | N25 | 12.93±4.38,^{∗∗} | N43 | 12.19±5.70,^{∗∗} |
| N8 | 19.02±3.53,^{∗} | N26 | 19.02±5.06,^{∗} | N44 | 11.26±3.34,^{∗∗∗} |
| N9 | 15.96±3.92,^{∗} | N27 | 9.62±3.01,^{∗∗∗} | N45 | 13.02±4.54,^{∗∗} |
| N10 | 13.6±4.62,^{∗∗} | N28 | 9.36±1.8,^{∗∗} | N46 | 14.83±4.35,^{∗∗} |
| N11 | 10.68±5.72,^{∗∗} | N29 | 18±4.47,^{∗} | N47 | 21.82±4.11,^{∗} |
| N12 | 15.41±3.55,^{∗∗} | N30 | 9.02±2.02,^{∗∗∗} | N48 | 13.06±3.2,^{∗∗} |
| N13 | 7.45±2.6,^{∗∗∗} | N31 | 18.7±3.05,^{∗} | N49 | 13.41±3.12,^{∗∗} |
| N14 | 14.59±5.36,^{∗} | N32 | 13.33±3.83,^{∗∗} | N50 | 14.46±5.73,^{∗∗} |
| N15 | 13.03±5.72,^{∗∗} | N33 | 22.68±3.02,^{∗} | N51 | 14.5±2.68,^{∗∗} |
| N16 | 14.99±6.11,^{∗} | N34 | 17.34±4.82,^{∗} | N52 | 13.19±4.84,^{∗∗} |
| N17 | 12.04±4.13,^{∗∗} | N35 | 18.12±5.23,^{∗} | N53 | 18.05±6.08,^{∗} |
| N18 | 18.64±6.98,^{∗} | N36 | 15.93±4.61,^{∗} | N54 | 13.37±5.89,^{∗∗} |
| Note: ^{∗}, *P<0.05;* ^{∗∗}, P<0.01; ^{∗∗∗}, P<0.001 | | | | | |

### Example 25 Inhibition on the migration ability of human breast cancer cells (MCF-7) (scratch assay)

This example was a scratch assay to evaluate the inhibitory effect of the polypeptide or derivative thereof (N1-N54) on the migration ability of human breast cancer cells (MCF-7). The experimental method was as described in Example 2. Figure 18 shows the microscope images of cell migration. Table 19 shows the statistical results of the migration rate (mean ± SD, *P).* The results show that compared to the control group, the migration rate of MCF-7 cells decreased significantly to different degrees after the treatment with the polypeptide or derivative thereof for 48 hours, demonstrating that the polypeptide and derivatives thereof (N1-N54) inhibited the fine migration ability of MCF-7 cell.

**Table 19 Statistical results of cell migration rate**

| Group | Migration rate (%) (mean ± SD, *P*) | Group | Migration rate (%) (mean ± SD, *P)* | Group | Migration rate (%) (mean ± SD, *P)* |
|---|---|---|---|---|---|
| Control group | 30.97±1.79 | | | | |
| N1 | 11.18±2.89,^{∗∗} | N19 | 11.84±3.95,^{∗∗} | N37 | 8.64±3.76,^{∗∗} |
| N2 | 12.32±2.26,^{∗∗} | N20 | 13.73±4.97,^{∗∗} | N38 | 6.93±2.24,^{∗∗} |
| N3 | 10.39±2.07,^{∗∗} | N21 | 10.43±2.18,^{∗∗} | N39 | 7.74±2.46,^{∗∗} |
| N4 | 13.6±2.28,^{∗∗} | N22 | 8.79±3.01,^{∗∗} | N40 | 4.36±1.35,^{∗∗} |
| N5 | 14.50±2.66,^{∗∗} | N23 | 10.20±2.86,^{∗∗} | N41 | 5.19±2.94,^{∗∗∗} |
| N6 | 15.69±4.09,^{∗} | N24 | 9.43±1.12,^{∗∗} | N42 | 10.04±2.28,^{∗∗} |
| N7 | 12.59±3.23,^{∗∗} | N25 | 9.16±2.51,^{∗∗} | N43 | 7.83±3.42,^{∗∗} |
| N8 | 11.56±2.35,^{∗∗} | N26 | 10.43±3.34,^{∗∗} | N44 | 5.89±1.97,^{∗∗∗} |
| N9 | 5.28±2.11,^{∗∗∗} | N27 | 8.40±2.44,^{∗∗} | N45 | 17.06±2.87,^{∗} |
| N10 | 9.55±2.72,^{∗∗} | N28 | 14.83±5.38,^{∗} | N46 | 15.66±2.57,^{∗} |
| N11 | 9.34±3.74,^{∗∗} | N29 | 19.15±3.04,^{∗} | N47 | 18.41±2.35,^{∗} |
| N12 | 11.65±3.32,^{∗∗} | N30 | 14.34±4.06,^{∗} | N48 | 15.62±2.70,^{∗} |
| N13 | 13.79±2.05,^{∗∗} | N31 | 9.27±2.23,^{∗∗} | N49 | 7.29±3.80,^{∗∗} |
| N14 | 7.05±1.40,^{∗∗∗} | N32 | 14.18±2.82,^{∗∗} | N50 | 6.83±4.55,^{∗∗} |
| N15 | 11.57±2.10,^{∗∗} | N33 | 18.97±2.44,^{∗∗} | N51 | 8.56±4.18,^{∗∗} |
| N16 | 9.81±3.29,^{∗∗} | N34 | 15.23±4.39,^{∗} | N52 | 7.23±2.41,^{∗∗} |
| N17 | 11.47±4.24,^{∗∗} | N35 | 17.38±2.55,^{∗∗} | N53 | 9.45±2.39,^{∗∗} |
| N18 | 10.40±2.72,^{∗∗} | N36 | 13.78±2.62,^{∗∗} | N54 | 16.52±2.93,^{∗} |
| **Note:** ^{∗}, *P<0.05;* ^{∗∗}, P<0.01; ^{∗∗∗}, P<0.001 | | | | | |

### Example 26 Inhibition on the migration ability of human melanoma cells (A375) (scratch assay)

This example was a scratch assay to evaluate the inhibitory effect of the polypeptide or derivative thereof (N1-N54) on the migration ability of human melanoma cells (A375). The experimental method was as described in Example 2. Figure 19 shows the microscope images of cell migration. Table 20 shows the statistical results of the migration rate (mean ± SD, *P).* The results show that compared to the control group, the migration rate of A375 cells decreased significantly to different degrees after the treatment with the polypeptide or derivative thereof for 48 hours, demonstrating that the polypeptide and derivatives (N1-N54) inhibited the migration ability of A375 cells.

**Table 20 Statistical results of cell migration rate**

| Group | Migration rate (%) (mean ± SD, *P*) | Group | Migration rate (%) (mean ± SD, *P*) | Group | Migration rate (%) (mean ± SD, *P)* |
|---|---|---|---|---|---|
| Control group | 44.03±6.21 | | | | |
| N1 | 8.49±2.99,^{∗∗∗} | N19 | 12.93±3.34,^{∗∗∗} | N37 | 14.48±2.51,^{∗∗∗} |
| N2 | 6.52±1.25,^{∗∗∗} | N20 | 14.61±3.12,^{∗∗∗} | N38 | 15.57±3.65,^{∗∗∗} |
| N3 | 4.91±1.19,^{∗∗∗} | N21 | 8.29±3.50,^{∗∗∗} | N39 | 9.21±3.20,^{∗∗∗} |
| N4 | 5.06±2.32,^{∗∗∗} | N22 | 5.12±3.69,^{∗∗∗} | N40 | 6.47±1.39,^{∗∗∗} |
| N5 | 9.82±3.84,^{∗∗∗} | N23 | 11.13±2.15,^{∗∗∗} | N41 | 10.01±2.07,^{∗∗∗} |
| N6 | 11.22±1.65, ^{∗∗∗} | N24 | 15.79±4.16, ^{∗∗} | N42 | 8.92±2.25,^{∗∗∗} |
| N7 | 8.95±1.27,^{∗∗∗} | N25 | 17.67±2.41,^{∗∗} | N43 | 11.22±1.74,^{∗∗∗} |
| N8 | 4.74±1.84,^{∗∗∗} | N26 | 12.23±2.48,^{∗∗∗} | N44 | 6.07±1.59,^{∗∗∗} |
| N9 | 8.96±2.46,^{∗∗∗} | N27 | 12.52±3.78, ^{∗∗∗} | N45 | 18.79±2.28,^{∗∗} |
| N10 | 6.71±2.07,^{∗∗∗} | N28 | 10.80±2.66,^{∗∗∗} | N46 | 9.15±2.10,^{∗∗∗} |
| N11 | 11.54±2.23, ^{∗∗∗} | N29 | 14.01±4.22,^{∗∗∗} | N47 | 8.82±1.49,^{∗∗∗} |
| N12 | 12.46±3.75, ^{∗∗∗} | N30 | 6.96±2.18,^{∗∗∗} | N48 | 9.09±2.23,^{∗∗∗} |
| N13 | 16.28±4.19,^{∗∗} | N31 | 12.5±2.29,^{∗∗∗} | N49 | 6.51±1.53,^{∗∗∗} |
| N14 | 6.28±1.12,^{∗∗∗} | N32 | 18.22±3.95,^{∗∗} | N50 | 15.97±2.33,^{∗∗} |
| N15 | 10.62±2.53,^{∗∗∗} | N33 | 9.98±3.11,^{∗∗∗} | N51 | 17.96±3.20,^{∗∗} |
| N16 | 11.28±3.14,^{∗∗∗} | N34 | 15.11±2.01,^{∗∗∗} | N52 | 19.57±2.21,^{∗∗} |
| N17 | 14.35±2.52,^{∗∗∗} | N35 | 19.51±2.28,^{∗∗} | N53 | 18.83±4.71,^{∗∗} |
| N18 | 12.67±1.33,^{∗∗∗} | N36 | 16.96±2.18,^{∗∗} | N54 | 16.91±3.21,^{∗∗} |
| **Note:** ^{∗∗}, P<0.01; ^{∗∗∗}, P<0.001 | | | | | |

### Example 27 Inhibition on the migration ability of human oral epidermoid carcinoma KB cells (scratch assay)

This example was a scratch assay to evaluate the inhibitory effect of the polypeptide or derivative thereof (N1-N54) on the migration ability of human oral epidermoid carcinoma cells (KB). The experimental method was as described in Example 2. Figure 20 shows the microscope images of cell migration. Table 21 shows the statistical results of the migration rate (mean ± SD, *P).* The results show that compared to the control group, the migration rate of KB cells decreased significantly to different degrees after the treatment with the polypeptide or derivative thereof for 48 hours, demonstrating that the polypeptide and derivatives thereof (N1-N54) inhibited the migration of KB cells.

**Table 21 Statistical results of cell migration rate**

| Group | Migration rate (%) (mean ± SD, *P*) | Group | Migration rate (%) (mean ± SD, *P*) | Group | Migration rate (%) (mean ± SD, *P)* |
|---|---|---|---|---|---|
| Control group | 54.03±4.21 | | | | |
| N1 | 7.49±2.99,^{∗∗∗} | N19 | 8.14±2.21,^{∗∗∗} | N37 | 24.48±2.51,^{∗} |
| N2 | 6.82±3.25,^{∗∗∗} | N20 | 6.92±3.28,^{∗∗∗} | N38 | 15.57±3.65,^{∗} |
| N3 | 9.95±2.19,^{∗∗∗} | N21 | 9.87±2.84,^{∗∗∗} | N39 | 9.21±4.20,^{∗∗∗} |
| N4 | 5.96±2.32,^{∗∗∗} | N22 | 10.02±2.35,^{∗∗∗} | N40 | 6.47±4.39,^{∗∗∗} |
| N5 | 9.62±3.84,^{∗∗∗} | N23 | 10.57±1.7,^{∗∗∗} | N41 | 20.01±2.07,^{∗∗} |
| N6 | 15.79±3.16,^{∗∗} | N24 | 24.03±4.21,^{∗} | N42 | 8.92±2.65,^{∗∗∗} |
| N7 | 11.11±3.86,^{∗∗∗} | N25 | 18.32±2.63,^{∗∗} | N43 | 11.22±1.74,^{∗∗∗} |
| N8 | 11.55±1.62,^{∗∗∗} | N26 | 23.71±4.86,^{∗} | N44 | 6.07±1.59,^{∗∗∗} |
| N9 | 9.27±2.63,^{∗∗∗} | N27 | 12.53±3.88, ^{∗∗} | N45 | 8.79±2.28,^{∗∗∗} |
| N10 | 13.87±3.48,^{∗∗∗} | N28 | 11.24±3.52,^{∗∗∗} | N46 | 9.15±1.60,^{∗∗∗} |
| N11 | 8.29±1.96,^{∗∗∗} | N29 | 8.07±1.79,^{∗∗∗} | N47 | 8.82±1.89,^{∗∗∗} |
| N12 | 11.53±2.78,^{∗∗∗} | N30 | 8.75±3.35,^{∗∗∗} | N48 | 9.09±4.23,^{∗∗∗} |
| N13 | 14.53±3.53,^{∗∗} | N31 | 7.17±1.67,^{∗∗∗} | N49 | 16.51±3.93,^{∗∗} |
| N14 | 11.22±2.66,^{∗∗∗} | N32 | 11.70±2.95,^{∗∗∗} | N50 | 15.97±2.93,^{∗∗} |
| N15 | 40.62±3.53,^{∗} | N33 | 6.01±1.62,^{∗∗∗} | N51 | 37.96±3.60,^{∗} |
| N16 | 18.28±3.45,^{∗∗} | N34 | 12.88±1.71,^{∗∗∗} | N52 | 9.57±1.61,^{∗∗∗} |
| N17 | 14.35±2.52,^{∗∗} | N35 | 6.07±2.30,^{∗∗∗} | N53 | 8.83±1.75,^{∗∗∗} |
| N18 | 12.67±2.33,^{∗∗∗} | N36 | 16.25±3.04,^{∗∗} | N54 | 6.91±3.44,^{∗∗∗} |
| **Note:** ^{∗}, *P<0.05;* ^{∗∗}, P<0.01; ^{∗∗∗}, P<0.001 | | | | | |

### Example 28 Inhibition on the migration ability of human pharyngeal squamous-cell carcinoma cells (Fadu) (scratch assay)

This example was a scratch assay to evaluate the inhibitory effect of the polypeptide or derivative thereof (N1-N54) on the migration ability of human pharyngeal squamous-cell carcinoma cells (Fadu). The experimental method was as described in Example 2. Figure 21 shows the microscope images of cell migration. Table 22 shows the statistical results of the migration rate (mean ± SD, *P).* The results show that compared to the control group, the migration rate of Fadu cells decreased significantly to different degrees after being treated with 10 µM polypeptide or derivative thereof for 48 hours, demonstrating that the polypeptide and derivatives thereof (N1-N54) inhibited Fadu cell migration.

**Table 22 Statistical results of cell migration rate**

| Group | Migration rate (%) (mean ± SD, *P)* | Group | Migration rate (%) (mean ± SD, *P)* | Group | Migration rate (%) (mean ± SD, *P)* |
|---|---|---|---|---|---|
| Control group | 28.76±4.08 | | | | |
| N1 | 5.68±2.86,^{∗∗∗} | N19 | 17.23±3.53,^{∗} | N37 | 15.89±3.88,^{∗} |
| N2 | 10.71±2.30,^{∗∗} | N20 | 15.21±4.90,^{∗} | N38 | 18.18±3.61,^{∗} |
| N3 | 12.97±3.58,^{∗∗} | N21 | 16.87±6.77,^{∗} | N39 | 18.48±5.74,^{∗} |
| N4 | 14.32±2.67,^{∗∗} | N22 | 15. 11±4.99, ^{∗} | N40 | 10.25±2.76,^{∗∗} |
| N5 | 9.07±2.08,^{∗∗∗} | N23 | 16.93±3.67,^{∗} | N41 | 12.00±4.73,^{∗∗} |
| N6 | 11.34±2.04,^{∗∗} | N24 | 14.07±5.12, ^{∗} | N42 | 18.63±2.79,^{∗} |
| N7 | 13.12±3.56,^{∗∗} | N25 | 9.89±2.47,^{∗∗∗} | N43 | 10.04±2.50,^{∗∗} |
| N8 | 9.54±5.75,^{∗∗} | N26 | 13.70±4.57,^{∗∗} | N44 | 13.19±3.83,^{∗∗} |
| N9 | 16.82±3.57,^{∗} | N27 | 15.99±3.96,^{∗} | N45 | 9.34±2.86,^{∗∗∗} |
| N10 | 15.93±4.73,^{∗} | N28 | 15.23±3.59,^{∗} | N46 | 7.58±3.07,^{∗∗∗} |
| N11 | 19±5.37,^{∗} | N29 | 12.27±2.23,^{∗∗} | N47 | 10.27±4.61,^{∗∗∗} |
| N12 | 17.72±2.53,^{∗∗} | N30 | 13.85±3.59,^{∗∗} | N48 | 8.78±4.34,^{∗∗∗} |
| N13 | 9.38±2.46,^{∗∗∗} | N31 | 12.13±5.10,^{∗∗} | N49 | 6.29±2.62,^{∗∗∗} |
| N14 | 12.90±2.47,^{∗∗} | N32 | 14.63±4.96,^{∗} | N50 | 5.44±1.44,^{∗∗∗} |
| N15 | 12.45±4.28,^{∗∗} | N33 | 10.28±2.06,^{∗∗} | N51 | 8.71±1.79,^{∗∗∗} |
| N16 | 5.51±1.32,^{∗∗∗} | N34 | 7.38±2.85,^{∗∗∗} | N52 | 7.76±2.08,^{∗∗∗} |
| N17 | 8.78±2.34,^{∗∗∗} | N35 | 8.71±1.79,^{∗∗∗} | N53 | 13.8±3.05,^{∗∗} |
| N18 | 6.29±2.62,^{∗∗∗} | N36 | 18.76±4.08,^{∗} | N54 | 15.88±5.38,^{∗} |
| **Note:** ^{∗}, P<0.05; ^{∗∗}, P<0.01; ^{∗∗∗}, P<0.001 | | | | | |

### Example 29 Inhibition on the migration ability of human colon cancer cells (HCT-116) (scratch assay)

This example was a scratch assay to evaluate the inhibitory effect of the polypeptide or derivative thereof (N1-N54) on the migration ability of human colon cancer cells (HCT-116). The experimental method was as described in Example 2. Figure 22 shows the microscope images of cell migration. Table 23 shows the results of migration rate statistics (mean ± SD, *P).* The results show that compared to the control group, the migration rate of HCT-116 cells decreased significantly to different degrees after being treated with 10 µM polypeptide or derivative thereof for 48 hours, demonstrating that the polypeptide and derivatives thereof inhibited the migration ability of HCT-116 cells.

**Table 23 Statistical results of cell migration rate**

| Group | Migration rate (%) (mean ± SD, *P)* | Group | Migration rate (%) (mean ± SD, *P)* | Group | Migration rate (%) (mean ± SD, *P)* |
|---|---|---|---|---|---|
| Control group | 47.78±6.07 | | | | |
| N1 | 10.27±3.64,^{∗∗∗} | N19 | 9.80±2.62,^{∗∗∗} | N37 | 4.99±1.68,^{∗∗∗} |
| N2 | 23.42±6.12,^{∗∗} | N20 | 8.17±1.07,^{∗∗∗} | N38 | 5.97±2.44,^{∗∗∗} |
| N3 | 22.06±4.59,^{∗∗} | N21 | 12.85±3.12,^{∗∗∗} | N39 | 4.29±2.88,^{∗∗∗} |
| N4 | 25.48±2.79,^{∗∗} | N22 | 7.78±3.07,^{∗∗∗} | N40 | 2.19±1.45,^{∗∗∗} |
| N5 | 12.75±2.82,^{∗∗∗} | N23 | 13.08±2.64,^{∗∗∗} | N41 | 5.78±2.76,^{∗∗∗} |
| N6 | 18.13±2.68,^{∗∗} | N24 | 12.14±2.49,^{∗∗∗} | N42 | 6.75±2.33,^{∗∗∗} |
| N7 | 20.09±2.41,^{∗∗} | N25 | 18.70±2.39,^{∗∗} | N43 | 4.56±1.52,^{∗∗∗} |
| N8 | 16.96±3.87,^{∗∗} | N26 | 9.81±2.92,^{∗∗∗} | N44 | 8.80±4.89,^{∗∗∗} |
| N9 | 25.96±3.84,^{∗∗} | N27 | 24.14±3.00,^{∗∗∗} | N45 | 28.34±2.87,^{∗∗} |
| N10 | 8.70±2.39,^{∗∗∗} | N28 | 9.19±3.22,^{∗∗∗} | N46 | 19.78±3.78,^{∗∗} |
| N11 | 13.81±2.92,^{∗∗∗} | N29 | 8.24±2.32,^{∗∗∗} | N47 | 18.92±2.11,^{∗∗} |
| N12 | 8.14±3.00,^{∗∗∗} | N30 | 12.41±2.99,^{∗∗∗} | N48 | 18.03±4.62,^{∗∗} |
| N13 | 12.19±3.22,^{∗∗∗} | N31 | 7.39±2.06,^{∗∗∗} | N49 | 18.84±2.99,^{∗∗} |
| N14 | 12.24±2.32,^{∗∗∗} | N32 | 8.17±1.07,^{∗∗∗} | N50 | 17.04±4.96,^{∗∗} |
| N15 | 10.41±2.99,^{∗∗∗} | N33 | 12.85±3.12,^{∗∗∗} | N51 | 16.11±3.20,^{∗∗} |
| N16 | 9.39±2.06,^{∗∗∗} | N34 | 17.96±3.84,^{∗∗} | N52 | 18.71±3.59,^{∗∗} |
| N17 | 23.09±2.41,^{∗∗∗} | N35 | 9.08±2.64,^{∗∗∗} | N53 | 14.42±3.21,^{∗∗} |
| N18 | 16.96±4.87,^{∗∗} | N36 | 22.14±2.49,^{∗∗} | N54 | 26.50±3.99,^{∗∗} |
| **Note:** ^{∗∗}, P<0.01; ^{∗∗∗}, P<0.001 | | | | | |

### Example 30 Inhibition on the migration ability of human thyroid cancer cells (FRO) (scratch assay)

This example was a scratch assay to evaluate the inhibitory effect of the polypeptide or derivative thereof (N1-N54) on the migration ability of human thyroid cancer cells (FRO). The experimental method was as described in Example 2. Figure 23 shows the microscope images of cell migration. Table 24 shows the statistical results of the migration rate (mean ± SD, *P).* The results show that compared to the control group, the migration rate of FRO cells decreased significantly to different degrees after being treated with 10 µM polypeptide or derivative thereof for 48 hours, demonstrating that the polypeptide and derivatives thereof (N1-N54) inhibited the migration ability of FRO cells.

**Table 24 Statistical results of cell migration rate**

| Group | Migration rate (%) (mean ± SD, *P)* | Group | Migration rate (%) (mean ± SD, *P)* | Group | Migration rate (%) (mean ± SD, *P)* |
|---|---|---|---|---|---|
| Control group | 60.34±8.32 | | | | |
| N1 | 7.45±2.78,^{∗∗∗} | N19 | 25.59±4.08,^{∗∗} | N37 | 36.47±5.69,^{∗} |
| N2 | 22.88±5.43,^{∗∗} | N20 | 24.03±8.61,^{∗} | N38 | 19.23±7.52,^{∗∗} |
| N3 | 3.51±1.34,^{∗∗∗} | N21 | 30.45±4.94,^{∗} | N39 | 36.51±4.36,^{∗} |
| N4 | 11.34±2.73,^{∗∗∗} | N22 | 42.34±4.98,^{∗} | N40 | 39.96±4.95,^{∗} |
| N5 | 4.71±1.90,^{∗∗∗} | N23 | 34.39±4.10,^{∗} | N41 | 40.10±2.73,^{∗} |
| N6 | 3.24±1.80,^{∗∗∗} | N24 | 16.54±3.19,^{∗∗} | N42 | 25.71±2.79,^{∗∗∗} |
| N7 | 5.73±2.04,^{∗∗∗} | N25 | 14.31±2.47,^{∗∗∗} | N43 | 19.78±4.60,^{∗∗} |
| N8 | 7.28±2.89,^{∗∗∗} | N26 | 11.21±4.01,^{∗∗∗} | N44 | 34.28±3.45, ^{∗} |
| N9 | 20.51±5.04,^{∗∗} | N27 | 13.42±3.35,^{∗∗∗} | N45 | 24.22±6.27,^{∗∗} |
| N10 | 14.14±3.64,^{∗∗∗} | N28 | 8.77±2.85,^{∗∗∗} | N46 | 9.06±2.42,^{∗∗∗} |
| N11 | 10.09±4.33,^{∗∗∗} | N29 | 5.12±2.67,^{∗∗∗} | N47 | 16.47±3.83,^{∗∗} |
| N12 | 7.67±3.29,^{∗∗∗} | N30 | 5.16±1.67,^{∗∗∗} | N48 | 13.78±2.61,^{∗∗∗} |
| N13 | 11.99±2.39,^{∗∗∗} | N31 | 9.77±1.96,^{∗∗∗} | N49 | 15.77±2.99,^{∗∗} |
| N14 | 14.09±2.47,^{∗∗∗} | N32 | 7.83±2.06,^{∗∗∗} | N50 | 11.80±4.15,^{∗∗∗} |
| N15 | 8.84±2.27,^{∗∗∗} | N33 | 7.03±2.02,^{∗∗∗} | N51 | 17.93±5.55,^{∗∗} |
| N16 | 11.72±4.41,^{∗∗∗} | N34 | 8.74±3.40,^{∗∗∗} | N52 | 10.86±3.90,^{∗∗∗} |
| N17 | 11.38±2.27,^{∗∗∗} | N35 | 8.04±2.90,^{∗∗∗} | N53 | 20.32±4.58,^{∗∗} |
| N18 | 22.96±5.05 | N36 | 18.16±2.04 | N54 | 8.26±3.47 |
| **Note:** ^{∗}, *P<0.05;* ^{∗∗}, P<0.01; ^{∗∗∗}, P<0.001 | | | | | |

### Example 31 Inhibition on the migration ability of human prostate cancer cells (22RV1) (scratch assay)

This example was a scratch assay to evaluate the inhibitory effect of the polypeptide or derivative thereof (N1-N54) on the migration ability of human prostate cancer cells (22RV1). The experimental method was as described in Example 2. Figure 24 shows the microscope images of cell migration. Table 25 shows the statistical results of the migration rate. The results show that compared to the control group, the migration rate of 22RV1 cells decreased significantly to different degrees after treating with 10 µM of polypeptide or derivative thereof for 48 hours, demonstrating that the polypeptide and derivatives thereof (N1-N54) inhibited the migration ability of 22RV1 cells.

**Table 25 Statistical results of cell migration rate**

| Group | Migration rate (%) (mean ± SD, P) | Group | Migration rate (%) (mean ± SD, P) | Group | Migration rate (%) (mean ± SD, *P)* |
|---|---|---|---|---|---|
| Control group | 25.41±5.01 | | | | |
| N1 | 7.26±3.36,^{∗∗} | N19 | 5.36±1.6,^{∗∗} | N37 | 4.86±1.91,^{∗∗} |
| N2 | 6.48±2.97,^{∗∗} | N20 | 4.88±1.48,^{∗∗} | N38 | 7.73±1.58,^{∗∗} |
| N3 | 6.15±4.65,^{∗∗} | N21 | 12.79±3.06,^{∗} | N39 | 4.21±2.81,^{∗∗} |
| N4 | 13 .44±3 .24, ^{∗} | N22 | 6.10±2.72,^{∗∗} | N40 | 6.65±2.54,^{∗∗} |
| N5 | 10.37±3.53,^{∗} | N23 | 5.7±3.23,^{∗∗} | N41 | 7.83±1.84,^{∗∗} |
| N6 | 12.31±2.85,^{∗} | N24 | 8.44±1.63,^{∗∗} | N42 | 5.89±1.02,^{∗∗} |
| N7 | 6.54±2.24,^{∗∗} | N25 | 13.02±2.27,^{∗} | N43 | 6.57±1.35,^{∗∗} |
| N8 | 8.75±1.98,^{∗∗} | N26 | 11.42±2.08,^{∗} | N44 | 15.11±2.39,^{∗} |
| N9 | 14.84±2.96,^{∗} | N27 | 14.76±2.79,^{∗} | N45 | 11.63±2.82,^{∗} |
| N10 | 13.39±2.3,^{∗} | N28 | 15.63±2.59,^{∗} | N46 | 10.10±2.08,^{∗} |
| N11 | 9.43±2.23,^{∗∗} | N29 | 9.99±3.89,^{∗} | N47 | 5.92±2.53,^{∗∗} |
| N12 | 6.61±2.83,^{∗∗} | N30 | 10.82±2.55,^{∗} | N48 | 5.61±3.14,^{∗∗} |
| N13 | 9.77±3.66,^{∗} | N31 | 12.40±2.24,^{∗} | N49 | 6.18±1.89,^{∗∗} |
| N14 | 14.06±2.37,^{∗} | N32 | 8.68±1.66,^{∗∗} | N50 | 6.20±3.31,^{∗∗} |
| N15 | 8.36±2.03,^{∗∗} | N33 | 10.03±2.15,^{∗} | N51 | 5.23±2.42,^{∗∗} |
| N16 | 9.75±2.14,^{∗∗} | N34 | 5.00±2.29,^{∗∗} | N52 | 5.43±1.97,^{∗∗} |
| N17 | 4.61±1.64,^{∗∗} | N35 | 7.13±2.23,^{∗∗} | N53 | 10.75±3.22,^{∗} |
| N18 | 6.75±3.22,^{∗∗} | N36 | 13.68±2.56,^{∗} | N54 | 12.94±2.70,^{∗} |
| Note: ^{∗}, *P<0.05;* ^{∗∗}, P<0.01 | | | | | |

### Example 32 Acute toxicity test on mice after continuous administration for 14 days

The experimental animals were C57BL/6J mice (purchased from Chengdu Dashuo Experimental Animal Co., Ltd., male, 4-5 weeks old). Experimental groups were: control group (physiological saline); treatment group (polypeptide or derivative thereof (N1-N54)). There were three experimental animals in each group. The control group was administered with normal saline via the tail vein, 100 µL/time/day. The dosage of polypeptide or derivative thereof groups (N1-N54) was 200 mg/kg/day via the tail vein, and the volume was 100 µL. The mice were administered continuously for 13 days. The experimental animals were sacrificed on the 14th day, and the brain, heart, liver, lung, kidney, spleen and other organs were collected for pathological analysis (HE staining). The experimental results are shown in figures 25-30. Figure 25 shows images of the brain sections. The staining results of the N1-N54 administration groups and the control group were not significantly different. The neurons in the hippocampus of the mouse brain section were arranged regularly, and there were no pathological phenomena such as bleeding points, inflammatory cell infiltration and edema. Figure 26 shows images of the cardiac sections. The staining results of the N1-N54 administration groups and the control group were not significantly different. There was no edema or hypertrophy of cardiomyocytes, and no pathological phenomena such as inflammatory cell infiltration, capillary and fibroblast proliferation. Figure 27 shows images of the liver sections. There is no significant difference between the staining results of the N1-N54 administration groups and the control group. Hepatocytes were arranged in a single row radially centered on the central vein. There is no pathological phenomenon such as vacuolar degeneration, necrosis, inflammatory cell infiltration or marginal fibrosis. Figure 28 shows the images of the lung sections. There was no significant difference between the staining results of each administration group and the control group. The alveolar cavity had a vacuolar thin-walled structure. And there was no pathological phenomenon such as alveolar wall thickening and inflammatory cell infiltration. Figure 29 shows images of the kidney sections. There was no significant difference between the staining results of each administration group and the control group. The glomerular structure was clear, without other pathological phenomenon such as granular degeneration, inflammatory cell infiltration, capillary congestion and the like. Figure 30 shows images of the spleen sections. There was no significant difference between the staining results of each administration group and the control group. The spleen structure was complete. The splenic sinusoid was surrounded by the splenic cord, connected to each other as a network. There was no pathological phenomenon such as thickening of the lymphatic sheath around the artery, and increased small spleen body numbers. In summary, the high-dose acute toxicity test results show that intravenous injection of any one of N1-N54 would not cause toxicity to organs in mice.

### Example 33 Test the effect on coagulation function in mice

C57BL/6J mice were used as experimental subjects (purchased from Chengdu Dashuo Experimental Animal Co., Ltd., male, weighing 16-17 g). Sterile saline or peptide drugs were injected via the tail vein once a day until Day 24. The mice in the control group were injected with sterile saline, and the mice in the administration groups (N1-N12) were injected with polypeptide drugs (200 mg/kg). There were three experimental animals in each group. On Day 25, the eyeballs were removed and blood (50 µL) was taken. The coagulation function of the mice was detected by the Abbott i-STAT blood gas analyzer with the ACT test card. The results are shown in Table 26. There was no significant change in the activated clotting time (ACT) of the N1 to N12 administration groups and the control group, showing that the peptide drugs do not affect the coagulation function of the mice.

**Table 26 Activated clotting time (s)**

| **Group** | **Activated clotting time (s)** (mean ± SD) | **Group** | **Activated clotting time (s)** (mean ± SD) | **Group** | **Activated clotting time (s)** (mean ± SD) |
|---|---|---|---|---|---|
| Control group | 73.50±7.15 | | | | |
| N1 | 73.64±9.31 | N5 | 72.91±8.04 | N9 | 72.09±8.07 |
| N2 | 74.82±9.62 | N6 | 74.09±8.51 | N10 | 73.27±7.36 |
| N3 | 72.55±8.27 | N7 | 73.27±8.43 | N11 | 75.18±7.99 |
| N4 | 73.64±8.12 | N8 | 74±8.92 | N12 | 75.55±7.61 |

### Example 34 Test of the immunogenicity of peptides

C57BL/6J mice were used as experimental subjects (purchased from Chengdu Dashuo Experimental Animal Co., Ltd., male, weighing 16-17 g). Sterile saline or peptide drugs were injected via the tail vein once a day until Day 13 or Day 24. The mice in the control group were injected with sterile saline, and the mice in the administration groups (N1-N12) were injected with peptide drugs (200 mg/kg). On Day 14 and Day 25, the eyeballs were removed and blood (800 µL) was taken. The serum was separated by a centrifuge, and the change of the level of immunoglobulin G (IgG) was detected by ELISA. The results are shown in Table 27. Compared with the control group, the IgG content of each administration group (N-N12) had no significant change on Day 14 and Day 25 after administration, showing that the peptide drugs barely have immunogenicity *in vivo.*

**Table 27 Immunoglobulin G (IgG) content (µg/mL)**

| **Group** | **IgG content on Day 14 (g/mL)** (mean ± SD) | **IgG content on Day 25 (g/mL)** (mean ± SD) | **Group** | **IgG content on Day 14 (g/mL)** (mean ± SD) | **IgG content on Day 25 (g/mL)** (mean ± SD) |
|---|---|---|---|---|---|
| Control group | 254.99±52.82 | 360.81±83.84 | | | |
| N1 | 258.98±37.06 | 354.74±54.77 | N7 | 252.97±42.1 | 359.38±74.34 |
| N2 | 253.22±42.06 | 365.31±75.38 | N8 | 263.26±40.67 | 375.64±79.02 |
| N3 | 252.64±42.46 | 365.08±75.47 | N9 | 250.58±44.17 | 368.19±81.96 |
| N4 | 245.65±31.16 | 366.54±75.87 | N10 | 260.26±46.67 | 356.46±85.56 |
| N5 | 243.26±33.26 | 354.43±74.78 | N11 | 250.37±44.38 | 359.38±82.67 |
| N6 | 250.26±44.5 | 358.77±74.93 | N12 | 250.04±44.7 | 343.54±58.66 |

The above are only the preferred embodiments of the present invention. It should be pointed out that for the ordinary skilled person in the art, several improvements and modifications can be made without departing from the principle of the present invention.

## Claims

1. Use of a polypeptide or a derivative thereof in the manufacture of a medicament for the prevention and/or treatment of a tumor, wherein the polypeptide comprises:
(I) an amino acid sequence set forth in SEQ ID NO: 1;
(II) an amino acid sequence derived from the amino acid sequence of (I) by deletion, substitution, addition and/or modification of one or more amino acids, and the polypeptide has a function identical or similar to that of the amino acid sequence of (I); or
(III) an amino acid sequence having more than 80% homology with the amino acid sequence of (I) or (II).

2. The use according to claim 1, wherein the deletion is selected from the group consisting of a deletion of an amino acid at the amino terminal of the polypeptide, a deletion of an amino acid at the carboxyl terminal of the polypeptide, a deletion of amino acid within the sequence of the polypeptide and a combination thereof.

3. The use according to claim 1 or 2, wherein the amino acid sequence of the polypeptide is selected from the group consisting of SEQ ID NO: 2, SEQ ID NO: 4, SEQ ID NO: 6, SEQ ID NO: 8, SEQ ID NO: 12 and SEQ ID NO: 13.

4. The use according to any one of claims 1 to 3, wherein the addition is selected from the group consisting of an addition of an amino acid at the amino terminal of the polypeptide, an addition of an amino acid at the carboxyl terminal of the polypeptide, an addition of an amino acid within the sequence of the polypeptide and a combination thereof.

5. The use according to claim 4, wherein the amino acid sequence of the polypeptide is set forth in SEQ ID NO: 17 or SEQ ID NO: 18.

6. The use according to any one of claims 1 to 5, wherein the substitution is selected from the group consisting of a substitution of an amino acid at the amino terminal of the polypeptide, a substitution of an amino acid at the carboxyl terminal of the polypeptide, a substitution of an amino acid within the sequence of the polypeptide and a combination thereof.

7. The use according to any one of claims 1 to 6, wherein the modification is selected from the group consisting of a modification of an amino acid at the amino terminal of the polypeptide, a modification of an amino acid at the carboxyl terminal of the polypeptide, a modification of an amino acid within the sequence of the polypeptide and a combination thereof.

8. The use according to any one of claims 1 to 7, wherein the polypeptide is derived from SEQ ID NO: 1 by a manner selected from the group consisting of: deletion and addition of an amino acid at the same time, substitution and addition of an amino acid at the same time, deletion and substitution of an amino acid at the same time, deletion and modification of an amino acid at the same time, addition and modification of an amino acid at the same time, substitution and modification of an amino acid at the same time, deletion, addition and modification of an amino acid at the same time, substitution, addition and modification of an amino acid at the same time, deletion, substitution and modification of an amino acid at the same time, deletion, substitution, addition and modification of an amino acid at the same time, and a combination thereof.

9. The use according to any one of claims 1 to 8, wherein the amino acid sequence of the polypeptide is selected from the group consisting of SEQ ID NO: 5, SEQ ID NO: 14, SEQ ID NO: 19, SEQ ID NO: 20, SEQ ID NO: 21, SEQ ID NO: 7, SEQ ID NO: 15, SEQ ID NO: 16, SEQ ID NO: 25, SEQ ID NO: 26, SEQ ID NO: 22, SEQ ID NO: 23, SEQ ID NO: 24, SEQ ID NO: 3, SEQ ID NO: 9, SEQ ID NO: 10, SEQ ID NO: 11, SEQ ID NO: 28, SEQ ID NO:29, SEQ ID NO: 30, SEQ ID NO: 31, SEQ ID NO: 27, SEQ ID NO: 37, SEQ ID NO: 38, SEQ ID NO: 39, SEQ ID NO: 32, SEQ ID NO: 33, SEQ ID NO: 40, SEQ ID NO: 41, SEQ ID NO: 42, SEQ ID NO: 43, SEQ ID NO: 44, SEQ ID NO: 34, SEQ ID NO: 35, SEQ ID NO: 36, SEQ ID NO: 50, SEQ ID NO: 51, SEQ ID NO: 52, SEQ ID NO: 53, SEQ ID NO: 54, SEQ ID NO: 45, SEQ ID NO: 46, SEQ ID NO: 48, SEQ ID NO: 49 and SEQ ID NO: 47.

10. The use according to any one of claims 1 to 9, wherein the tumor is selected from the group consisting of a head and neck cancer, a respiratory system tumor, a gastrointestinal tumor, a urinary system tumor, a male reproductive tumor, a gynecologic tumor, a skin cancer, an endothelial cell tumor, a brain tumor, a nervous system tumor, an endocrine organ tumor and a combination thereof.

11. The use according to claim 10, wherein
the head and neck cancer is selected from the group consisting of a lip cancer, an oral cancer, a salivary gland cancer, an oropharyngeal cancer, a nasopharyngeal cancer, a hypopharyngeal cancer and a combination thereof;
the respiratory system tumor is selected from the group consisting of a laryngeal cancer, a lung cancer, a mesothelioma and a combination thereof;
the gastrointestinal tumor is selected from the group consisting of a colorectal cancer, an anal cancer, an esophageal cancer, a gastric cancer, a liver cancer, a gallbladder carcinoma, a pancreatic cancer and a combination thereof;
the urinary system tumor is selected from the group consisting of a kidney cancer, a bladder cancer and a combination thereof;
the male reproductive tumor is selected from the group consisting of a penile cancer, a prostate cancer, a testicular cancer and a combination thereof;
the gynecologic tumor is selected from the group consisting of a breast cancer, a vulvar cancer, a vaginal cancer, a cervical cancer, a corpus carcinoma, an ovarian cancer and a combination thereof;
the skin cancer is selected from the group consisting of a melanoma, a non-melanoma skin cancer and a combination thereof;
the endothelial cell tumor is Kaposi's sarcoma;
the brain tumor is a brain cancer;
the nervous system tumor is a central nervous system tumor;
and the endocrine organ tumor is a thyroid cancer.

12. The use according to any one of claims 1 to 11, wherein the medicament comprises an active ingredient selected from the group consisting of the polypeptide or a derivative thereof as a single active ingredient, a combination of the polypeptide and a derivative thereof, a combination of the polypeptide or a derivative thereof with other medicament, a conjugate of the polypeptide or a derivative thereof labelled with a chemical or a biomarker, a solid medium or a semi-solid medium coupled with the polypeptide, a derivative thereof, a conjugate or a combination thereof, and a pharmaceutically acceptable carrier.

13. The use according to claim 12, wherein the pharmaceutically acceptable carrier is selected from the group consisting of a diluent, a filler, an excipient, a binder, a wetting agent, a disintegrant, an effervescent agent, a surfactant, an absorption enhancer, a lubricant, an adsorption carrier, a sustained-release microsphere, an implant, an in-situ microsphere, a liposome, amicroemulsion, an in-situ hydrogel, a nanoparticle, a protease inhibitor, a biological adhesive, a fusion protein, an antibody, a polypeptide and a combination thereof.
